# EUROPEAN PATENT APPLICATION

(11) **EP 4 094 762 A1**
(43) Date of publication of application: **30.11.2022**
(21) Application number: 21175673.9
(22) Date of filing: 25.05.2021
(51) Int. Cl.: A61K 31/4412, A61P 25/28, A61K 31/437, A61K 31/443, A61K 31/4436, A61K 31/50, A61K 31/505, A61K 31/538, A61K 31/5386

(54) **TREATMENT OF NEUROLOGICAL INDICATIONS**

(71) Applicant: UCL Business Ltd, London WC1E 6BT (GB)
(72) Inventor: The designation of the inventor has not yet been filed
(74) Representative: J A Kemp LLP

(57) **Abstract**

The present invention relates to the treatment of CNS disorders. The present invention relates in particular to the treatment of Alzheimer's disease.

## Description

The present invention relates to the treatment of CNS disorders. The present invention relates in particular to the treatment of Alzheimer's disease.

### Background to the invention

The P2Y1 receptor is a purinergic receptor that has been identified as a target in treating and/or preventing CNS disorders through the modulation of astrocyte hyperactivity. Examples of CNS disorders that may be susceptible to treatment and/or prevention by astroglial P2Y1 receptor inhibition include Alzheimer's disease, pain (both neuropathic and nociceptive), epilepsy, stroke (via neuroprotection), traumatic brain injury and amyotrophic lateral sclerosis. Non-limiting examples of literature evidence substantiating the principle of applying astroglial P2Y1 receptor inhibition for treating and/or preventing these CNS disorders includes:
- Alzheimer's disease - see (i) Delakate A, et al. Nat. Commun., 2014;5:5422; (ii) Reichenbach N, et al. J. Exp. Med. 2018;215(6):1649-1663; (iii) Reichenbach N, et al. EMBO Mol Med. 2019 11:e9665; and (iv) Shi A, et al. Cereb Cortex. 2020;30(3):1272-1290.
- Neuropathic pain - see (i) Barragán-Iglesias P, et al. Brain Res. 2016 1636:43-51; (ii) Mitchell R, et al. Mol Neurobiol. 2019 56(8):5917-5933; and (iii) Yang CT, et al. Sci Rep. 2019 9(1):16032.
- Nociceptive pain - see (i) Hockley JR, et al. J Neurosci. 2016 36(8):2364-76; (ii) Kwon SG, et al. Brain Res Bull. 2017 130:165-172; (iii) Mitchell R, et al. Mol Neurobiol. 2019 56(8):5917-5933; and (iv) Yang CT, et al. Sci Rep. 2019 9(1):16032.
- Epilepsy - see (i) Alvarez-Ferradas C, et al. Glia. 2015 63(9):1507-21; (ii) Alves M, et al. J Neurosci. 2019 39(27):5377-5392; (iii) Alves M, et al. Front Pharmacol. 2020 10:1558; (iv) Alves M, et al. Epilepsia. 2017 58(9):1603-1614; (v) Nikolic L, et al. Glia. 2018 66(12):2673-2683; and (vi) Wellmann M, et al. Front Cell Neurosci. 2018 12:446.
- Stroke (*via* neuroprotection) - see (i) Carmo MR, et al. Eur J Neurosci. 2014 39:614-622; (ii) Chin Y, et al. J Neuroinflammation. 2013 10:95; (iii) Forster D, Reiser G. Purinergic Signal. 2015 11:441-454; (iv) Fukumoto Y, et al. J Cereb Blood Flow Metab. 2019 39:2144-2156;(v) Kuboyama K, et al. J Cereb Blood Flow Metab. 2011 31:1930-1941; (vi) Giovanna, M., et al. PloS one 2014, vol. 9, 12 e115273; and (vii) Guo, H., et al. Molecular Medicine Reports, 2018 17, 1819-1824.
- Traumatic brain injury - see (i) Choo AM, et al. Brain. 2013 136:65-80; and (ii) Shinozaki Y, et al. Cell Rep. 2017 19:1151-1164.
- Amyotrophic lateral sclerosis - see (i) Cieślak M, et al. Purinergic Signal. 2019 15(1):1-15; (ii) Izrael M, et al. Front Neurosci. 2020;14:824; (iii) Jiang MC, et al. Neuroscience. 2017 362:33-46; and (iv) Pehar M, et al. Curr Pharm Des. 2017 23:5010-5021.

Further CNS disorders associated with astrocytes include Huntington's disease, Parkinson's disease and frontotemporal dementia (see (i) Wilton DK & Stevens B. Neurobiol Dis. 2020 143; (ii) Miyazaki I & Asanuma M. Cells. 2020 9:2623; and (iii) Radford RA, et al. Front Cell Neurosci. 2015 9:414). These conditions therefore represent additional candidate CNS disorders for treatment and/or prevention through P2Y1 inhibition.

A number of P2Y1 inhibitors have previously been developed, as described in e.g. WO 2005/113511, WO 2005/113537, WO 2006/078621, WO 2008/023235, WO 2014/022253, WO 2014/022349, WO 2014/022343, Yang et al., J. Med. Chem. 2014 57(14) 6150-6164 and Peng et al., European Journal of Medicinal Chemistry 158 (2018) 302-310. However, each of these references concerns the use of P2Y1 inhibitors in treating thromboembolic disorders, and therefore seek only to provide compounds capable of entering, and circulating within, the blood stream. In contrast to achieving systemic delivery, providing active compounds that are capable of crossing the blood-brain barrier [BBB] is known to be extremely challenging (see Pardridge W M., Alzheimers Dement. 2009;5(5):427-43). For instance, as of 2009, it was determined that more than 98% of all small molecule drugs, and ∼100% of all large molecule drugs, do not cross the BBB. This has the implication that drugs which might hypothetically be useful for treating CNS disorders will - in many cases - only be administrable to the CNS *via* highly invasive, and so often impractical, drug delivery methods such as transcranial drug delivery. Such an administration mode is disadvantageous, indeed to the extent of becoming practically infeasible, not only because of the need for neurosurgical intervention, but also due to problems with the extent to which the active agent is capable of diffusing from the injection site throughout the human brain.

It is consequently desirable to identify P2Y1 inhibitors that are capable of crossing the blood-brain barrier, i.e. to provide P2Y1 inhibitors that are CNS penetrants and that are therefore susceptible to administration outside of the CNS (e.g. by oral administration), whilst still being able subsequently to access the CNS.

The present inventors have now identified specific P2Y1 inhibitors that are capable of crossing the blood-brain barrier. The ability of these inhibitors to pass across the blood-brain barrier is surprising at least in view of the well-known wide inability of most small molecule active pharmaceutical ingredients (APIs) to demonstrate such properties, the absence of any suggestion in the existing literature that previously described P2Y1 inhibitors may have such properties, and, still further, the physicochemical properties of these compounds, being inconsistent with the accepted CNS drug-like chemical space and with *in silico* predictors of brain penetration such as the state-of-the-art CNS Multiparameter Optimization (CNS MPO) score (Wager, T.T et al. ACS Chem. Neurosci. 2010, 1, 435-449). Furthermore, the ability of the presently identified compounds to cross the blood-brain barrier, and thereafter exert clinically significant effects, following e.g. oral administration is not shared with some other P2Y1 inhibiting compounds. One particular focus of the present invention is Alzheimer's disease.

### Summary of the invention

The present invention provides a compound for use in a method of treating and/or preventing a CNS disorder (typically a CNS disorder susceptible to treatment by P2Y1 inhibition). The present invention also provides a pharmaceutical composition for use in a method of treating and/or preventing a CNS disorder (typically a CNS disorder susceptible to treatment by P2Y1 inhibition), the pharmaceutical composition comprising such a compound, in association with one of more pharmaceutically acceptable carriers.

The present invention also provides the use of such a compound, or such a pharmaceutical composition, for the manufacture of a medicament for use in a method for treating and/or preventing a CNS disorder (typically a CNS disorder susceptible to treatment by P2Y1 inhibition).

The present invention also provides a method of treating and/or preventing a CNS disorder (typically a CNS disorder susceptible to treatment by P2Y1 inhibition) in a patient in need thereof, the method comprising administering such a compound, or such a pharmaceutical composition, to the patient.

In the present invention, the compound in the 'compound for use' of the invention, the compound in the 'pharmaceutical composition', the compound in the 'use of a compound for the manufacture of a medicament' of the invention, and the compound in the 'method of treating and/or preventing a CNS disorder' of the invention, is referred to herein as a 'compound for use of the invention'. For avoidance of doubt, such a 'compound for use of the invention' corresponds to the compound recited in any of the 'compound for use', 'pharmaceutical composition', 'use of a compound for the manufacture of a medicament' and 'method of treating and/or preventing a CNS disorder' aspects of the invention. The 'compound for use of the invention' is further defined in the following disclosure, including but not limited to its enumerated aspects, and the claims.

In a specific embodiment, the present invention provides a compound that (a) is 1-(2-(2-(*tert*-butyl)phenoxy)pyridin-3-yl)-3-(4-(trifluoromethoxy)phenyl)urea, or (b) is a tautomer, pharmaceutically acceptable salt or solvate thereof, for use in a method for treating and/or preventing Alzheimer's disease, the method comprising orally administering the compound.

In a further specific embodiment, the present invention also provides a pharmaceutical composition for use in a method for treating and/or preventing Alzheimer's disease, the method comprising orally administering the pharmaceutical composition, the pharmaceutical composition comprising: a compound that (a) is 1-(2-(2-(*tert-*butyl)phenoxy)pyridin-3-yl)-3-(4-(trifluoromethoxy)phenyl)urea, or (b) is a tautomer, pharmaceutically acceptable salt or solvate thereof; in association with one or more pharmaceutically acceptable carriers.

The present invention also provides a solid dosage form for oral administration comprising: a compound that (a) is 1-(2-(2-(*tert*-butyl)phenoxy)pyridin-3-yl)-3-(4-(trifluoromethoxy)phenyl)urea, or (b) is a tautomer, pharmaceutically acceptable salt or solvate thereof; in association with one or more pharmaceutically acceptable carriers.

The present invention also provides a liquid dosage form for oral administration comprising: a compound that (a) is 1-(2-(2-(*tert*-butyl)phenoxy)pyridin-3-yl)-3-(4-(trifluoromethoxy)phenyl)urea, or (b) is a tautomer, pharmaceutically acceptable salt or solvate thereof; in association with one or more pharmaceutically acceptable carriers.

### Brief description of the drawings

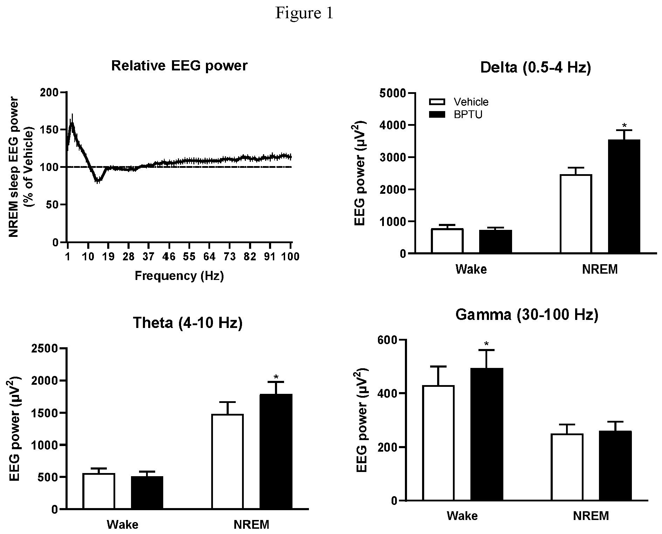

Figure 1 shows changes in EEG power induced by BPTU as measured in Example 2.

### Definitions

Compounds for use of the invention may have asymmetric centers. Compounds for use of the invention containing an asymmetrically substituted atom may be isolated in optically active or racemic forms. It is well known in the art how to prepare optically active forms, such as by resolution of racemic forms or by synthesis from optically active starting materials. Geometric isomers of double bonds such as olefins and C=N double bonds can also be present in the compounds described herein, and all such stable isomers are contemplated. Cis and trans geometric isomers of the compounds are described and may be isolated as a mixture of isomers or as separated isomeric forms. All chiral, diastereomeric, racemic forms and all geometric isomeric forms of a structure are intended, unless the specific stereochemistry or isomeric form is specifically indicated. Hence, the compounds for use of the invention include all possible chiral, diastereomeric and racemic forms thereof and all possible geometric isomers thereof. When no specific mention is made of the configuration (*cis, trans,* R, S, etc.) of a compound (or of an asymmetric carbon), then any one of the isomers or a mixture of more than one isomer is intended. Limitation to a particular asymmetric form of a compound is only intended where expressly indicated.

The processes for preparation can use racemates, enantiomers, or diastereomers as starting materials. When enantiomeric or diastereomeric products are prepared, they can be separated by conventional methods, for example, by chromatographic or fractional crystallization. The prepared compounds may be in the free or hydrate form.

Compounds for use of the invention encompass both compounds in which: (a) all contained atoms are in their natural isotopic form ("natural isotopic form of the compound"); and (b) compounds in which one or more contained atoms are in a non-natural isotopic form ("unnatural variant isotopic form of the compound"), for instance compounds comprising isotopic replacement, enrichment, or depletion. An unnatural variant isotopic form of the compound may thus contain one or more artificial or uncommon isotopes such as deuterium (²H or D), carbon-11 (¹¹C), carbon-13 (¹³C), carbon-14 (¹⁴C), nitrogen-13 (¹³N), nitrogen-15 (¹⁵N), oxygen-15 (¹⁵O), oxygen-17 (¹⁷O), oxygen-18 (¹⁸O), phosphorus-32 (³²P), sulphur-35 (³⁵S), chlorine-36 (³⁶Cl), chlorine-37 (³⁷Cl), fluorine-18 (¹⁸F) iodine-123 (¹²³I), iodine-125 (¹²⁵I) in one or more atoms or may contain an increased proportion of said isotopes as compared with the proportion that predominates in nature in one or more atoms.

Unnatural variant isotopic forms of the compound comprising radioisotopes may, for example, be used for drug and/or substrate tissue distribution studies. The radioactive isotopes tritium, i.e. ³H, and carbon-14, i.e. ¹⁴C, are particularly useful for this purpose in view of their ease of incorporation and ready means of detection. Unnatural variant isotopic forms which incorporate deuterium i.e. ²H or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as ¹¹C, ¹⁸F, ¹⁵O and ¹³N, and would be useful in Positron Emission Topography (PET) studies for examining substrate receptor occupancy.

The following are definitions of terms used in this specification. The initial definition provided for a group or term herein applies to that group or term throughout the present specification, individually or as part of another group, unless otherwise indicated.

Preferably, the molecular weight of compounds for use of the invention is less than about 500, 550, 600, 650, 700, 750, or 800 grams per mole.

Preferably, the molecular weight is less than about 800 grams per mole. More preferably, the molecular weight is less than about 750 grams per mole. Even more preferably, the molecular weight is less than about 700 grams per mole.

The term "substituted" as used herein, means that any one or more hydrogens on the designated atom is replaced with a selection from the indicated group, provided that the designated atom's normal valency is not exceeded, and that the substitution results in a stable compound. When a substituent is keto (i.e., =O), then 2 hydrogens on the atom are replaced. When a ring system (e.g., carbocyclic or heterocyclic) is said to be substituted with a carbonyl group or a double bond, it is intended that the carbon atom of the carbonyl group or one carbon atom of the double bond be part of (i.e., within) the ring. Ring double bonds, as used herein, are double bonds that are formed between two adjacent ring atoms (e.g., C=C, C=N, or N=N).

When any variable (e.g., R^{2b}, R^{8b}, etc.) occurs more than one time in any constituent or formula for a compound, its definition at each occurrence is independent of its definition at every other occurrence. Thus, for example, if a group is shown to be substituted with 0-3 R^{2b}, then said group may optionally be substituted with up to three R^{2b} groups and R^{2b} at each occurrence is selected independently from the definition of R^{2b}. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

As used herein, "alkyl" or "alkylene" is intended to include both branched and straight-chain saturated aliphatic hydrocarbon groups having the specified number of carbon atoms. For example, "C₁-C₁₀ alkyl" (or alkylene), is intended to include C₁, C₂, C₃, C₄, C₅, C₆, C₇, C₈, C₉, and C₁₀ alkyl groups. Additionally, for example, "C₁-C₆ alkyl" denotes alkyl having 1 to 6 carbon atoms. Examples of alkyl include, but are not limited to, methyl, ethyl, n-propyl, i-propyl, n-butyl, i-butyl, sec-butyl, t-butyl, n-pentyl, n-hexyl, 2-methylbutyl, 2-methylpentyl, 2-ethylbutyl, 3-methylpentyl, and 4-methylpentyl.

"Alkoxy" or "alkyloxy" represents an alkyl group as defined above with the indicated number of carbon atoms attached through an oxygen bridge. For example, "C₁-C₆ alkoxy" (or alkyloxy), is intended to include C₁, C₂, C₃, C₄, C₅, and C₆ alkoxy groups (and can also be written equivalently as -O-C₁₋₆ alkyl). Examples of alkoxy include, but are not limited to, methoxy, ethoxy, n-propoxy, i-propoxy, n-butoxy, s-butoxy, t-butoxy, n-pentoxy, and s-pentoxy.

As used herein, "carbocycle" refers to a monocyclic ring or polycyclic (e.g., bicyclic or tricyclic) ring system whose ring atoms consist of carbon atoms.

A polycyclic carbocycle may comprise fused rings (where a first ring and a second ring share two adjacent ring carbon atoms), bridged rings (where two non-adjacent ring carbon atoms of a first ring are shared with a second ring) and/or spirocyclic rings (where a first ring shares only a single ring carbon atom with a second ring). A tricyclic or higher order polycyclic carbocycle may comprise a mixture of fused, bridged and spirocyclic relationships between its constituent rings. A bicyclic carbocycle is one of fused, bridged and spirocyclic.

The carbocycle may be saturated (also referred to herein as cycloalkyl), partially unsaturated, or aromatic (also referred to herein as aryl).

The term "cycloalkyl" refers to a carbocycle in which all ring bonds between adjacent carbon atoms are single bonds (i.e. a saturated ring system). A typical cycloalkyl is C₃₋₁₀ cycloalkyl, which is intended to include C₃, C₄, C₅, C₆, C₇, C₈, C₉ and C₁₀ cycloalkyl groups. Example cycloalkyl groups include, but are not limited to, cyclopropyl, cyclobutyl, cyclopentyl, cyclohexyl, norbomyl, and the like.

Examples of carbocycles include C₃₋₁₀ carbocycles, such as monocyclic or bicyclic (including fused, bridged and spirocyclic where chemically possible in view of the number of carbon ring atoms) carbocycles, further including C₃₋₇ carbocycles, C₃₋₆ carbocycles, C₃₋₅ carbocycles and C₃₋₄ carbocycles.

Examples of carbocycles include, but are not limited to, cyclopropyl, cyclobutyl, cyclopentyl, cyclohexyl, cycloheptyl, adamantyl, cyclooctyl, [3.3.0]bicyclooctane, [4.3.0] bicyclononane, [4.4.0] bicyclodecane (decalin), [2.2.2] bicyclooctane, fluorenyl, phenyl, naphthyl, dihydronaphthyl, 1,2,3,4- tetrahydronaphthyl, indanyl, adamantyl, or tetrahydronaphthyl (tetralin). Preferred carbocycles, unless otherwise specified, are cyclopropyl, cyclobutyl, cyclopentyl, cyclohexyl, phenyl, naphthyl, and indanyl. When the term "carbocycle" is used, it is intended to include "aryl".

As used herein, the term "aryl" is intended to mean an aromatic moiety containing, if specified, the specified number of carbon atoms; for example phenyl or naphthyl.

As used herein, "heterocycle" or "heterocyclic" refers to a monocyclic ring or polycyclic (e.g., bicyclic or tricyclic) ring system whose ring atoms consist of carbon atoms and at least one heteroatom.

A polycyclic heterocycle may comprise fused rings (where a first ring and a second ring share two adjacent ring atoms), bridged rings (where two non-adjacent ring atoms of a first ring are shared with a second ring) and/or spirocyclic rings (where a first ring shares only a single ring atom with a second ring). A tricyclic or higher order polycyclic heterocycle may comprise a mixture of fused, bridged and spirocyclic relationships between its constituent rings. A bicyclic heterocycle is one of fused, bridged and spirocyclic. The heterocycle may be saturated, partially unsaturated, or aromatic (also referred to herein as heteroaryl).

The heterocycle may be saturated, partially unsaturated, or aromatic. When the term "heterocycle" is used, it is intended to include heteroaryl. The term "heterocycle" also includes cycloheteroalkyl.

The heterocycle contains at least one heteroatom ring atom. Typically, heteroatoms are selected from N, NR⁵, O and S(O)ₚ, where p and R⁵ are defined elsewhere herein. The at least one heteroatom is commonly 1-4 heteroatoms, for instance 1-3 heteroatoms, 1-2 heteroatoms or 1 heteroatom. It is preferred that when the total number of S(O)ₚ and O ring atoms in the heterocycle exceeds 1, then these heteroatoms are not adjacent to one another. It is preferred that the total number of S(O)ₚ and O ring atoms in the heterocycle is not more than 1.

Typically the heterocycle contains, in addition to heteroatom ring atoms, at least 1 carbon ring atom, preferably at least two ring atoms. In polycyclic heterocycles, typically each ring contains at least 1 carbon atom. In polycyclic heterocycles at least one ring of the polycyclic ring system contains at least one heteroatom ring atom (for avoidance of doubt, it is not necessary for each ring of a polycyclic heterocycles to contain at least one heteroatom).

Typical examples of heterocycles include 5- to 10-membered heterocycles, wherein "X-membered" means that the total number of ring atoms possessed by the heterocycle is X.

Examples of heterocycles include, but are not limited to, pyridyl, pyrimidinyl, pyrazinyl, pyridazinyl, triazinyl, furyl, quinolyl, isoquinolyl, thienyl, imidazolyl, thiazolyl, indolyl, pyrryl, oxazolyl, benzofuryl, benzothienyl, benzthiazolyl, isoxazolyl, pyrazolyl, triazolyl, tetrazolyl, indazolyl, isothiazolyl, purinyl, carbazolyl, benzimidazolyl, 2,3-dihydrobenzofuranyl, 2,3-dihydrobenzothienyl, 2,3-dihydrobenzothienyl-S-oxide, 2,3-dihydrobenzothienyl-S-dioxide, benzoxazolin-2-on-yl, indolinyl, benzodioxolanyl, benzodioxane, 2-pyrrolidonyl, 2H,6H-1,5,2-dithiazinyl, 2H-pyrrolyl, 3H-indolyl, 4-piperidonyl, 4aH-carbazole, 4H-quinolizinyl, 6H-1,2,5-thiadiazinyl, acridinyl, azocinyl, benzofuranyl, benzothiofuranyl, benzothiophenyl, benzoxazolyl, benzoxazolinyl, benztriazolyl, benztetrazolyl, benzisoxazolyl, benzisothiazolyl, benzimidazalonyl, 4aH-carbazolyl, b-carbolinyl, chromanyl, chromenyl, cinnolinyl, decahydroquinolinyl, 2H,6H-1,5,2-dithiazinyl, dihydrofuro[2,3-b]tetrahydrofuran, furanyl, furazanyl, imidazolidinyl, imidazolinyl, imidazolopyridinyl, 1*H*-indazolyl, indolenyl, indolizinyl, isatinoyl, isobenzofuranyl, isochromanyl, isoindazolyl, isoindolinyl, isoindolyl, isoquinolinyl, isothiazolopyridinyl, isoxazolopyridinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolopyridinyl, oxazolidinylperimidinyl, oxindolyl, phenanthridinyl, phenanthrolinyl, phenarsazinyl, phenazinyl, phenothiazinyl, phenoxathiinyl, phenoxazinyl, phthalazinyl, piperazinyl, piperidinyl, piperidonyl, pteridinyl, pyranyl, pyrazolidinyl, pyrazolinyl, pyrazolopyridinyl, pyridooxazole, pyridoimidazole, pyridothiazole, pyridinyl, pyrrolidinyl, pyrrolinyl, pyrrolyl, quinazolinyl, quinolinyl, 4*H*-quinolizinyl, quinoxalinyl, quinuclidinyl, carbolinyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolopyridinyl, thienothiazolyl, thienooxazolyl, thienoimidazolyl, thiophenyl, 1,2,3-triazolyl, 1,2,4-triazolyl, 1,2,5-triazolyl, 1,3,4-triazolyl, xanthenyl, tetrahydrothiophenyl, tetrahydropyranyl, and morpholinyl.

Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrryl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, 2,3-dihydrobenzofuranyl, 2,3-dihydrobenzothienyl, 2,3-dihydrobenzothienyl-S-oxide, 2,3-dihydrobenzothienyl-S-dioxide, benzoxazolin-2-on-yl, indolinyl, benzodioxolanyl, benzodioxane, and the like.

Non-limiting examples of exemplary heterocycles include: (a) pyridinyl, pyrimidinyl, furanyl, isoxazolyl, thiazolyl, thiadiazolyl, benzothiazolyl, thiazolopyridinyl, indolyl, benzodioxolyl, and benzimidazolyl (b) pyridinyl, pyrimidinyl, thiadiazolyl, thiazolopyridinyl, benzodioxolyl, and benzimidazolyl; and (c) thiazolopyridinyl.

"Alkenyl" or "alkenylene" is intended to include hydrocarbon chains of either a straight or branched configuration having the specified number of carbon atoms and one or more unsaturated carbon-carbon bonds which may occur in any stable point along the chain. For example, "C₂-C₆ alkenyl" (or alkenylene), is intended to include C₂, C₃, C₄, C₅, and C₆ alkenyl groups. Examples of alkenyl include, but are not limited to, ethenyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2- methyl-2-propenyl, 4-methyl-3-pentenyl, and the like.

"Alkynyl" or "alkynylene" is intended to include hydrocarbon chains of either a straight or branched configuration and one or more carbon-carbon triple bonds which may occur in any stable point along the chain. For example, "C₂-C₆ alkynyl" (or alkynylene), is intended to include C₂, C₃, C₄, C₅, and C₆ alkynyl groups; such as ethynyl, propynyl, butynyl, pentynyl, hexynyl and the like.

The term "cycloheteroalkyl" refers to cyloalkyl where one or more of the carbon atoms in the ring has been substituted, independently, with any oxygen, sulfur or nitrogen atom, such as pyrolidine, piperidine, tetrahydrofuran, tetrahydrothiophene, tetrahydropyran, morpholine, and the like.

The term "alkylthio" represents an alkyl group as defined above with the indicated number of carbon atoms attached through a sulfur (-S-) bridge; for example methyl-S-, ethyl-S-, and the like.

"Halo" or "halogen" as used herein refers to fluoro, chloro, bromo, and iodo (F, Cl, Br, and I).

"Haloalkyl" is intended to include both branched and straight-chain saturated aliphatic hydrocarbon groups having the specified number of carbon atoms, substituted with 1 or more halogen (for example -CᵥF_{w}H_{2v+1-w} where v = 1 to 3 and w = 1 to (2v+1)). Examples of haloalkyl include, but are not limited to, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" which is intended to include both branched and straight-chain saturated aliphatic hydrocarbon groups having the specified number of carbon atoms, substituted with 1 or more fluorine atoms.

"Haloalkoxy" or "haloalkyloxy" represents a haloalkyl group as defined above with the indicated number of carbon atoms attached through an oxygen (-O-) bridge. For example, "C₁-C₆ haloalkoxy", is intended to include C₁, C₂, C₃, C₄, C₅, and C₆ haloalkoxy groups. Examples of haloalkoxy include, but are not limited to, trifluoromethoxy, 2,2,2-trifluoroethoxy, pentafluoroethoxy, and the like.

The term "optionally substituted" as used herein, without a further qualification as to the nature of the substituents (e.g. "optionally substituted phenyl") means that one or more substituents can be present such that the resulting compound retains both efficacy and pharmaceutical acceptability, and for instance can preferably mean an optional substitution of one to three, preferably one or two, groups independently selected from halo, hydroxyl, cyano, nitro, C₁-C₄ alkyl, and C₁-C₄ alkoxy.

The phrase "pharmaceutically acceptable" is employed herein to refer to those compounds, materials, compositions, and/or dosage forms which are, within the scope of sound medical judgment, suitable for use in contact with the tissues of human beings and animals, especially human beings, without excessive toxicity, irritation, allergic response, or other problem or complication, commensurate with a reasonable benefit/risk ratio.

The compounds for use of the invention encompass pharmaceutically acceptable salts (in particular, pharmaceutically acceptable salts of compounds of a specified general formula, for instance formula (I), as well as solvates, N-oxides and tautomers thereof). As used herein, "pharmaceutically acceptable salts" refer to derivatives of the disclosed compounds wherein the parent compound (e.g., of formula (I)) is modified by making pharmaceutically acceptable acid or base salts thereof. Examples of pharmaceutically acceptable salts include, but are not limited to, mineral or organic acid salts of basic groups such as amines; and alkali or organic salts of acidic groups such as carboxylic acids. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic, and the like.

The pharmaceutically acceptable salts can be synthesized from the parent compound (e.g., of formula (I)) which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p.1418, the disclosure of which is hereby incorporated by reference.

Non-pharmaceutically acceptable salt forms of the compounds for use of the invention may be of use during the preparation of non-salt or pharmaceutically acceptable salt forms.

The compounds for use of the invention encompass solvates (in particular, solvates of compounds of a specified general formula, for instance formula (I), as well as salts, N-oxides and tautomers thereof). Solvates include, and preferably are, hydrates. Methods of solvation are generally known in the art.

The compounds for use of the invention encompass tautomers (in particular, tautomers of compounds of a specified general formula, for instance formula (I), as well as salts, solvates and N-oxides thereof). Some compounds for use of the invention may exist in a plurality of tautomeric forms, in which hydrogen atoms are transposed to other parts of the molecules and the chemical bonds between the atoms of the molecules are consequently rearranged. It should be understood that all tautomeric forms, insofar as they may exist, are encompassed by the compounds for use of the invention.

In cases wherein there are nitrogen atoms (e.g., amines) on compounds for use of the invention (e.g. of formula (I)), these can be converted to N-oxides by treatment with an oxidizing agent (e.g., MCPBA and/or hydrogen peroxides) to afford other compounds for use of this invention. Thus, all shown nitrogen atoms are considered to cover both the shown nitrogen and its N-oxide (N→O) derivative. The compounds for use of the invention encompass N-oxides (in particular, N-oxides of compounds of a specified general formula, for instance formula (I), as well as salts, solvates and tautomers thereof).

For avoidance of doubt, many compounds for use of the invention can exist simultaneously in the form of two or more of a tautomer, N-oxide, pharmaceutically acceptable salt, and solvate of a particular parent compound (e.g. a compound of formula (I)), and all such possible compounds are encompassed within the definition of "compound for use of the invention". Hence, a compound for use of the invention (to the extent chemically possible in view of the relevant specified formula (e.g. formula (I)) expressly includes any one of the following: (1) a compound of the specified formula (e.g. formula (I)) (which may also be referred to herein as a "free base form" of the compound); (2) a tautomer of the specified formula (e.g. formula (I)); (3) an N-oxide of the specified formula (e.g. formula (I)); (4) a pharmaceutically acceptable salt of the specified formula (e.g. formula (I)); (5) a solvate of the specified formula (e.g. formula (I)); (6) an N-oxide of a tautomer of the specified formula (e.g. formula (I)); (7) a pharmaceutically acceptable salt of a tautomer of the specified formula (e.g. formula (I)); (8) a solvate of a tautomer of the specified formula (e.g. formula (I)); (9) a pharmaceutically acceptable salt of an N-oxide of a tautomer of the specified formula (e.g. formula (I)); (10) a solvate of an N-oxide of a tautomer of the specified formula (e.g. formula (I)); (11) a solvate of a pharmaceutically acceptable salt of a tautomer of the specified formula (e.g. formula (I)); (12) a solvate of a pharmaceutically acceptable salt of an N-oxide of a tautomer of the specified formula (e.g. formula (I)); (13) a pharmaceutically acceptable salt of an N-oxide of the specified formula (e.g. formula (I)); (14) a solvate of an N-oxide of the specified formula (e.g. formula (I)); (15) a solvate of a pharmaceutically acceptable salt of an N-oxide of a tautomer of the specified formula (e.g. formula (I)); and (16) a solvate of a pharmaceutically acceptable salt of the specified formula (e.g. formula (I)).

Compounds of the invention are, subsequent to their preparation, preferably isolated and purified to obtain a composition containing an amount by weight equal to or greater than 99% compound ("substantially pure"), which is then used or formulated as described herein. Such "substantially pure" compounds are also contemplated herein as compounds for use of the invention.

"Stable compound" and "stable structure" are meant to indicate a compound that is sufficiently robust to survive isolation to a useful degree of purity from a reaction mixture, and formulation into an efficacious therapeutic agent. It is preferred that compounds for use of the invention do not contain a N-halo, S(O)₂H, or S(O)H.

Purely for the avoidance of doubt, it should be noted that wherever a compound is disclosed as being either a particular structure (e.g. a particular general chemical formula, such as of formula (I) or another formula defined herein) or a tautomer, N-oxide, pharmaceutically acceptable salt, or solvate thereof, then, unless expressly indicated to the contrary, any disclosed further embodiment of that compound (e.g. "A compound ... according to ..."), and describing additional limitations to the compound structure, continues to embrace any such tautomer, N-oxide, pharmaceutically acceptable salt, or solvate thereof.

As used herein, "treating and/or preventing" or "treatment and/or prevention" of a disease-state in a mammal, particularly a human, include: (a) preventing the disease-state from occurring in a mammal, in particular, when such mammal is predisposed to the disease-state but has not yet been diagnosed as having it; (b) inhibiting the disease-state, i.e., slowing or arresting its development; and/or (c) relieving the disease-state, i.e., causing regression of the disease state or a reduction in associated symptoms.

"Therapeutically effective amount" is intended to include an amount of a compound that is effective to achieve a desirable effect in treating and/or preventing a disease-state. A desirable effect is typically clinically significant and/or measurable, for instance in the context of (a) preventing the disease-state from occurring in a mammal, in particular, when such mammal is predisposed to the disease-state but has not yet been diagnosed as having it; (b) inhibiting the disease-state, i.e., slowing or arresting its development; and/or (c) relieving the disease-state, i.e., causing regression of the disease state or a reduction in associated symptoms. The therapeutically effective amount may be one that is sufficient to achieve the desirable effect either when the compound is administered alone, or alternatively when it is administered in combination with one or more further APIs, which either are further compounds for use of the invention or are different from the compounds for use of the invention. Furthermore, a therapeutically effective amount is typically an amount that is sufficient to inhibit P2Y1 receptors, preferably P2Y1 receptors in the CNS, again when administered either alone or in combination with one or more further APIs (which may also target P2Y1 receptors, or alternatively may exert their pharmacological effects by a different mechanism). Thus, "therapeutically effective amount" is intended to include an amount of a combination of compounds that each are compounds for use of the invention that is effective to inhibit P2Y1 receptors, preferably P2Y1 receptors in the CNS. The combination of compounds is preferably a synergistic combination.

Synergy, as described, for example, by Chou and Talalay, Adv. Enzyme Regul. 1984, 22: 27-55, occurs when the effect (in this case, inhibition of P2Y1) of the compounds when administered in combination is greater than the additive effect of the compounds when administered alone as a single agent. In general, a synergistic effect is most clearly demonstrated at sub-optimal concentrations of the compounds. Synergy can be in terms of lower cytotoxicity, or some other beneficial effect of the combination compared with the individual components.

For avoidance of doubt, a "therapeutically effective amount" as recited herein can be achieved by any suitable dosage regimen, including but not limited to exemplary dosage regimens described elsewhere herein. Hence, for example, references herein to administering a therapeutically effective amount of a compound by a particular administration route including achieving the therapeutically effective amount via a single dose or by plural doses administered by the specified administration route. For instance, orally administering a therapeutically effective amount includes both orally administering a single dose and orally administering any plural number of doses, provided that a therapeutically effective amount is thereby achieved by oral administration.

The present pharmaceutical compositions are compositions comprising one or more compounds for use of the invention and a pharmaceutically acceptable carrier.

A "pharmaceutically acceptable carrier" refers to media generally accepted in the art for the delivery of biologically active agents to animals, in particular, mammals. Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and, the therapeutic indication being targeted. Pharmaceutically acceptable carriers include both aqueous and non-aqueous liquid media, as well as a variety of solid and semi- solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Remington's Pharmaceutical Sciences, 17th ed., 1985, which is incorporated herein by reference in its entirety.

### Detailed description of the invention

### Compounds

The compounds for use of the invention are as defined in the detailed aspects 1 to 93. Further preferred sub-aspects of the compounds for use of the invention are provided below.

Preferably, in the formula (Ia) of aspect 12, ring A is:

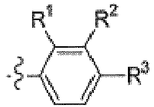

Further preferably, in the formula (Ia) of aspect 12, ring B is:

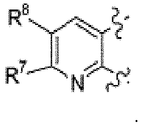

Further preferably, in the formula (Ia) of aspect 12, Y is O.

Preferably, in the compound for use according to any one of aspects 34 to 37, where ring B is a 6-membered heteroaryl substituted with 1 R⁷ or C₆ aryl substituted with 1 R⁷, it is unsubstituted at the 4-position relative to the -NH-C(=W)-NH- linkage.

Preferably, in the compound for use according to any one of aspects 34 to 37, where ring B is a 6-membered heteroaryl substituted with 1 R⁷ or C₆ aryl substituted with 1 R⁷, it is substituted at the 2-position relative to the -NH-C(=W)-NH- linkage.

Preferably, in the compound for use according to any one of aspects 34 to 36, ring B is not:

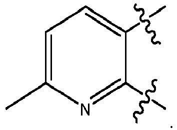

Preferably, the compound for use of the invention is not 1-(2-(2-(tert-butyl)phenoxy)-6-methylpyridin-3-yl)-3-(4-(trifluoromethoxy)phenyl)urea, nor a tautomer, N-oxide, pharmaceutically acceptable salt, or solvate thereof.

Further preferably, the compound for use of the invention is not a compound selected from the group: 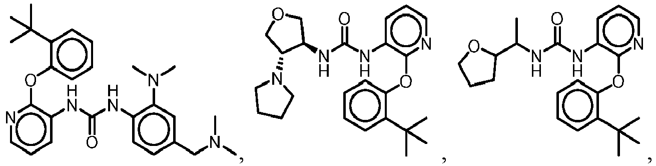 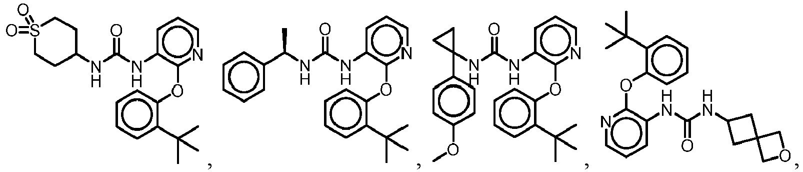 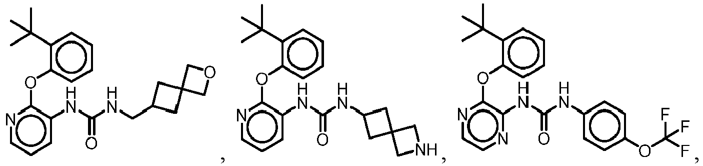 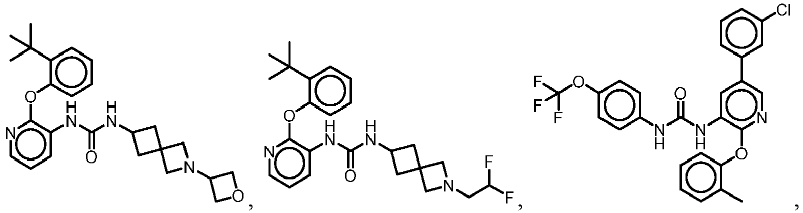 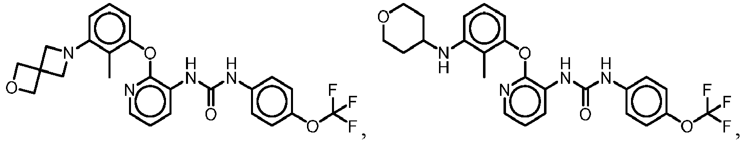 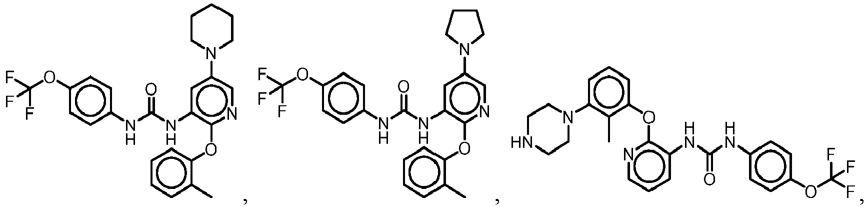 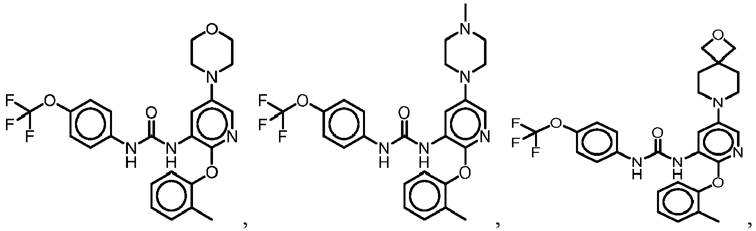 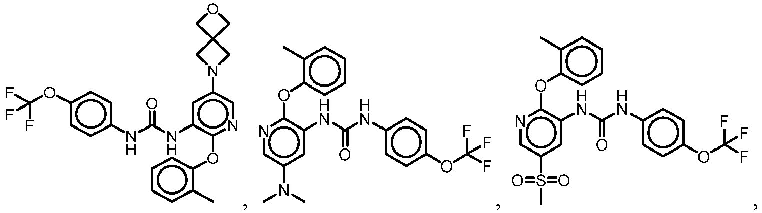 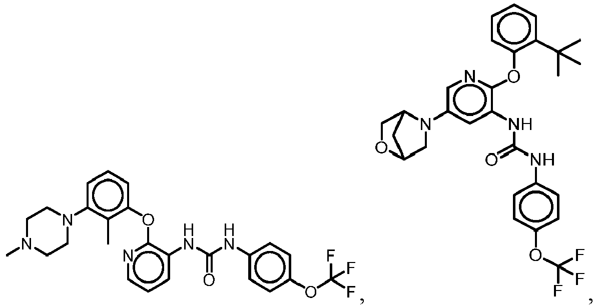 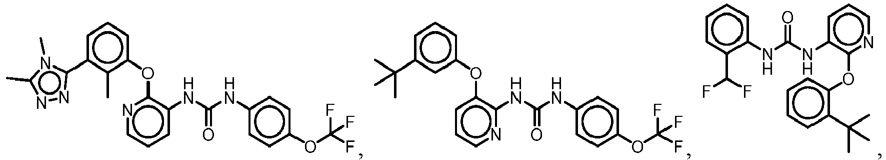 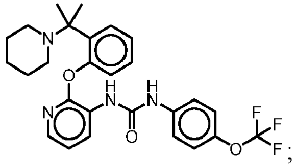 nor a tautomer, N-oxide, pharmaceutically acceptable salt, or solvate thereof.

Compounds of aspects 1 to 19 are described in detail in WO 2005/113511, incorporated herein by reference.

Compounds of aspects 66 to 83 are described in detail in WO 2008/023235, incorporated herein by reference.

### CNS penetration

The compounds disclosed herein are typically capable of penetrating the CNS, e.g. following administration to a subject to a location other than the CNS. Hence, when used according to the invention, the compounds can typically exert a desired therapeutic effect without the need for administration directly to the CNS. Such a compound is also referred to herein as a "CNS penetrant". Most generally, a CNS penetrant is a compound that penetrates the CNS after administration to a location other than the CNS (e.g., in a therapeutically significant concentration, e.g. to thereby exert a therapeutically relevant effect).

In one preferred aspect, a compound is a CNS penetrant when the ratio of the concentration of the compound in the brain to the concentration of the compound in the plasma of a test subject (for instance, a mouse, such as a C57B16/J mouse) is at least 0.3 (preferably at least 0.5, further preferably at least 0.7, most preferably at least 1.0) at a time of from 2-4 hours following administration (preferably at a time of 2 hours following administration).

It is particularly preferred that the concentration of the compound in the brain and the concentration of the compound in the plasma be obtained by the following procedure:
- formulate compound in a vehicle;
- administer the compound to a mouse;
- cull the mouse at 2 hours following dosing, collect brain and plasma;
- prepare plasma samples for bioanalysis;
- prepare brain tissue samples for bioanalysis by homogenisation;
- determine the concentration of the compound in each of the brain tissue and plasma samples.

It is still more preferred that the concentration of the compound in the brain and the concentration of the compound in the plasma be obtained by the following procedure:
- construct a LC-MS/MS standard curve for the compound, having a range of 0.5ng/mL to 2000ng/mL
- formulate compound in a vehicle comprising DMSO, Kolliphor EL and HPCD (20% w/v) to a final dose of 0.4mg/kg;
- administer the compound via an intravenous route to a C57B16/J mouse, optionally with up to four other compounds for analysis;
- cull the mouse at 2 hours following dosing, collect brain and plasma;
- prepare plasma samples for bioanalysis in a 1:10 dilution in plasma;
- prepare brain tissue samples for bioanalysis by homogenizing the brain in 0.1M phosphate buffer solution at a ratio of 1:4;
- add internal standard to the samples and then centrifuge for 30 minutes at 4000rpm;
- transfer supernatant, dilute with an equal volume of water and mixed thoroughly;
- analyse the supernatant samples by LC-MS/MS analysis;
- using the standard curve, convert the test peak area of the compound in each of the samples, as obtained by the LC-MS/MS analysis, into a concentration;
- determine whether the compound is a CNS penetrant by assessing if the brain to plasma ratio is 0.3 or greater (preferably at least 0.5, further preferably at least 0.7, most preferably at least 1.0).

Further preferably, the compounds for use in the invention are CNS penetrants according to the method disclosed in Reference Example 1.

### Dosage and formulation

The compounds for use of the invention can be administered alone, but generally will be administered with a pharmaceutical carrier selected on the basis of the chosen route of administration and standard pharmaceutical practice. Thus, preferably, the compounds for use of the invention can be included in a pharmaceutical composition for use as defined in aspect 94.

Preferably, the compounds or pharmaceutical composition for use according to the invention are for use in a method of treatment comprising administering a therapeutically effective amount of the compound or pharmaceutical composition, and particularly preferably a method of treatment comprising orally administering a therapeutically effective amount of the compound or pharmaceutical composition (as defined, for instance, in aspects 97 and 98). Thus, the compounds for use in the invention are preferably administered in pharmaceutical compositions that are solid dosage forms for oral administration (such as tablets, capsules (each of which includes sustained release or timed release formulations), pills, powders, or granules) or liquid dosage forms for oral administration (such as, elixirs, tinctures, suspensions, syrups, and emulsions), as defined in aspects 117 and 118.

However, the compounds for use in the invention may also be administered in intravenous (bolus or infusion), intraperitoneal, subcutaneous, or intramuscular form, all using dosage forms well known to those of ordinary skill in the pharmaceutical arts.

The dosage regimen for the compounds for use of the invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. A physician or veterinarian can determine and prescribe the effective amount of the drug required to prevent, counter, or arrest the progress of the thromboembolic disorder.

By way of general guidance, the daily oral dosage of each active ingredient, when used for the indicated effects, will range between about 0.001 to 1000 mg/kg of body weight, preferably between about 0.01 to 100 mg/kg of body weight per day, and most preferably between about 1.0 to 20 mg/kg/day.

Intravenously, the most preferred doses will range from about 1 to about 10 mg/kg/minute during a constant rate infusion. Compounds for use of the invention may be administered in a single daily dose, or the total daily dosage may be administered in divided doses of two, three, or four times daily.

Compounds for use of the invention can be administered in intranasal form via topical use of suitable intranasal vehicles, or via transdermal routes, using transdermal skin patches. When administered in the form of a transdermal delivery system, the dosage administration will, of course, be continuous rather than intermittent throughout the dosage regimen.

The compounds are typically administered in admixture with suitable pharmaceutical diluents, excipients, or carriers (collectively referred to herein as pharmaceutical carriers) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and coloring agents can also be incorporated into the mixture. Suitable binders include starch, gelatin, natural sugars such as glucose or beta-lactose, com sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

The compounds for use of the invention can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

Compounds for use of the invention may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxide-polylysine substituted with palmitoyl residues. Furthermore, the compounds for use of the invention may be coupled to a class of biodegradable polymers useful in achieving controlled release of a drug, for example, polylacetic acid, polyglycolic acid, copolymers of polylacetic and polyglycolic acid, polyepsilon caprolactone, polyhydroxy butyric acid, polyorthoesters, polyacetals, polydihydropyrans, polycyanoacylates, and crosslinked or amphipathic block copolymers of hydrogels.

Dosage forms (pharmaceutical compositions) suitable for administration may contain from about 1 milligram to about 100 milligrams of active ingredient per dosage unit. In these pharmaceutical compositions the active ingredient will ordinarily be present in an amount of about 0.5-95% by weight based on the total weight of the composition.

Gelatin capsules may contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl-or propyl-paraben, and chlorobutanol.

Suitable pharmaceutical carriers are described in Remington's Pharmaceutical Sciences, Mack Publishing Company, a standard reference text in this field.

### Compound/composition for in method for treating and/or preventing a CNS disorder

In embodiments of the invention, the compound or pharmaceutical composition is for use in a method for treating and/or preventing a CNS disorder, as defined in aspects 1-94 and 97-116.

### CNS disorders

Typically, the CNS disorder to be treated and/or prevented in the present invention is a CNS disorder susceptible to treatment by P2Y1 inhibition.

Further preferably, the CNS disorder to be treated and/or prevented is selected from Alzheimer's disease, pain, epilepsy, stroke, traumatic brain injury, amyotrophic lateral sclerosis, Huntington's disease, Parkinson's disease and frontotemporal dementia.

Further preferably, the CNS disorder to be treated and/or prevented is selected from Alzheimer's disease, pain, epilepsy, stroke, traumatic brain injury and amyotrophic lateral sclerosis.

Further preferably still, the CNS disorder to be treated and/or prevented is selected from Alzheimer's disease, pain, epilepsy and stroke.

In one (particularly) preferred aspect of the present invention, the CNS disorder to be treated and/prevented is Alzheimer's disease. The Alzheimer's disease may be either early-onset or late-onset Alzheimer's disease.

Alzheimer's disease is a dementia-causing neurodegenerative disease. It is an irreversible, progressive brain disorder that causes a slow decline in memory, thinking and reasoning skills before ultimately leading to death. The disease pathology is associated with the formation of abnormal protein agglomerations in the brain tissue known as "plaques" and "tangles" which disrupt neuron functioning, lead to the loss of neuronal connections, and ultimately lead to neuron death.

A number of treatments are available to improve the symptoms of Alzheimer's disease, namely treatment with acetylcholinesterase (AChE) inhibitors (such as donepezil, galantamine and rivastigmine) or with memantine. However, new treatments for Alzheimer's disease would be highly desirable.

Pathological astrocytic network activity (intracellular calcium waves and single-cell astrocyte hyperactivity) is observed in Alzheimer's disease. Recent reports have connected such pathological activity with purinergic signaling (Delekate A, et al. Nat. Commun., 2014;5:5422). Such studies have identified that the P2Y1 receptor (a purinergic receptor) is particularly strongly expressed in the reactive astrocytes surrounding plaques, and have demonstrated that P2Y1 inhibition is capable of normalizing astrocyte activity. The studies further suggest that such that P2Y1 inhibition may represent a potential therapeutic strategy for ameliorating inflammation and cognitive decline associated with Alzheimer's disease.

A proof-of-concept study has been carried out to demonstrate the application of P2Y1 inhibition in murine models of Alzheimer's disease (Reichenbach N, et al. J. Exp. Med. 2018;215(6): 1649-1663). This study demonstrated that chronic *intracerebroventricular* infusion of P2Y1 inhibitors normalizes astroglial and neuronal network dysfunction and is capable of protecting against decline of spatial learning and memory.

Intracerebroventricular infusion is an invasive mode of drug delivery, used when a pharmaceutical agent is either empirically known or otherwise believed - due to its physicochemical properties - to be incapable of crossing the blood-brain barrier. Such an administration mode is disadvantageous, not only because of the need for neurosurgical intervention, but also due to problems with the extent to which the active agent is capable of diffusing from the injection site throughout the human brain (Pardridge W M., Alzheimers Dement. 2009;5(5):427-43).

It is consequently particularly desirable to provide a P2Y1 inhibitor for use in a method of treating Alzheimer's disease that is capable of crossing blood-brain barrier, i.e. to provide a P2Y1 inhibitor for use in a method of treating Alzheimer's disease that is a CNS penetrant. This will enable the P2Y1 inhibitor to be administered to the patient in a simple manner (i.e., by oral administration), whilst still being able subsequently to access the CNS.

In another preferred aspect of the invention, the CNS disorder to be treated and/prevented is pain. The pain that is treated and/or prevented may comprise or consist of neuropathic pain. The pain that is treated and/or prevented may comprise or consist of nociceptive pain.

In another preferred aspect of the invention, the CNS disorder to be treated and/or prevented is epilepsy.

In another preferred aspect of the invention, the CNS disorder to be treated and/or prevented is stroke. The treatment and/or prevention of stroke according to the present invention is typically achieved by neuroprotection within the CNS (e.g., by inhibition of P2Y1 receptors expressed on astrocytes), rather than through the treatment and/or prevention of thromboembolism-induced stroke mediated via blood clotting.

In another preferred aspect of the invention, the CNS disorder to be treated and/or prevented is traumatic brain injury.

In another preferred aspect of the invention, the CNS disorder to be treated and/or prevented is amyotrophic lateral sclerosis.

### Use for manufacture of a medicament

The present invention provides the use of a compound or pharmaceutical composition, for the manufacture of a medicament for use in a method for treating and/or preventing a CNS disorder as defined in aspects 95 and 97 to 116.

It will be understood that all preferred disclosure provided herein in connection with the compound or pharmaceutical composition for use according to the invention is equally contemplated as preferred in the context of the use of a compound or pharmaceutical composition for the manufacture of a medicament for use according to the invention.

### Method of treatment

The present invention provides a method of treating and/or preventing a CNS disorder according to the invention as defined in aspects 96 to 116.

It will be understood that all preferred disclosure provided herein in connection with the compound or pharmaceutical composition for use according to the invention is equally contemplated as preferred in the context of the method of treatment and/or prevention according to the invention.

### Examples

### Reference Example 1

A protocol for determining CNS penetration (i.e. to determine whether a test compound is a "CNS penetrant") is as follows:
- construct a LC-MS/MS standard curve for the compound, having a range of 0.5ng/mL to 2000ng/mL
- formulate compound in a vehicle comprising DMSO, Kolliphor EL and HPCD (20% w/v) to a final dose of 0.4mg/kg;
- administer the compound via an intravenous route to a C57B16/J mouse, optionally with up to four other compounds for analysis;
- cull the mouse at 2 hours following dosing, collect brain and plasma;
- prepare plasma samples for bioanalysis in a 1:10 dilution in plasma;
- prepare brain tissue samples for bioanalysis by homogenizing the brain in 0.1M phosphate buffer solution at a ratio of 1:4;
- add internal standard to the samples and then centrifuge for 30 minutes at 4000rpm;
- transfer supernatant, dilute with an equal volume of water and mixed thoroughly;
- analyse the supernatant samples by LC-MS/MS analysis;
- using the standard curve, convert the test peak area of the compound in each of the samples, as obtained by the LC-MS/MS analysis, into a concentration;
- determine whether the compound is a CNS penetrant by assessing if the brain to plasma ratio is 0.3 or greater (preferably at least 0.5, further preferably at least 0.7, most preferably at least 1.0).

### Example 1

This example compares the *in silico* predicted CNS penetration properties of selected compounds with their observed CNS penetration properties. Also provided are P2Y1 inhibition data for each compound.

### In silico prediction of CNS penetration

There is a body of modern research aiming to rationalise and predict whether a compound would be CNS penetrant (CNS+). There are a number of predictive models that essentially focus on a core group of molecular and physicochemical properties of the molecules, e.g. size (as measures by mol wt), lipophilicity (clogP, clogD), polarity (TPSA), H-bonding (HBA, HBD), aromatic rings (NAr), rotatable bonds (NRB) and ionisation state (pKa). These models have been constructed through the analysis of large data sets of experimentally measured brain exposures of drug-like molecules in rodent models. These *in silico* models have become adopted as good practice in the design of molecules that require CNS+ as an important feature in their use.

A state-of-the-art model is the CNS multiparameter optimisation (CNS MPO) score (Wager et al., ACS Chem. Neurosci. 2010, 1, 435-449), which combines features of these properties underpinned by a probabilistic analysis (i.e. soft failings are allowed, rather than binary yes/no cut-off). The novel CNS MPO algorithm showed that 74% of marketed CNS drugs display a high CNS MPO score (MPO desirability score ≥ 4, using a scale of 0-6). This analysis suggests that this algorithm could potentially be used to identify compounds with a higher probability of successfully testing hypotheses in the clinic.

However, application of the CNS MPO to BPTU, i.e. the compound 1-(2-(2-(*tert-*butyl)phenoxy)pyridin-3-yl)-3-(4-(trifluoromethoxy)phenyl)urea: 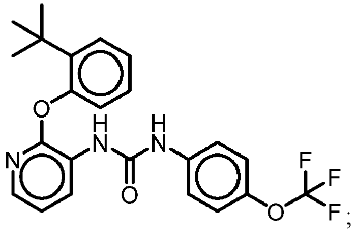 shows a low score, which predicts that it is unlikely to have CNS+:

| **CNS MPO Calculator** | | |
|---|---|---|
| **Property*** | **Value** | **T0** |
| ClogP | 5.2 | 0.000 |
| ClogD | 5.2 | 0.000 |
| TPSA | 72 | 1.000 |
| MW | 445 | 0.393 |
| HBD | 2 | 0.500 |
| pKa | 0** | 1.000 |
| **CNS MPO** | | **2.9** |

| | | |
|---|---|---|
| * *calculated using Star Drop^{™}, Optibrium Ltd. Version: 6.6.3.23027* ***BPTU is a neutral molecule and does not contain a group that would be expected to be ionized at physiologically relevant pH. Hence, the pKa value has been set as zero.* | | |

### Method of assessing CNS penetration

To assess penetration of compounds into the mouse central nervous system, intravenous pharmacokinetic studies were undertaken in C57B16/J mice. In each study, up to five compounds were formulated in a vehicle comprising DMSO, Kolliphor EL and HPCD (20% w/v) to a final dose for each compound of 0.4mg/kg. Compounds were then combined into a single dosing solution and administered together via the intravenous route into 24 mice. Total compound dose administered to each mouse was 2mg/kg. Mice were culled at various times post dosing and brain and plasma collected for bioanalysis. In particular, mice were culled and plasma collected for bioanalysis at 8 time points out to 24 hours post dosing. Brains were also collected for bioanalysis at various time points to assess CNS penetration. 2 hour and 4 hour brain collection was carried out in all studies. Additional time points were collected in selected studies.

For bioanalysis, compounds were prepared in DMSO and aliquoted together to ensure a final concentration of 1mg/mL per compound in DMSO. Analytical standards were prepared from the initial 1mg/mL stock solution of compound, with a 12-point standard curve range from 0.5ng/mL to 2000ng/mL, with independent quality control tests from 1ng/mL to 1000ng/mL run to validate the standards. All standards and QC samples were matrix matched to eliminate possible suppression. Plasma samples were prepared with a 1:10 dilution in plasma. Brain tissue samples were homogenized in 0.1M phosphate buffer solution at a ratio of 1:4, and used without further dilution. Dose solutions were diluted in a control matrix to a relative concentration that fell on the standard curve. Cold acetonitrile containing internal standard was added to all samples, standards, dose samples and QC samples; samples were then centrifuged for 30 minutes at 4000rpm. After centrifugation, supernatant was transferred to a new 96-well plate, diluted with an equal volume of water and mixed thoroughly before LC-MS analysis.

Supernatents were analysed by LC-MS/MS. From the analysis data a response ratio was obtained using the formula Response Ratio = Test Peak Area/Internal Standard Peak Area. From the standard curve, the test peak area was converted to concentration of test compound.

To establish CNS penetration, the ratio of the concentration of each compound in the plasma samples to the brain samples was assessed.

### Measurement of IC50 values

IC50 values were determined using a cell based assay in stably transfected cells overexpressing the P2Y1 receptor. The assay system is a high throughput resonance transfer (HTRF) assay measuring accumulation of the intracellular messenger inositol monophosphate (IP1), generated intracellularly following activation of P2Y1. Activity of test compounds was generated by measuring antagonism of the increase in IP1 generated by addition of a selective P2Y1 agonist, MRS2365.

P2Y1 overexpressing cells were maintained in T175 flasks. The day before the assay, these cells were split and 20µl cell suspension transferred to each well of white 384-well assay plates at a cell density of 0.5x10⁶ cells/mL. Plates were pulse centrifuged, then incubated overnight at 37°C / 5% CO₂ prior to assay.

On day of assay, medium was removed from cells and replaced with stimulation buffer. Compounds were prepared in 100% DMSO for addition to cells using the Tecan D300e digital dispenser; compounds were prepared in cassette heads and added directly to wells in the required concentration range, to give a 10-point concentration response curve from 10µM with semi-log dilutions and a final DMSO concentration of 0.1%. Positive control antagonist curves with standard compounds were included on each plate. Plates were incubated with antagonists for 1 hour prior to addition of 5nM of the agonist MRS2365, dispensed using a Bravo liquid handler. Following agonist addition, plates were incubated for 60 minutes prior to assay. Both incubations were carried out at 37°C / 5% CO₂.

For the assay, IP1 accumulation was quantified using the IPOne kit according to manufacturer's instructions. In short, a standard curve was prepared for IP1 quantification, and cells lysed using kit lysis buffer. Assay reagents were added to the lysates, and incubated for 1 hour at room temperature without spinning or shaking. Plates were analysed on a BMG Pherastar plate reader, and IP1 signal generated in cells interpolated from the standard curve.

### Results

| **Entry** | **Compound** | **IC50^{a} (nM)** | **MPO^{b} (0-6)** | **CNS penetration^{c}** |
|---|---|---|---|---|
| **1^{e}** | 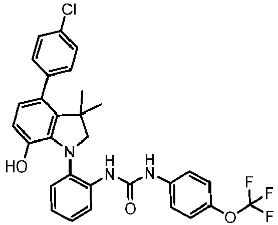 | 33 | 2.2 | BLLQ^{d} |
| **2 (BPTU)** | 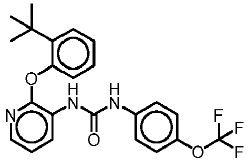 | 35 | 2.9 | 1.5 |
| **3^{f}** | 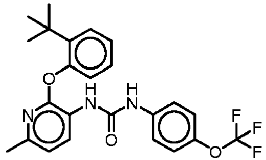 | 36 | 2.8 | BLLQ^{d} |
| **4** | 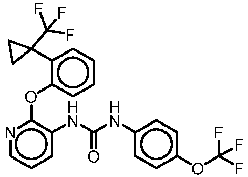 | 58 | 2.6 | 0.53 |
| **5** | 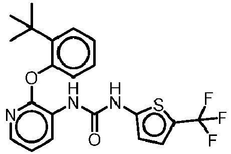 | 121 | 3 | 0.75 |
| **6** | 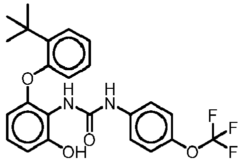 | 150 | 2.5 | 3.1 |

| | | | | |
|---|---|---|---|---|
| ^{a} All values rounded to nearest whole number ^{b} All values presented to two significant figures ^{c}total brain to plasma ratio (mouse) at t = 2 hr; values presented to two significant figures ^{d}BLLQ = below lower limit of quantification ^{e}Reference compound RS1 ^{f}Reference compound RS2 | | | | |

The compounds of entries 2 (BPTU) and 4 to 6 exhibit CNS penetration. In contrast, the compounds of entry 1 (disclosed in WO 2005/113537; referred to herein as reference compound RS1) and entry 3 (a close analogue to BPTU; referred to herein as reference compound RS2) give no apparent CNS penetration.

The compounds of entries 2, 4 to 6 also strongly inhibit P2Y1 as shown by the measured IC50 values. Therefore these representative compounds demonstrate a combination of ability to inhibit P2Y1 and capacity to penetrate the CNS.

### Example 2

Study to assess *in vivo* pharmacodynamic activity of BPTU (S1) in the CNS of rats following oral administration.

### Study schedule:

Pre-surgery: The animals were allowed a minimum period of 7 days acclimatisation on arrival in the facility.

Surgery: Male Sprague-Dawley rats were anaesthetised with isoflurane (2-5% in Oxygen at 11/min) throughout the procedure. The scalp, neck and right flank were cleared of hair using clippers and cleaned using diluted Hibitane (50% (v/v) in water). A non-steroidal anti-inflammatory agent was administered (Carprofen 5mg/kg, s.c.) and the animals placed on a homeothermic blanket (37 °C) with the head fixed in a stereotaxic frame. Incisions were made in the scalp and neck, as well as in the right flank. Blunt dissection of the abdominal muscle was then made to gain access to the peritoneal cavity. A radio transmitter (HD-S02, Data Sciences International) was implanted with the body of the transmitter placed in the peritoneal cavity and secured to the muscle wall with non-absorbable sutures through the body of the transmitter. The wires of the transmitter were passed through the muscle wall and then sub-dermally using a trochar (16G needle) to the scalp to act as EEG/EMG electrodes. The scalp was cleared of connective tissue and two bore holes were made in the skull with a trepanning drill (fronto-parietal coordinates: Bregma +2mm anterior, midline +1.0mm lateral and Lambda 0mm, +1.5mm lateral). The positive EEG electrode was attached to the anterior bore hole and the negative EEG electrode to the posterior bore hole. Both electrodes were secured in place using a suitable adhesive agent (Cyanoacrylate gel, RS components). A second set of electrodes were sutured into the nuchal muscle to act as EMG electrodes. All incisions were closed using absorbable suture. The animals were allowed to recover in a warm environment and given 5ml of saline s.c. The animals were kept singly in the cage until next day, then pair housed for the duration of post-surgical recovery (min 7 days). During the recovery period, the rats received 5 days of standard post-operative care with no further experimental procedures until the pre-operative body weight was regained. The animals were maintained on a 12/12hr light dark cycle after the surgery.

Testing: On study days, the animals were placed in recording boxes individually and EEG/EMG as well as locomotor activity were recorded for 0.5 h before and 24 h after each dosing. All animals were dosed with vehicle or 10 mg/kg (p.o.) of S1 prepared in a vehicle comprising PEG400, Cremaphor EL and water just prior the light onset or dark onset. All animals received all treatment conditions in a pseudo randomised cross-over fashion with a minimum period of 3 days between doses (see below). Six animals were used in this study, weighing 550-600g.

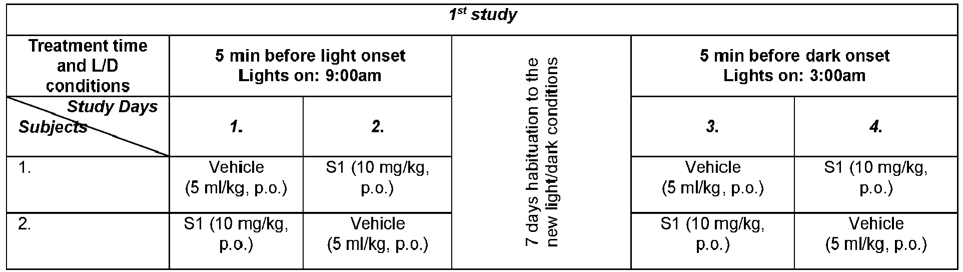

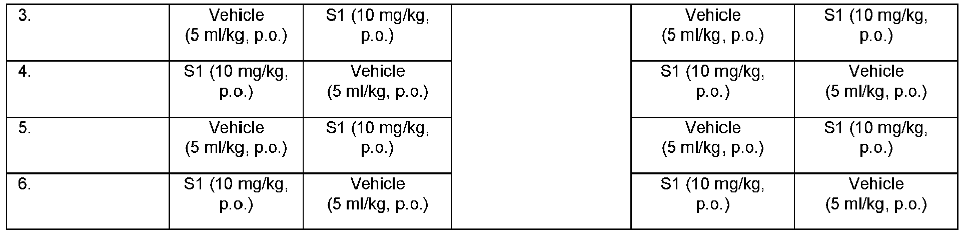

At the end of each recording session, the transmitters were turned off and the animals were returned to their home cages.

Data recording and analysis: EEG, EMG and locomotor activity data were acquired for 0.5 h before and 24 h after each treatment with Spike2 software (CED, Cambridge UK). EEG/EMG signals were amplified, analogue filtered (0.5-100 Hz), digitized (256 Hz), and then digitally filtered (EEG: 0.5-100 Hz and EMG: 5-100 Hz).

The subsequent EEG/EMG recordings were automatically scored (see Table below for sleep stage definitions) as wake, non-REM (NREM) sleep, or REM sleep in 10 second epochs using SleepSign (Kissei Comtec, Japan).

| ***Sleep Stage*** | ***Definition*** | |
|---|---|---|
| Wake | a. | Activity integral ≥ 65,000 AU or |
| | b. | EMG integral > 4.5 µV-sec |
| NREM sleep | a. | EEG delta (0.65-4.5 Hz) power ≥ 900 µV2 and EMG integral < 11.5 µV-sec or |
| | b. | EEG delta oscillations / 10 s ≥ 29% and EMG integral < 6.5 µV-sec |
| REM sleep | a. | EEG theta (6-10 Hz) power ratio ≥ 31% and EMG integral < 2 µV-sec or |
| | b. | EEG theta oscillations / 10 s ≥ 29% and EMG integral < 4.5 µV-sec |

Power spectral analysis was performed on EEG data recorded over the first 3 h. EEG power spectra were computed for consecutive 2 s epochs by fast Fourier transformation (Hanning window, 0.5 Hz resolution) between 0.5-100Hz. Epochs with artefacts (5xSTD of RMS) were discarded. Data were presented in 1 Hz bins, and the bins were marked by their upper limits. In addition, EEG spectral data was presented for the following frequency bands: delta (0.5-4 Hz), theta (4-10Hz), beta (10-30 Hz) and gamma (30-100Hz).

Statistical analysis: Repeated measures ANOVA followed by appropriate post-hoc test was used to compare the different treatment groups (GraphPad, Prism 8). A p<0.05 was deemed to be statistically significant.

Results: CNS pharmacodynamic effect shown in rat EEG studies.
As shown in Figure 1, BPTU dosed orally at 10mg/kg caused significant changes in the EEG power spectrum in in the first three hours post dosing. The change in EEG power (in the non-REM vigilance state) is shown in the top left graph of Figure 1; the data show percentage change induced by BPTU in the EEG power across the frequency spectrum. The changes are quantified in the accompanying bar charts. Significant increases were observed in the delta and theta bands during non-REM sleep, and in the gamma frequency band during wake. These pharmacodynamic responses demonstrate functional effects of BPTU within the CNS following systemic administration, and confirm that the CNS penetration observed in pharmacokinetic studies reaches sufficient CNS exposure to exert pharmacological effects.

### Example 3

P2Y1 inhibition data is provided below for selected compounds. An asterisk (*) indicates those compounds that have also been assayed for CNS penetration. Each of these assayed compounds has been empirically determined to be a CNS penetrant.

| **Structure** | **ID** | **IC50 IP1 (nM)^{a}** |
|---|---|---|
| 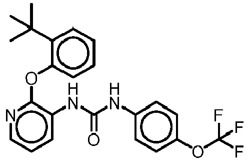 | **S1 (BPTU)*** | 35 |
| 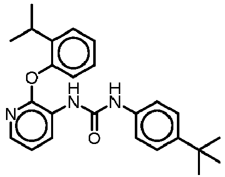 | S2 | 52 |
| 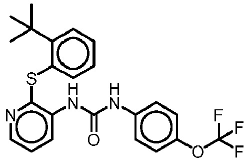 | S3 | 57 |
| 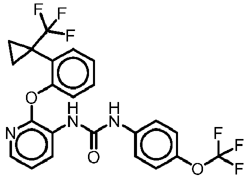 | S4* | 58 |
| 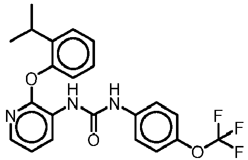 | S5 | 65 |
| 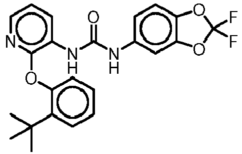 | S6 | 76 |
| 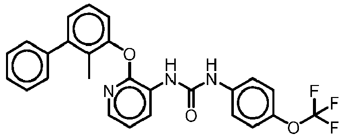 | S7 | 99 |
| 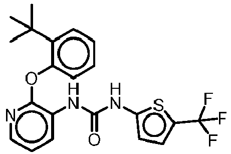 | S8* | 121 |
| 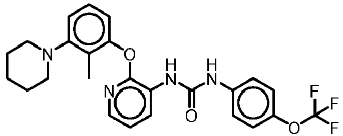 | S9* | 122 |
| 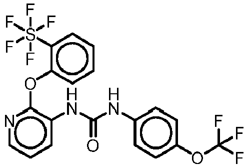 | S10 | 124 |
| 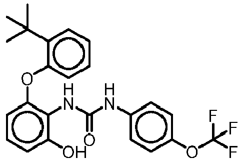 | S11* | 150 |
| 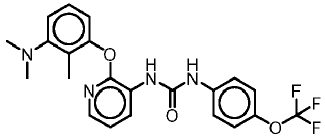 | S12 | 151 |
| 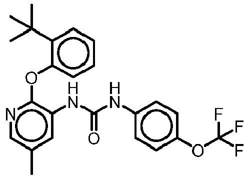 | S13 | 151 |
| 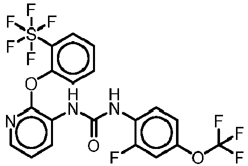 | S14 | 173 |
| 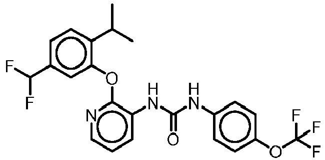 | S15 | 179 |
| 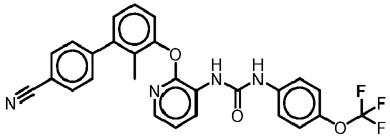 | S16 | 202 |
| 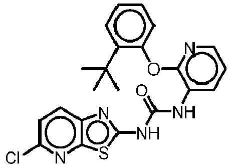 | S17 | 209 |
| 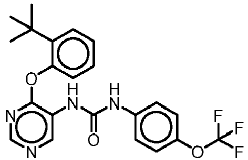 | S18 | 236 |
| 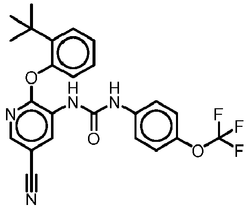 | S19 | 264 |
| 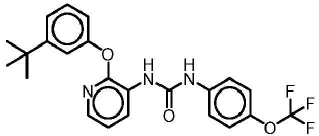 | S20 | 276 |
| 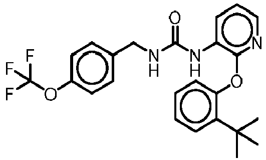 | S21 | 301 |
| 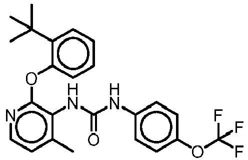 | S22 | 302 |
| 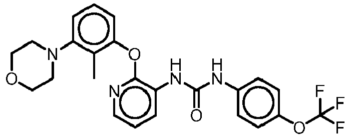 | S23 | 332 |
| 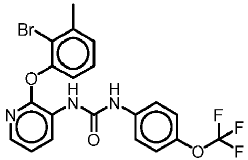 | S24 | 398 |
| 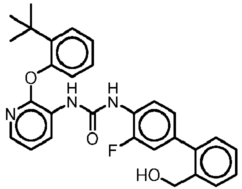 | S25 | 400 |
| 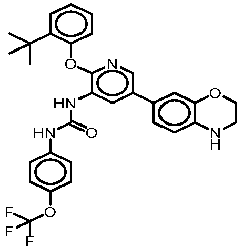 | S26 | 493 |
| 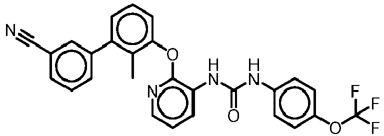 | S27 | 512 |
| 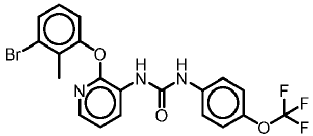 | S28 | 513 |
| 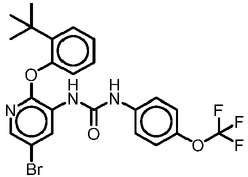 | S29 | 514 |
| 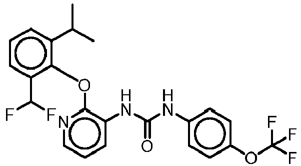 | S30 | 574 |
| 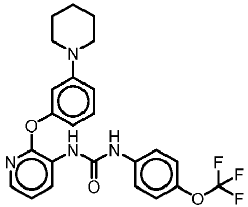 | S31 | 607 |
| 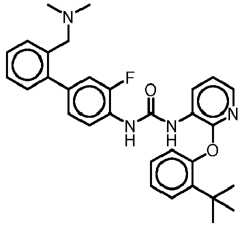 | S32 | 626 |
| 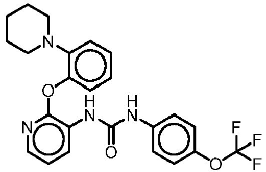 | S33 | 632 |
| 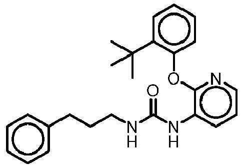 | S34 | 640 |
| 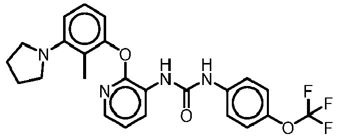 | S35 | 655 |
| 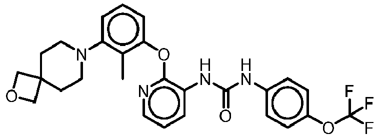 | S36 | 732 |
| 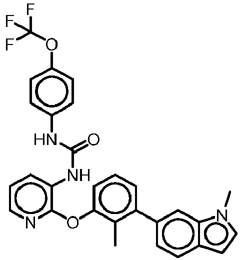 | S37 | 778 |
| 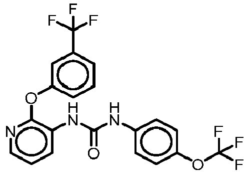 | S38 | 787 |
| 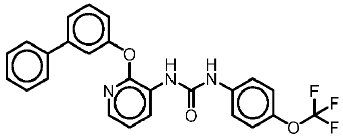 | S39 | 827 |
| 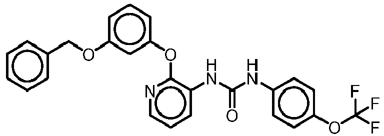 | S40 | 924 |
| 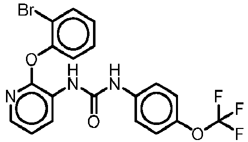 | S41 | 925 |
| 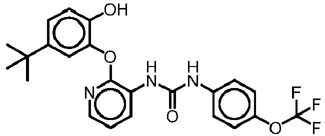 | S42 | 948 |
| 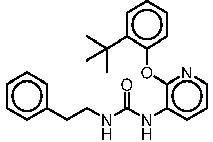 | S43 | 958 |
| 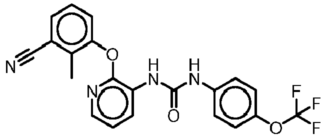 | S44 | 1027 |
| 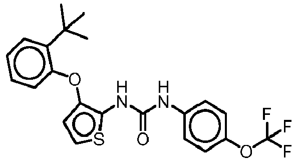 | S45 | 1060 |
| 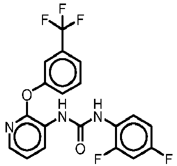 | S46 | 1063 |
| 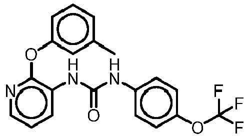 | S47 | 1095 |
| 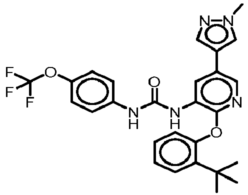 | S48 | 1115 |
| 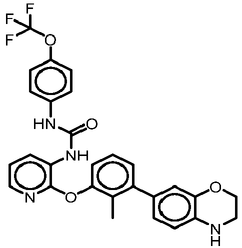 | S49 | 1243 |
| 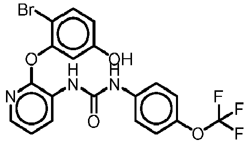 | S50 | 1277 |
| 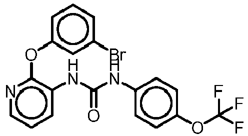 | S51 | 1315 |
| 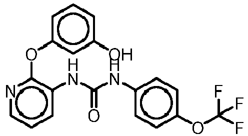 | S52 | 1407 |
| 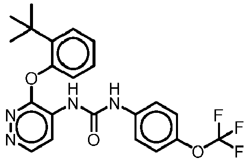 | S53 | 1768 |
| 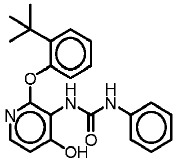 | S54 | 1769 |
| 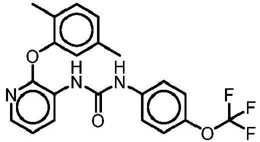 | S55 | 1974 |
| 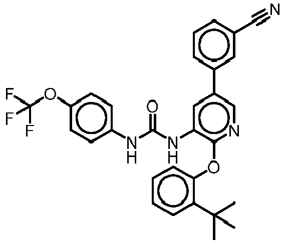 | S56 | 1958 |
| 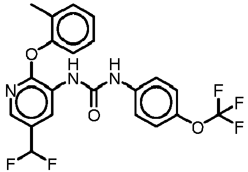 | S57 | 1989 |
| 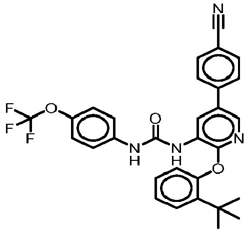 | S58 | 2103 |
| 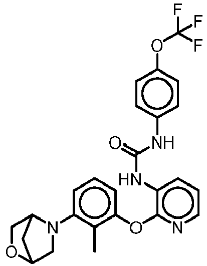 | S59 | 2136 |
| 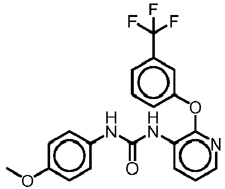 | S60 | 2405 |
| 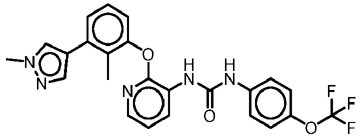 | S61 | 2461 |
| 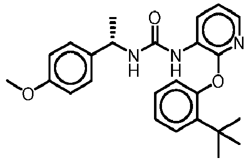 | S62 | 2466 |
| 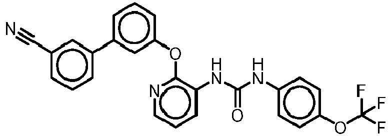 | S63 | 2508 |
| 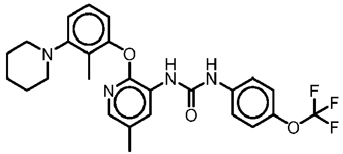 | S64 | 2810 |
| 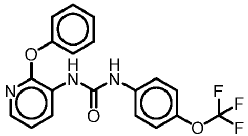 | S65 | 2864 |
| 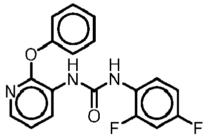 | S66 | 3175 |
| 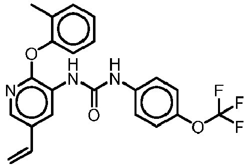 | S67 | 3353 |
| 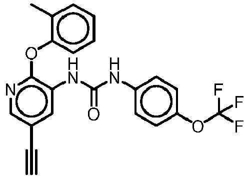 | S68 | 3590 |
| 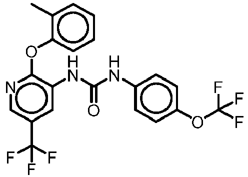 | S69 | 4387 |
| 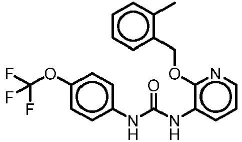 | S70 | 4594 |
| 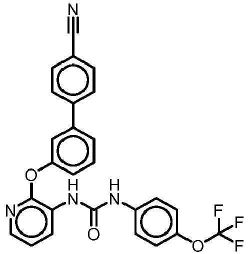 | S71 | 4943 |
| 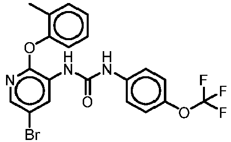 | S72 | 4943 |
| 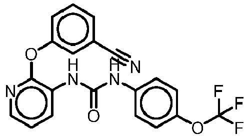 | S73 | 5841 |
| 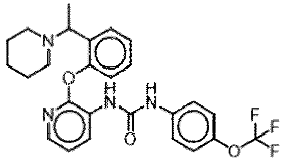 | S74 | 5995 |
| 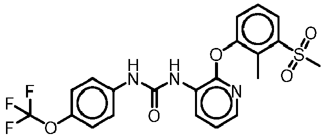 | S75 | 6595 |
| 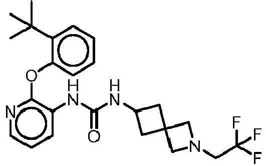 | S76 | 7075 |
| 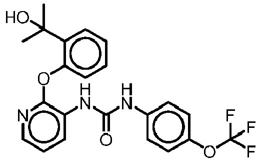 | S77 | 7118 |
| 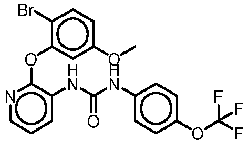 | S78 | 7158 |
| 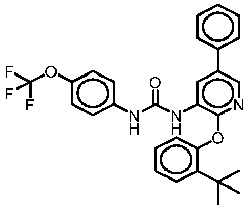 | S79 | 7581 |
| 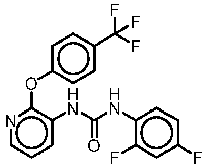 | S80 | 7940 |
| 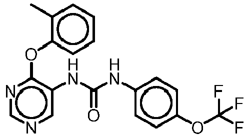 | S81 | 8124 |
| 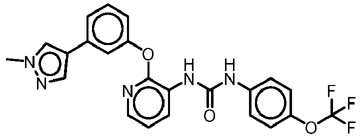 | S82 | 8182 |
| 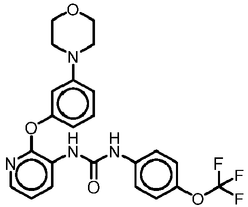 | S83 | 8733 |
| 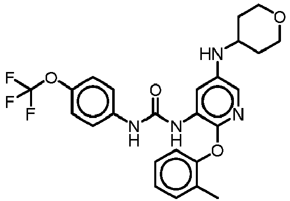 | S84 | 8985 |
| 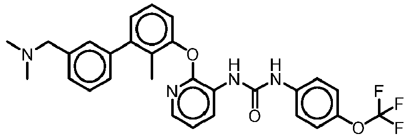 | S85 | 9528 |
| 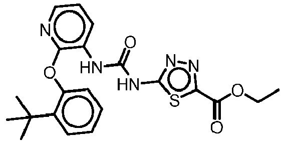 | S86 | 9679 |

| | | |
|---|---|---|
| ^{a}All values rounded to nearest whole number | | |

### Compound synthesis

Detailed, exemplary procedures for the synthesis of the compounds of aspects 1 to 59 are provided in WO 2005/113511. Detailed, exemplary procedures for the synthesis of the compounds of aspects 66 to 83 are provided in WO 2008/023235. Further, specific synthesis information for representative compounds is provided below.

### S1: full experimental

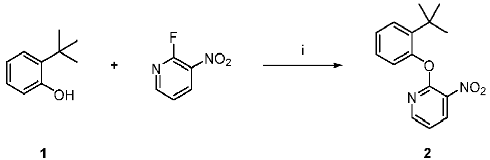

STEP 1: To a 2-neck flask with internal thermometer and stirrer was charged with 2-fluoro-3-nitro-pyridine (5.0 g, 35.2 mmol, 1.0 eq.), 2-tert-butylphenol 1 (6.49mL, 42.2 mmol, 1.2 eq.) and potassium carbonate (5.84 g, 42.2mmol, 1.2 eq.). The flask was fitted with a water-less condenser, evacuated and back-filled with argon (x3). With slow stirring, the reaction mixture was heated until an internal temperature of 120 °C was achieved, during such time an orange solid is observed to form and pockets of gas are observed to be released. The reaction mixture was held at 120 °C for a further 2 hr, then cooled to room temperature and diluted with dichloromethane (100 mL), washed with water (100 mL) and the organic phase passed through a hydrophobic frit. The filtrate was concentrated *under reduced pressure to* give an orange residue. The residue was recrystallized from ethanol (30 mL) to give 2-(2-(tert-butyl)phenoxy)-3-nitropyridine **2** as orange prisms (8.1 g, 85%). LCMS: m/z 273.1 [M+H]⁺; ¹H NMR (400 MHz, CDCl₃) δ = 8.33 - 8.49 (m, 2H), 7.44 - 7.58 (m, 1H), 7.20 - 7.34 (m, 2H), 7.16 (dd, *J=* 4.86, 7.79 Hz, 1H), 6.90 - 7.02 (m, 1H), 1.40 (s, 9H).

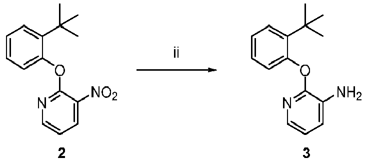

STEP 2: To a stirred solution of 2-(2-tert-butylphenoxy)-3-nitro-pyridine **2** (6.0 g, 22.0 mmol, 1.0 eq.) in tetrahydrofuran (60 mL), under argon, was added zinc powder (7.20 g, 110.2 mmol, 5.0 eq.) followed by a solution of ammonium chloride (5.89 g, 110.2 mmol, 5.0 eq.) in water (20 mL). The reaction mixture heated at 80 °C for 3 hr after such time LCMS showed complete consumption of compound **2.** The reaction mixture was allowed to cool to room temperature, diluted with water (50 mL) and the biphasic mixture passed through a 2 cm pad of Celite^{®}, eluting with ethyl acetate (100 mL). The solution was concentrated under reduced pressure to give 2-(2-tert-butylphenoxy)pyridin-3-amine 3 as an off-white solid. (4.40 g, 82%) that was used directly in the next reaction without further purification. LCMS: m/z 243.1 [M+H]⁺; ¹H NMR (400 MHz, CDCl₃) δ =7.61 (dd, *J* = 4.9, 1.6 Hz, 1H), 7.46 (dd, *J* = 7.8, 1.7 Hz, 1H), 7.19 - 7.25 (m, 1H), 7.09 - 7.18 (m, 1H), 7.07 (dd, *J* = 7.6, 1.7 Hz, 1H), 6.96 (dd, *J* = 8.0, 1.4 Hz, 1H), 6.85 (dd, *J* = 7.6, 4.9 Hz, 1H), 3.96 (br. s., 2H), 1.44 (s, 9H).

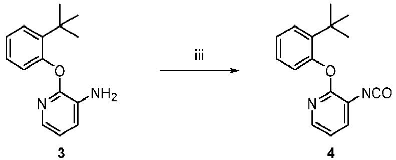

STEP 3: A stirred solution of 2-(2-tert-butylphenoxy)pyridin-3-amine 3 (5.0 g, 20.6 mmol, 1.0 eq.) in dichloromethane (100 mL), under argon, was cooled to 0 °C. Trichloromethyl chloroformate (2.12 mL, 17.5 mmol, 0.85 eq.) was added dropwise followed by *N*1,*N*1,*N*8,*N*8-tetramethylnaphthalene-1,8-diamine (8.84 g, 41.3 mmol, 2.0 eq.) in a single portion. The reaction mixture was allowed to warm to room temperature overnight. The reaction mixture was washed with 0.5 N aq. HCl (3 x 100 mL), 1.0 N aq. NaOH (100 mL) and brine. The organic phase was passed through a hydrophobic frit and then concentrated under reduced pressure to give an oil that solidified on standing to give 2-(2-tert-butylphenoxy)-3-isocyanato-pyridine **4** (5.45 g, 98%) as a beige solid that was used directly in the next reaction without further purification. ¹H NMR (400 MHz, CDCl₃) δ = 7.88 (dd, *J* = 4.9, 1.8 Hz, 1H), 7.41 (dd, *J* = 7.7, 1.9 Hz, 1H), 7.33 (dd, *J* = 7.7, 1.7 Hz, 1H), 7.07 - 7.19 (m, 2H), 6.80 - 6.89 (m, 2H), 1.29 - 1.35 (m, 9H).

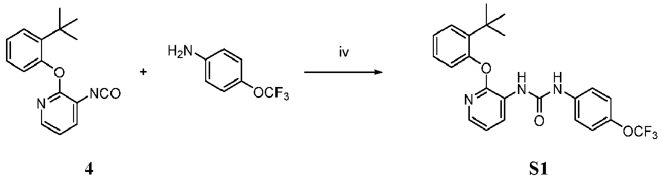

### 1-(2-(2-(tert-butyl)phenoxy)pyridin-3-yl)-3-(4-(trifluoromethoxy)phenyl)urea (S1) (BPTU)

STEP 4: To a stirred solution of 2-tert-butylphenoxy)-3-isocyanato-pyridine **4** (1.50 g, 5.59 mmol, 1.0 eq.), under argon, in toluene (50 mL) was added 4-(trifluoromethoxy)aniline (0.75 mL, 5.59 mmol, 1.0 eq.). The reaction mixture was warmed to 50 °C and stirred for 15 h. LCMS showed complete conversion of the isocyanate to the urea product. The solvent was partially removed in vacuo until solid was observed to precipitate from the solution. The flask was removed and the product allowed to crystallize out from the remaining solvent. The solid was filtered and washed with ice-cold 50% aqueous acetonitrile. The solid was dried in a vacuum oven overnight (40 °C, <1 mm Hg) to give the title compound (1.64 g, 66%) as a white solid. Concentration of the mother liquor and re-crystallization from 50% aqueous acetonitrile yielded a second crop of the title compound (756 mg, 30%) as a white solid. LCMS: m/z 446.3 [M+H]⁺; ¹H NMR (400 MHz, DMSO-d₆) δ = 9.63 (s, 1H), 8.65 (s, 1H), 8.54 (dd, J = 7.9, 1.7 Hz, 1H), 7.70 (dd, J = 4.8, 1.7 Hz, 1H), 7.54 - 7.61 (m, 2H), 7.45 (dd, J = 7.9, 1.7 Hz, 1H), 7.28 - 7.34 (m, 2H), 7.21 - 7.28 (m, 1H), 7.13 - 7.21 (m, 1H), 7.08 (dd, J = 7.9, 4.9 Hz, 1H), 6.93 (dd, J = 7.9, 1.4 Hz, 1H), 1.34 (s, 9H).

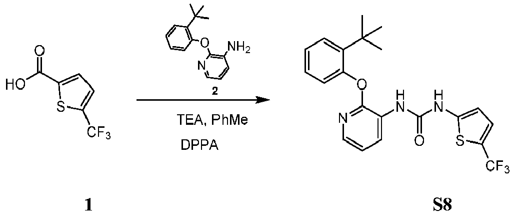

### 1-(2-(2-(tert-butyl)phenoxy)pyridin-3-yl)-3-(5-(trifluoromethyl)thiophen-2-yl)urea (S8)

STEP 1: To a solution of 5-(trifluoromethyl)thiophene-2-carboxylic acid 1 (221 mg, 1.13 mmol, 1.0 eq.) in toluene (5 mL) was added diphenylphosphoryl azide (465 mg, 1.69 mmol, 366 uL, 1.5 eq.) and TEA (228 mg, 2.25 mmol, 313 uL, 2.0 eq.). The mixture was stirred at 20 °C for 0.5 hr, then stirred at 80 °C for 2 hr. Then compound **2** (0.30 g, 1.24 mmol, 1.1 eq.) was added at 20 °C, the mixture was stirred at 80 °C for 12 hr. LC-MS showed compound **1A** was completely consumed and the desired product mass was detected. The reaction mixture was concentrated under reduced pressure to give a residue. The residue was purified by prep-HPLC (FA condition; LCMS_03 column: Phenomenex Luna C18 200^{∗}40mm^{∗}10um; mobile phase: [water(0.2%FA)-ACN];B%: 50%-90%,8min) to give the title material (67 mg, 14%) as a yellow solid. LC-MS: m/z 436.0 [M+H]⁺; ¹H NMR (400MHz, CDCl₃) δ = 8.60 (dd, *J=* 1.3, 8.0 Hz, 1H), 7.89 - 7.80 (m, 1H), 7.62 (br s, 1H), 7.52 - 7.39 (m, 2H), 7.25 - 7.15 (m, 3H), 7.02 (dd, *J=* 4.9, 8.0 Hz, 1H), 6.86 (dd, *J* = 1.3, 7.8 Hz, 1H), 6.54 (d, *J=* 3.9 Hz, 1H), 1.36 (s, 9H).

### RS2: full experimental

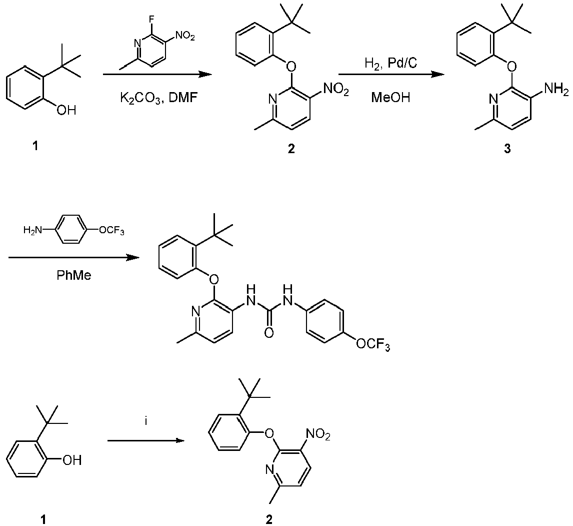

STEP 1: To a solution of 2-tert-butylphenol 1 (1.01 g, 6.66 mmol, 1.02 mL, 1.0 eq.) in DMF (10 mL) was added K₂CO₃ (2.76 g, 19.97 mmol, 3.0 eq.) and 2-fluoro-6-methyl-3-nitro-pyridine (1.04 g, 6.66 mmol, 1.0 eq.). The mixture was stirred at 80 °C for 12 hr. LC-MS showed the main peak was the desired mass. The reaction mixture was concentrated under reduced pressure to remove solvent. The residue was purified by column chromatography (SiO₂, petroleum ether/ethyl acetate = 1/0 to 1/1) to give 2-(2-(tert-butyl)phenoxy)-6-methyl-3-nitropyridine **3** (1.5 g, 5.24 mmol, 79% yield) as a light yellow solid. LC-MS: m/z 287.1 [M+H]⁺.

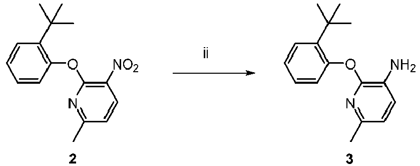

STEP 2: To a solution of 2-(2-(tert-butyl)phenoxy)-6-methyl-3-nitropyridine 3 (500 mg, 1.75 mmol, 1.0 eq.) in MeOH (10 mL) was added Pd/C (10% wt., 200 mg, 0.19 mmol, .1 eq.) under N₂. The suspension was degassed under vacuum and purged with H₂ (15 psi) several times. The mixture was stirred under H₂ (15 psi) at 20 °C for 2 hr. LC-MS showed that compound **3** was completely consumed and the desired product mass was detected. The reaction mixture was purged with N₂, filtered and the filtrate concentrated under reduced pressure. 2-(2-(tert-butyl)phenoxy)-6-methylpyridin-3-amine **3** (447 mg, crude) was obtained as a white solid and used without further purification. LC-MS: m/z 257.1 [M+H]⁺.

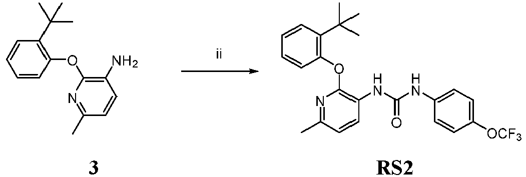

### 1-(2-(2-(tert-butyl)phenoxy)-6-methylpyridin-3-yl)-3-(4-(trifluoromethoxy)phenyl)urea (RS2)

STEP 3: To a solution of 2-(2-(tert-butyl)phenoxy)-6-methylpyridin-3-amine **3** (447 mg, 1.74 mmol, 1.0 eq.) in THF (5 mL) was added TEA (529 mg, 5.23 mmol, 728 uL, 3.0 eq.) and 1-isocyanato-4-(trifluoromethoxy) benzene (354 mg, 1.74 mmol, 262 uL, 1.0 eq.). The mixture was stirred at 0 °C for 1 hr. LC-MS showed that compound **3** was completely consumed and the desired product mass was detected. The reaction mixture was concentrated under reduced pressure to remove solvent. The residue was purified by prep-HPLC (column: Waters Xbridge Prep OBD C18 150^{∗}40mm^{∗}10um; mobile phase: [water(0.05% NH₃H₂O+10mM NH4HCO3)-ACN];B%: 55%-85%,8min) to give the title compound (371 mg, 46%) as a light yellow solid. LC-MS: m/z 460.2 [M+H]⁺; ¹H NMR (400MHz, CDCl₃) δ = 8.41 - 8.38 (m, 1H), 8.39 (d, *J* = 8.0 Hz, 1H), 8.75 - 8.26 (m, 1H), 7.44 - 7.41 (m, 1H), 7.45 - 7.36 (m, 1H), 7.37 (d, *J* = 9.0 Hz, 1H), 7.21 - 7.11 (m, 4H), 7.02 (s, 1H), 6.90 - 6.81 (m, 2H), 6.52 (s, 1H), 2.31 (s, 3H), 1.34 (s, 9H).

### S4: full experimental

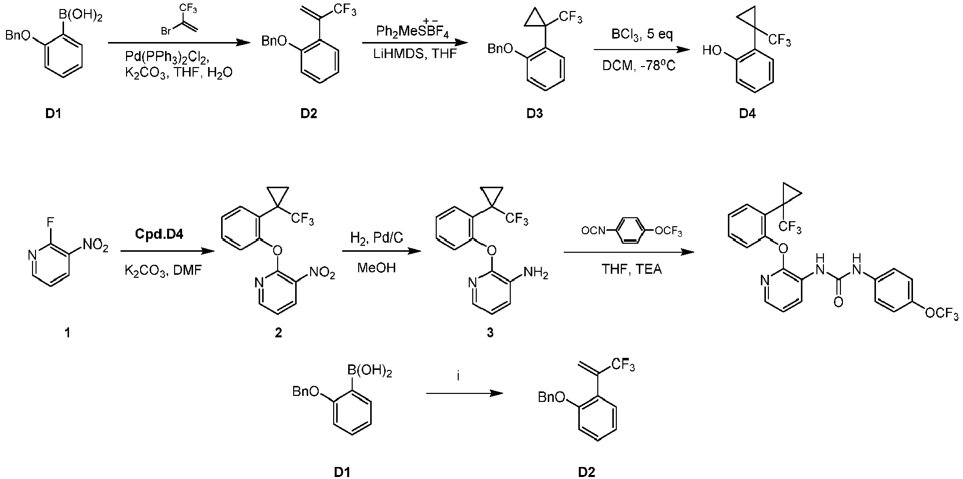

STEP 1: To a solution of (2-(benzyloxy)phenyl)boronic acid **D1** (15.0 g, 65.77 mmol, 1.0 eq.) in THF (150 mL) and H₂O (45 mL) was added K₂CO₃ (22.73 g, 164.44 mmol, 2.5 eq.), 2-bromo-3,3,3-trifluoro-prop-1-ene (11.51 g, 65.77 mmol, 1.0 eq.) and Pd(PPh₃)₂Cl₂ (4.62 g, 6.58 mmol, 0.1 eq.). The mixture was stirred at 25°C for 5 hr. LC-MS showed compound **D1** was consumed completely and the desired product mass was detected. The reaction mixture was concentrated under reduced pressure to give a residue. The residue was partitioned between water (200 mL) and EtOAc (300 mL) and the aqueous phase extracted with EtOAc (300 mL x2). The combined organic phases were dried over Na₂SO₄, filtered and the filtrate concentrated under reduced pressure to give a residue. The residue was purified by MPLC (SiO₂, petroleum ether/ ethyl acetate=1/ 0 to 25/ 1) to give 1-(benzyloxy)-2-(3,3,3-trifluoroprop-1-en-2-yl)benzene **D2** (8.0 g, 44 %) as a colorless oil. TLC (petroleum ether/ethyl acetate = 10/1, R_{f}= 0.58); LC-MS: m/z 279.1 [M+H]⁺; ¹H NMR (400MHz, CDCl₃) δ = 7.24 - 7.51 (m, 7H), 6.86 - 7.10 (m, 2H), 6.13 (d, *J=* 1.38 Hz, 1H), 5.70 (d, *J* = 1.00 Hz, 1 H), 5.13 (s, 2H).

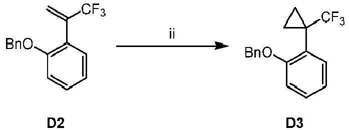

STEP 2: A mixture of compound 1-(benzyloxy)-2-(3,3,3-trifluoroprop-1-en-2-yl)benzene **D2** (8.0 g, 28.75 mmol, 1.0 eq.), methyl(diphenyl)sulfonium tetrafluoroborate (20.93 g, 69.00 mmol, 2.4 eq.) in THF (80 mL) was degassed and purged with N₂ (x3), and then LiHMDS (1.0 M in THF, 86 mL, 3.0 eq.) was added. The mixture was stirred at -78 °C for 3 hr under a N₂ atmosphere. TLC (petroleum ether/ethyl acetate = 1/0) indicated multiple new products had formed. The reaction mixture was quenched with water (100 mL) at 0 °C, and then extracted with EtOAc (100 mL x3). The combined organic phases were dried over Na₂SO₄, filtered and the filtrate concentrated under reduced pressure to give a residue. The residue was purified by MPLC (SiO₂, petroleum ether/ethyl acetate = 1/0 to 20/ 1) and all fractions containing compound **D3** were combined to give 4.5 g of a crude fraction. A sample of the crude fraction containing compound **D3** (2.0 g) was further purified by column chromatography (SiO₂, petroleum ether/ ethyl acetate = 1/0 to 20/1) to give pure 1-(benzyloxy)-2-(1-(trifluoromethyl)cyclopropyl)benzene **D3** (500 mg) as a colourless oil. TLC (petroleum ether/ethyl acetate = 1/0, R_{f} = 0.44). ¹H NMR (400MHz, CDCl₃) δ = 7.28 - 7.54 (m, 10H); 6.97 - 7.05 (m, 3H), 5.16 - 5.19 (m, 1H), 5.13 - 5.20 (m, 2H), 1.40 - 1.46 (m, 2H), 1.08 (s, 2H).

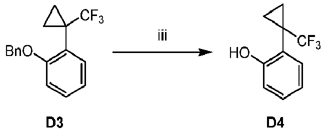

STEP 3: A stirred solution of 1-(benzyloxy)-2-(1-(trifluoromethyl)cyclopropyl)benzene **D3** (500 mg, 1.71 mmol, 1.0 eq.) in CH₂Cl₂ (5 mL) was stirred at -78°C for 0.5 hr, then BCl₃ (1.0 M in CH₂Cl₂, 8.55 mL, 5 eq.) was added. The mixture was stirred at -78°C for 1.5 hr. LC-MS showed that compound **D3** was completely consumed and the desired product mass was detected. The reaction mixture was quenched with MeOH (5 mL) at 0 °C, concentrated under reduced pressure to give a residue. The residue was purified by prep-TLC (SiO₂, petroleum ether/ethyl acetate = 3/1) to give 2-(1-(trifluoromethyl)cyclopropyl)phenol **D4** (260 mg, 75%). TLC (petroleum ether/ethyl acetate = 3/1, R_{f} = 0.58); LC-MS: m/z 201.0 [M-H]⁻.

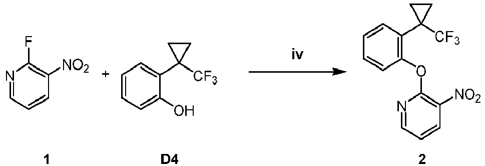

STEP 4: To a solution of 2-(1-(trifluoromethyl)cyclopropyl)phenol **D4** (0.26 g, 1.29 mmol, 1.0 *eq.)* and compound **1** (183 mg, 1.29 mmol, 1.0 *eq.)* in DMF (5 mL) was added K₂CO₃ (356 mg, 2.57 mmol, 2.0 *eq.).* The mixture was stirred at 110°C for 12 hr. LC-MS showed that compound **D4** was completely consumed and the desired product mass was detected. The reaction mixture was diluted with water (10 mL) and extracted with ethyl acetate (10 mL x4), dried over Na₂SO₄, filtered and the filtrate concentrated under reduced pressure to give a residue. The residue was purified by prep-TLC (SiO₂, petroleum ether/ethyl acetate = 2/1) to give 3-nitro-2-(2-(1-(trifluoromethyl)cyclopropyl)phenoxy)pyridine 2 (50 mg, 12%) as a yellow oil. TLC (petroleum ether/ethyl acetate = 2/1, R_{f}= 0.53; LC-MS: m/z 325.2 [M+H]⁺.

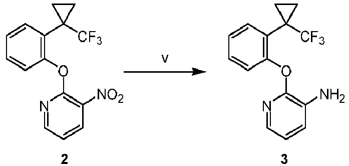

STEP 5: To a solution of 3-nitro-2-(2-(1-(trifluoromethyl)cyclopropyl)phenoxy)pyridine 2 (50 mg, 154 umol, 1.0 eq.) in MeOH (5 mL) was added Pd/C (1.6 mg, 15.4 ummol, 0.1 eq., 10% wt.). The mixture was purged with nitrogen, the flask evacuated and back-filled with H₂ (x3). The reaction mixture was stirred at 25 °C for 2 hr under H₂ (15 psi). LC-MS showed that compound **2** was completely consumed and the desired product mass was detected. The reaction mixture was filtered and the filtrate concentrated under reduced pressure to give 2-(2-(1-(trifluoromethyl)cyclopropyl)phenoxy)pyridin-3-amine 3 (40 mg, 88%) as a white solid. LC-MS: m/z 295.1 [M+H]⁺.

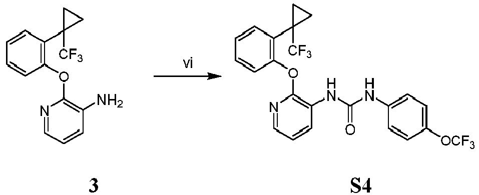

### 1-(4-(trifluoromethoxy)phenyl)-3-(2-(2-(1-(trifluoromethyl)cyclopropyl)phenoxy)pyridin-3-yl)urea (S4)

STEP 6: To a solution of 2-(2-(1-(trifluoromethyl)cyclopropyl)phenoxy)pyridin-3-amine **3** (35 mg, 119 umol, 1 *eq.)* in THF (5 mL) was added TEA (60 mg, 595 umol, 83 uL, 5.0 eq.) and 1-isocyanato-4-(trifluoromethoxy)benzene (73 mg, 357 umol, 54 uL, 3.0 eq.). The mixture was stirred at 0°C for 1 hr. LC-MS showed that compound **3** was completely consumed and the desired product mass was detected. The reaction mixture was concentrated under reduced pressure to remove solvent. The residue was purified by prep-HPLC (column: Phenomenex Gemini-NX C18 75^{∗}30mm^{∗}3um;mobile phase: [water(0.05%NH₃H₂O+10mM NH₄HCO₃)-ACN];B%: 55%-80%,8min) to give the title compound (32 mg, 55%) as a white solid. LC-MS: m/z 498.0 [M+H]⁺; ¹H NMR (400MHz, CDCl₃) δ = 8.52 (br. d, *J* = 1.50 Hz, 1H), 7.70 (dd, *J* = 4.88, 1.50 Hz, 1H), 7.46 (dd, J=7.63, 1.25 Hz, 1H), 7.24 - 7.39 (m, 4H), 7.09 - 7.18 (m, 2H), 7.04 (d, *J* = 8.13 Hz, 1H), 6.93 - 7.00 (m, 1H), 6.67 (s, 1H), 1.11 - 1.24 (m, 2H), 0.79 - 0.93 (m, 2H).

### RS1: full experimental

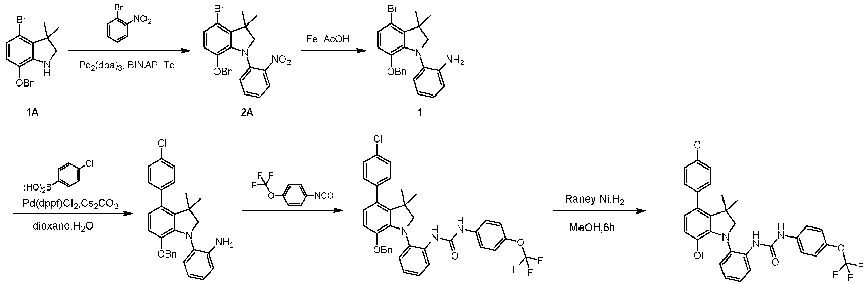

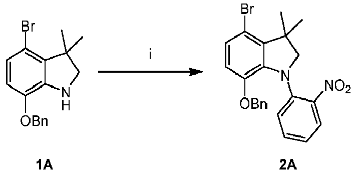

STEP 1: To a solution of 7-(benzyloxy)-4-bromo-3,3-dimethylindoline 1A (3.0 g, 9.03 mmol, 1.0 eq.) in toluene (30 mL) was added Cs₂CO₃ (8.83 g, 27.09 mmol, 3.0 eq.), 1-bromo-2-nitro-benzene (2.19 g, 10.84 mmol, 1.2 eq.), rac-BINAP (562 mg, 0.90 mmol, 0.1 eq.) and Pd₂(dba)₃ (413 mg, 0.52 mmol, 0.05 eq.). The mixture was degassed and purged with N₂ (x3), and then the mixture was stirred at 110 °C for 12 hr under N₂ atmosphere. The reaction mixture was diluted with H₂O (30 mL), and then extracted with EtOAc (30mL x3). The combined organic layers were washed with brine (50 mL), dried over Na₂SO₄, filtered and the filtrate concentrated under reduced pressure to give a residue. The residue was purified by column chromatography (SiO₂, petroleum ether/ethyl acetate=0/1 to 9/1). 7-(benzyloxy)-4-bromo-3,3-dimethyl-1-(2-nitrophenyl)indoline **2A** (3.26 g, 80%) was obtained as red solid. LC-MS: m/z 453.2/455.2[M+H]⁺.

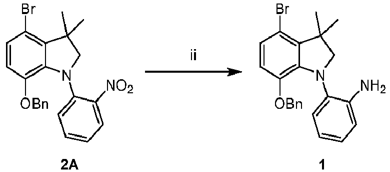

STEP 2: A mixture of 7-(benzyloxy)-4-bromo-3,3-dimethyl-1-(2-nitrophenyl)indoline **2A** (1.30 g, 2.87 mmol, 1.0 eq.) and Fe (801 mg, 14.34 mmol, 5.0 eq.) in AcOH (30 mL) was stirred at 80 °C for 3 hr. LC-MS showed that compound **2A** was completely consumed and the desired product mass was detected. The reaction mixture was concentrated under reduced pressure to remove AcOH. Then the residue was taken-up in H₂O (50 mL) and extracted with EtOAc (50 mL x3). The combined organic layers were washed with sat. NaHCO₃ solution (50 mL x3), dried over Na₂SO₄, filtered and the filtrate concentrated under reduced pressure to give a residue. 2-(7-(benzyloxy)-4-bromo-3,3-dimethylindolin-1-yl)aniline **1** (1.36 g, crude) was obtained as black oil and used directly in the next reaction without further purification. LC-MS: m/z 423.0/425.0[M+H]⁺.

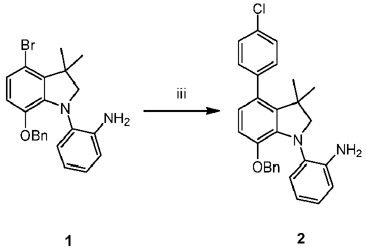

STEP 3: To a mixture of 2-(7-(benzyloxy)-4-bromo-3,3-dimethylindolin-1-yl)aniline **1** (300 mg, 0.71 mmol, 1.0 eq.), (4-chlorophenyl)boronic acid (133 mg, 0.85 mmol, 1.2 eq.), Cs₂CO₃ (577 mg, 1.77 mmol, 2.5 eq.) in dioxane (5 mL) and H₂O (0.5 mL) was added Pd(dppf)Cl₂ (52 mg, 71 umol, 0.1 eq.). The mixture was degassed and purged with N₂ (x3) and then the mixture was stirred at 80 °C for 12 hr under a N₂ atmosphere. LC-MS showed that compound 1 was completely consumed and the desired product mass was detected. The reaction mixture was diluted with H₂O (10 mL) and extracted with EtOAc (20 mL x3). The combined organic layers were washed with brine (50 mL), dried over Na₂SO₄, filtered and the filtrate concentrated under reduced pressure to give a residue. The residue was purified by prep-TLC (SiO₂, petroleum ether: ethyl acetate = 4:1) to give 2-(7-(benzyloxy)-4-(4-chlorophenyl)-3,3-dimethylindolin-1-yl)aniline 2 (200 mg, 62%) as a brown solid. LC-MS: m/z 455.2 [M+H]⁺.

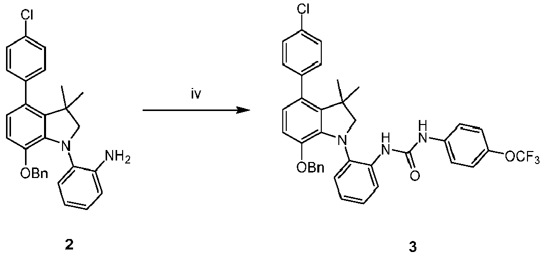

STEP 4: To a solution of 2-(7-(benzyloxy)-4-(4-chlorophenyl)-3,3-dimethylindolin-1-yl)aniline 2 (160 mg, 0.35 mmol, 1.0 eq.) and TEA (36 mg, 0.35 mmol, 49 uL, 1.0 eq.) in THF (2 mL) was added 1-isocyanato-4-(trifluoromethoxy)benzene (86 mg, 0.42 mmol, 64 uL, 1.2 eq.) at 0°C. The mixture was stirred at 15°C for 2 hr. LC-MS showed that compound **2** was completely consumed and the desired product mass was detected. The reaction mixture was concentrated under reduced pressure to give a residue. 1-(2-(7-(Benzyloxy)-4-(4-chlorophenyl)-3,3-dimethylindolin-1-yl)phenyl)-3-(4-(trifluoromethoxy)phenyl)urea (200 mg, crude) was obtained as a dark-brown oil.

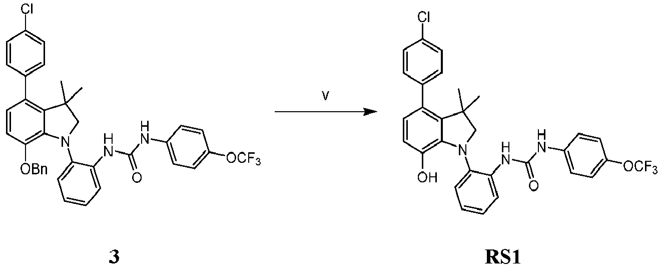

### 1-(2-(4-(4-chlorophenyl)-7-hydroxy-3,3-dimethylindolin-1-yl)phenyl)-3-(4-(trifluoromethoxy)phenyl)urea (RS1)

STEP 5: A mixture of 1-(2-(7-(benzyloxy)-4-(4-chlorophenyl)-3,3-dimethylindolin-1-yl)phenyl)-3-(4-(trifluoromethoxy)phenyl)urea **3** (190 mg, 0.29 mmol, 1.0 eq.) and Raney Ni (95 mg, 1.62 mmol, 5.6 eq.) in MeOH (2 mL) was degassed and purged with H₂ (x3), and then the mixture was stirred at 50°C for 6 hr under H₂ (15 psi) atmosphere. LC-MS showed that compound **3** was completely consumed and the desired product mass was detected. The reaction mixture was filtered and concentrated under reduced pressure to give a residue. The residue was purified by prep-HPLC (basic condition LC) column: Phenomenex Gemini-NX C18 75^{∗}30mm^{∗}3um;mobile phase: [water(10mM NH₄HCO₃)-ACN];B%: 75%-95%,8min to give the title material (115 mg, 70%) as a white solid. LC-MS: m/z 568.0 [M+H]⁺; ¹H NMR (400MHz, Methanol-d₄) δ = 7.92 - 7.83 (m, 1H), 7.56 - 7.47 (m, 2H), 7.42 - 7.34 (m, 2H), 7.31 - 7.24 (m, 2H), 7.20 - 7.14 (m, 2H), 7.12 - 7.06 (m, 1H), 7.03 - 6.94 (m, 2H), 6.66 - 6.47 (m, 2H), 3.74 (d, *J* = 9.9 Hz, 1H), 3.12 (d, *J* = 9.9 Hz, 1H), 1.08 (s, 6H).

### S11: full experimental

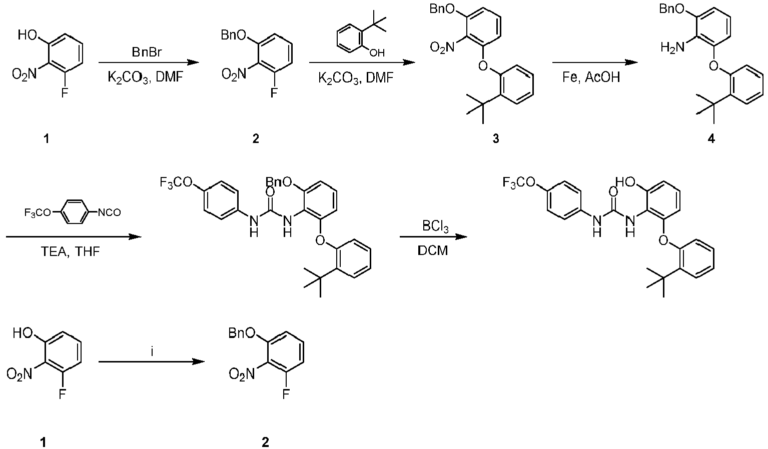

STEP 1: To a solution of 3-fluoro-2-nitrophenol **1** (1.5 g, 9.55 mmol, 1.0 eq.) in DMF (20 mL) was added K₂CO₃ (1.58 g, 11.46 mmol, 1.2eq.) and BnBr (1.63 g, 9.55 mmol, 1.13 mL, 1.0 eq.). The mixture was stirred at 20 °C for 12 hr. TLC indicated compound 1 was consumed completely, and one major new spot was observed. The reaction mixture was diluted with H₂O (200 mL) and extracted with ethyl acetate (200 mL x3). The combined organic layers were dried over Na₂SO₄, filtered and the filtrate concentrated under reduced pressure to give a residue. The residue was purified by column chromatography (SiO₂, petroleum ether/ ethyl acetate = 1/0 to 5/ 1) to give 1-(benzyloxy)-3-fluoro-2-nitrobenzene 2 (2.0 g, 85%) as a yellow oil. TLC: petroleum ether/ ethyl acetate = 5/1. R_{f} = 0.47.

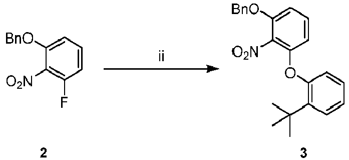

STEP 2: To a solution of 1-(benzyloxy)-3-fluoro-2-nitrobenzene **2** (11.0 g, 44.49 mmol, 1.0 eq.) in DMF (100 mL) was added K₂CO₃ (7.38 g, 53.39 mmol, 1.2 eq.) and 2-tertbutylphenol (6.68 g, 44.49 mmol, 6.83 mL, 1.0 eq.) at 20 °C. The mixture was stirred at 100 °C for 12 hr. TLC showed one major new spot and trace **2** remaining. The reaction mixture was diluted with H₂O (200 mL) and extracted with ethyl acetate (200 mL x3). The combined organic layers were dried over Na₂SO₄, filtered and concentrated under reduced pressure to give a residue. The residue was purified by column chromatography (SiO₂, petroleum ether/ethyl acetate=1/0 to 5/1)to give 1-(benzyloxy)-3-(2-(tert-butyl)phenoxy)-2-nitrobenzene **3** (8.8 g, 52%) as a white solid. TLC: petroleum ether/ethyl acetate=5/1. R_{f} = 0.65.

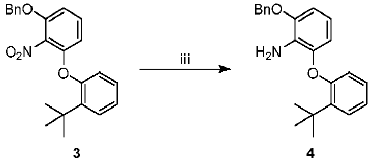

STEP 3: To a solution of 1-(benzyloxy)-3-(2-(tert-butyl)phenoxy)-2-nitrobenzene **3** (3.5 g, 9.27 mmol, 1.0 eq.) in AcOH (30 mL) was added Fe (5.18 g, 92.73 mmol, 10.0 eq.). The mixture was stirred at 80 °C for 1 hr. LC-MS showed that compound **3** was completely consumed and the desired product mass was detected. The reaction mixture was concentrated under reduced pressure to remove AcOH, and then the residue was dissolved in DCM (60 mL). The mixture was filtered, and the filter washed with DCM. The filtrate was washed with sat. NaHCO₃ solution (60 mL x3) and then concentrated under reduced pressure to give a residue. 2-(benzyloxy)-6-(2-(tert-butyl)phenoxy)aniline **4** (4.75 g, crude) was obtained as a black oil and used directly in the next reaction.

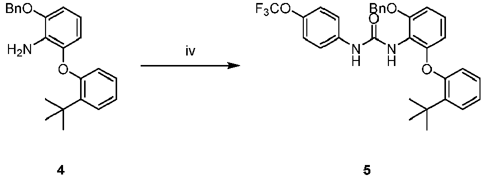

STEP 4: To a solution of 2-(benzyloxy)-6-(2-(tert-butyl)phenoxy)aniline **4** (400 mg, 1.15 mmol, 1.0 eq.) in THF (6 mL) was added TEA (350 mg, 3.45 mmol, 481 uL, 3.0 eq.) and 1-isocyanato-4-(trifluoromethoxy)benzene (468 mg, 2.30 mmol, 346 uL, 2.0 eq.) at 0 °C. The mixture was stirred at 20 °C for 3hr. LC-MS showed compound that **4** was completely consumed and the desired product mass was detected. The reaction mixture was diluted with H₂O (5 mL) and extracted with ethyl acetate (3 mL x3). The combined organic layers were dried over Na₂SO₄, filtered and the filtrate concentrated under reduced pressure to give a residue. 1-(2-(benzyloxy)-6-(2-(tert-butyl)phenoxy)phenyl)-3-(4-(trifluoromethoxy)phenyl)urea **5** (845 mg, crude) was obtained as a brown oil and used directly in the next reaction.

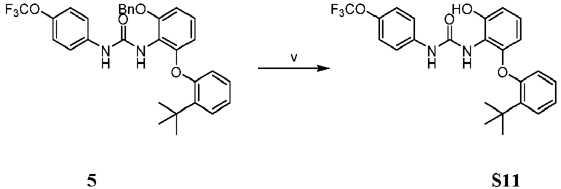

### 1-(2-(2-(tert-butyl)phenoxy)-6-hydroxyphenyl)-3-(4-(trifluoromethoxy)phenyl)urea (S11)

STEP 5: To a solution of 1-(2-(benzyloxy)-6-(2-(tert-butyl)phenoxy)phenyl)-3-(4-(trifluoromethoxy)phenyl)urea **5** (750 mg, 1.36 mmol, 1.0 eq.) in DCM (10 mL), cooled to -78°C, was added BCl₃ (1.0 M, 13.6 mL, 10 eq.). The mixture was warmed to room temperature and then stirred at 20 °C for 1 hr. LC-MS showed that compound **5** was completely consumed and the desired product mass was detected. The reaction mixture was quenched by adding MeOH (2 mL) and then the reaction mixture was concentrated under reduced pressure to give a residue. The residue was purified by prep-HPLC (column: Waters Xbridge Prep OBD C18 150^{∗}40mm^{∗}10um;mobile phase: [water(10mM NH₄HCO₃)-ACN];B%: 60%-85%,8min) to give the title material (176 mg, 28% over 3 steps) as a white solid. LC-MS: m/z 461.1 [M+H]⁺; ¹H NMR (400MHz, CDCl₃) δ = 9.68 (br. s, 1H), 7.40 (dd, *J* = 7.75, 1.88 Hz, 1H), 7.28 - 7.34 (m, 2H), 7.06 - 7.18 (m, 5H), 6.96 - 7.03 (m, 1H), 6.82 (dd, J= 8.25, 1.25 Hz, 1H), 6.73 (dd, J= 7.94, 1.44 Hz, 1H), 6.64 (s, 1H), 6.32 (dd, *J* = 8.13, 1.25 Hz, 1H), 1.32 (s, 9H).

### ASPECTS

The following aspects provide, in conjunction with the disclosure elsewhere herein, non-limiting exemplary embodiments of the present invention. For the avoidance of doubt, the present invention encompasses the subject-matter defined in any one of these aspects, any combination thereof such as those recited by dependency, and any combination thereof with features of the invention that are disclosed elsewhere herein.
1. A compound that (a) is of Formula (I): 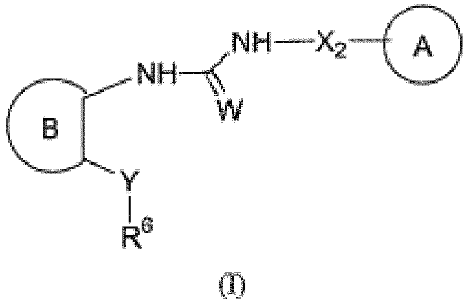 or (b) is a tautomer, N-oxide, pharmaceutically acceptable salt, or solvate thereof; wherein:
   ring A is C₆₋₁₀ aryl substituted with 0-5 R¹, or a 5- to 10-membered heterocycle comprising: carbon ring atoms and 1-4 ring heteroatoms selected from N, NR¹¹, O, and S(O)ₚ, wherein said heterocycle is substituted with 0-5 R¹;
   ring B is: (a) a 5- to 6- membered heteroaryl comprising: carbon ring atoms and 1-4 ring heteroatoms selected from N, NR¹¹, N→O, S(O)ₚ, and O, wherein said heteroaryl is substituted with 0-4 R⁷; or (b) a C₆₋₁₀ aryl substituted with from 0-4 R⁷;
   W is O or S;
   X₂ is -(CR¹⁶R¹⁷)s-, or -(CR¹⁶R¹⁷)ₜC(O)(CR¹⁶R¹⁷)r-;
   Y is O, S, NH, -OCR¹⁸R¹⁹-, -CH=CH-, or-CONH-;
   R¹ is, independently at each occurrence, =O, F, Cl, Br, I, CF₃, -CF₂CF₃, OCF₃, -OCF₂CF₂H, -OCF₂CF₃, SiMe₃, -(CR^{f}R^{f})ᵣ-OR^{c}, SR^{c}, -SF₅, CN, NO₂, -(CR^{f}R^{f})ᵣ-NR¹²R¹³, -(CR^{f}R^{f})ᵣ-C(O)R^{c}, -(CR^{f}R^{f})r-CO₂R^{c}, -(CR^{f}R^{f})ᵣ-C(O)NR¹²R¹³, -C(O)NR¹⁴(CR^{f}R^{f})ₜN¹²R¹³, -(CR^{f}R^{f})ᵣ-OC(O)NR¹²R¹³, -(CR^{f}R^{f})ᵣ-NR¹⁴C(O)NR¹²R¹³, -(CR^{f}R^{f})ᵣ-NR¹⁴C(O)R^{d}, -(CR^{f}R^{f})ᵣ-NR¹⁴C(O)OR^{h}, -NR¹⁴(CR^{f}R^{f})ₙC(0)R^{d}, -NR¹⁴CO(CR^{f}Rf)ₙOR^{c}, -(CH₂)ᵣ-CR¹³(=NOR^{c}), -S(O)ₚNR¹²R¹³, -(CR^{f}R^{f})ᵣ-NR¹⁴S(O)ₚNR¹²R¹³, -NR¹⁴SO₂CF₃, -NR¹⁴S(O)ₚR^{d}, -S(O)₂CF₃, -S(O)R^{d}, -S(O)₂R^{d}, -OP(O)(Oet)₂, -O(CH₂)₂OP(O)(Oet)₂, 4,4,5,5-tetramethyl-1,3,2-dioxaborolanyl, C₁₋₈ alkyl substituted with 0-2 R^{a}, C₂₋₈ alkenyl substituted with 0-2 R^{a}, C₂₋₈ alkynyl substituted with 0-2 R^{a}, -(CR^{f}R^{f})ᵣ-C₃₋₁₃ carbocycle substituted with 0-5 R^{b}, or -(CR^{f}R^{f})ᵣ-5- to 10-membered heterocycle comprising: carbon ring atoms and 1-4 ring heteroatoms selected from N, NR¹¹, O, and S(O)ₚ, wherein said heterocycle is substituted with 0-5 R^{b};
   alternatively, two R¹s on two adjacent carbon atoms are combined with the carbon atoms to which they attached, form a 5- to 7-membered carbocycle or heterocycle comprising: carbon ring atoms and 0-3 additional ring heteroatoms selected from N, NR¹¹, O, and S(O)ₚ, and 0-2 carbonyl groups, wherein said carbocycle or heterocycle is substituted with 0-4 R^{b};
   R⁶ is -(CR^{f}R^{f})ₙ-phenyl substituted with 0-3 R^{6a} or -(CR^{f}R^{f})ₙ-pyridyl substituted with 0-3 R^{6a};
   R^{6a} is, independently at each occurrence, F, Cl, Br, I,-(CRⁱRⁱ)ᵣ-OR^{c}, SR^{c}, -SF₅, CN, NO₂, CF₃, OCF₃,-CF₂CF₃, -OCF₂CF₂H, -OCF₂CF₃, -NR¹²R¹³, -C(O)R^{c}, -C(O)OR^{c}, -C(O)NR¹²R¹³, -NR¹⁴C(O)R^{d}, -S(O)ₚNR¹²R¹³, -S(O)R^{d}, -S(O)₂R^{d}, Si(Me)₃, Si(C₁₋₄ alkyl)₃, C₁₋₄ haloalkyl, C₁₋₄ haloalkyloxy-, C₁₋₄ alkyloxy-, C₁₋₄ alkylthio-, C_{1-C4} alkyl-C(O)-, C₁₋₄ alkyl-O-C(O)-, C₁₋₄ alkyl-C(O)NH-, C₁₋₈ alkyl substituted with 0-2 R^{a}, C₂₋₈ alkenyl substituted with 0-2 R^{a}, C₂₋₈ alkynyl substituted with 0-2 R^{a}, -(CR^{f}R^{f})ᵣ-C₃₋₁₀ carbocycle substituted with 0-2 R^{e}, or -(CR^{f}R^{f})ᵣ-5- to 10-membered heterocycle comprising: carbon ring atoms and 1-4 ring heteroatoms selected from N, NR¹¹, O, and S(O)ₚ, wherein said heterocycle is substituted with 0-2 R^{e};
   alternatively, when two R^{6a} groups are attached to adjacent atoms, together with the atoms to which they are attached they form a 5- to 7-membered carbocyclic or heterocyclic ring comprising: carbon ring atoms and 0-2 ring heteroatoms selected from N, NR¹¹, O, and S(O)ₚ, 0-1 carbonyl and 0-3 ring double bonds, wherein said carbocyclic or heterocyclic ring is substituted with 0-2 R^{b};
   R⁷ is, independently at each occurrence, F, Cl, Br, I, OCF₃, CF₃, OR^{c}, SR^{c}, -SF₅, CN, NO₂, -NR¹²R¹³, -C(O)R^{c}, -C(O)OR^{c}, -C(O)NR¹²R¹³, -NR¹⁴C(O)R^{d}, -S(O)ₚNR¹²R¹³, -S(O)R^{d}, -S(O)₂R^{d}, C₁₋₈ alkyl substituted with 0-2 R^{a}, C₂₋₈ alkenyl substituted with 0-2 R^{a}, C₂₋₈ alkynyl substituted with 0-2 R^{a}, -(CR^{f}R^{f})ᵣ-C₃₋₁₀ carbocycle substituted with 0-3 R^{b}, or -(CR^{f}R^{f})ᵣ-5- to 10-membered heterocycle comprising: carbon ring atoms and 1-4 ring heteroatoms selected from N, NR^{7b}, O, and S(O)ₚ, wherein said heterocycle is substituted with 0-3 R^{b};
   alternatively, two R⁷s on two adjacent carbon atoms form a 5- to 7-membered carbocyclic or heterocyclic ring comprising: carbon ring atoms and 0-3 ring heteroatoms selected from O, N, NR^{7b}, and S(O)ₚ, wherein said carbocyclic or heterocyclic ring is substituted with 0-2 R^{7c};
   R^{7b} is H, C₁₋₄ alkyl, -C(O)(C₁₋₄ alkyl), -C(O)phenyl, -C(O)benzyl, or benzyl;
   R^{7c} is, independently at each occurrence, H, F, Cl, Br, I, OCF₃, CF₃, OR^{c}, SR^{c}, -SF₅, CN, NO₂, -NR¹²R¹³, -C(O)R^{c}, -C(O)OR^{c}, -C(O)NR¹²R¹³, -NR¹⁴C(O)R^{d}, -S(O)ₚNR¹²R¹³, -S(O)R^{d}, -S(O)₂R^{d}, C₁₋₄ alkyl, phenyl substituted with 0-3 R^{b}, or benzyl substituted with 0-3 R^{b};
   R¹¹ is, independently at each occurrence, H, C₁₋₄ alkoxy, C₁₋₈ alkyl substituted with 0-2 R^{a}, C₂₋₄ alkenyl substituted with 0-1 R^{a}, C₂₋₄ alkynyl substituted with 0-1 R^{a},-C(O)(C₁₋₆ alkyl), -C(O)(CH₂)ₙ(C₃-₆ cycloalkyl), -C(O)(CH₂)ₙ(C₆₋₁₀ aryl), -C(O)(CH₂)ₙ(5- to 10-membered heteroaryl), -C(O)O(C₁₋₈ alkyl), -C(O)O(CH₂)ₙ(C₃₋₆ cycloalkyl), -C(O)O(CH₂)ₙ(C₆₋₁₀ aryl), -C(O)O(CH₂)ₙ(5- to 10-membered heteroaryl), -C(O)O(CH₂)₂₋₄(C₁₋₄ alkyl), -C(O)NH(C₁₋₈ alkyl), -C(O)NH(CH₂)ₙ(C₃₋₆ cycloalkyl), -C(O)NH(CH₂)ₙ(C₆₋₁₀ aryl), -C(O)NH(CH₂)ₙ(5- to 10-membered heteroaryl), -S(O)₂(C₁₋₈ alkyl), -S(O)₂(CH₂)ₙ(C₃₋₆ cycloalkyl), -S(O)₂(CH₂)ₙ(C₆₋₁₀ aryl), -S(O)₂(CH₂)ₙ(5- to 10-membered heteroaryl), -(CR^{f}R^{f})ᵣ-C₃₋₁₀ carbocycle, or -(CR^{f}R^{f})ᵣ-5- to 10-membered heterocycle; wherein said alkyl, cycloalkyl, phenyl, aryl, and carbocycle are substituted with 0-2 R^{b}, and said heteroaryl and heterocycle are substituted with 0-2 R^{b} and comprise: carbon ring atoms and 1-4 ring heteroatoms selected from N, NR^{f}, O, and S(O)ₚ;
   R¹² is, independently at each occurrence, H, C₁₋₆ alkyl, -C(O)(C₁₋₆ alkyl), -C(O)(CH₂)ₙ(C₆₋₁₀ aryl), -C(O)(CH₂)ₙ(5- to 10-membered heteroaryl), -C(O)O(C₁₋₄ alkyl), -C(O)OCH₂(C₆₋₁₀ aryl), -(CH₂)ₙC(O)OCH₂(5- to 10-membered heteroaryl), -(CH₂)ₙOC(O)(C₁₋₄ alkyl), -(CH₂)ₙOC(O)(C₆₋₁₀ aryl), -(CH₂)ₙOC(O)(5- to 10-membered heteroaryl),-(CH₂)ₙC(O)O(C₁₋₄ alkyl), -(CH₂)ₙC(O)O(C₆₋₁₀ aryl), -(CH₂)ₙC(O)O(5- to 10-membered heteroaryl), -(CH₂)ₙC(O)NH(C₁₋₆ alkyl), -(CH₂)ₙC(O)NH(C₆₋₁₀ aryl), -(CH₂)ₙC(O)NH(5- to 10-membered heteroaryl), -(CH₂)ₜOC(O)NH(C₁₋₆ alkyl), -(CH₂)ₜOC(O)NH(C₆₋₁₀ aryl), -(CH₂)ₜOC(O)NH(5- to 10-membered heteroaryl), -S(O)₂(C₁₋₆ alkyl), -S(O)₂(CH₂)ₙ(C₆₋₁₀ aryl), -S(O)₂(CH₂)ₙ(5- to 10-membered heteroaryl), -(CR^{f}R^{f})ₙ-(C₆₋₁₀ aryl), -(CR^{f}R^{f})ₙ- 5- to 10-membered heteroaryl, or -(CR^{f}R^{f})ₙ- 5- to 10-membered non-aromatic heterocycle; wherein said alkyl, and aryl are substituted with 0-2 R^{g}; and said heteroaryl and non-aromatic heterocycle are substituted with 0-2 R^{g} and comprises: carbon ring atoms and 1-4 ring heteroatoms selected from N, NR¹¹, O, and S(O)ₚ;
   R¹³ is, independently at each occurrence, H, C₁₋₆ alkyl, or -(CH₂)ₙ-phenyl;
   alternatively, R¹² and R¹³, when attached to the same nitrogen, combine to form a 5-to 10-membered heterocyclic ring comprising: carbon ring atoms and 1-2 additional ring heteroatoms selected from N, NR¹¹, O, and S(O)ₚ;
   R¹⁴ is, independently at each occurrence, H, C₁₋₆ alkyl substituted with 0-2 R^{14a}, C₂₋₆ alkenyl substituted with 0-2 R^{14a}, C₂₋₆ alkynyl substituted with 0-2 R^{14a}, -(CH₂)ᵣ-C₃₋₁₀ carbocycle substituted with 0-3 R^{g}, or -(CH₂)ᵣ-5- to 10-membered heterocycle comprising: carbon ring atoms and 1-4 ring heteroatoms selected from N, NR¹¹, O, and S(O)ₚ, wherein said heterocycle is substituted with 0-3 R^{g};
   R^{14a} is, independently at each occurrence, H, C₁₋₄ alkyl, OR^{f},Cl, F, Br, I, =O, CF₃, CN, NO₂, -C(O)R^{f} -C(O)OR^{f}, -C(O)NR¹²R¹³, or -S(O)ₚR^{f};
   R¹⁶ is, independently at each occurrence, H, F, C₁₋₆ alkyl substituted with 0-2 R^{a}, C₂₋₆ alkenyl substituted with 0-2 R^{a}, C₂₋₆ alkynyl substituted with 0-2 R^{a}, or -(CH₂)ᵣ-phenyl substituted with 0-2 R^{b};
   R¹⁷ is, independently at each occurrence, H, C₁₋₆ alkyl, or-(CH₂)ₙ-phenyl;
   alternatively, R¹⁶ and R¹⁷ on the same carbon atom combine to form a 3- to 7-membered carbocyclic or heterocyclic ring comprising: carbon ring atoms and 0-2 ring heteroatoms selected from N, NR¹¹, O, and S(O)ₚ, 0-1 carbonyl, and 0-3 double bonds, wherein said carbocyclic or heterocyclic ring is substituted with 0-2 R^{b};
   alternatively, two R¹⁶ groups on adjacent atoms combine to form a 3- to 7-membered carbocyclic or heterocyclic ring comprising: carbon ring atoms and 0-2 ring heteroatoms selected from N, NR¹¹, O, and S(O)ₚ, 0-1 carbonyl, and 0-3 double bonds, wherein said carbocyclic or heterocyclic ring is substituted with 0-2 R^{b};
   R¹⁸ is, independently at each occurrence, H, F, or C₁₋₆ alkyl;
   R¹⁹ is, independently at each occurrence, H, OH, -C(O)OR^{f} or C₁₋₆ alkyl;
   R^{a} is, independently at each occurrence, F, OCF₃, CF₃, OR^{c}, SR^{c}, CN, -NR¹²R¹³, -C(O)R^{c}, -C(O)OR^{c}, -C(O)NR¹²R¹³, -NR¹⁴C(O)R^{d}, -S(O)ₚNR¹²R¹³, -S(O)R^{d}, -S(O)₂R^{d}, -(CH₂)ᵣ-C₃₋₁₀ carbocycle substituted with 0-3 R^{e}, or -(CH₂)ᵣ-5- to 10-membered heterocycle comprising: carbon ring atoms and 1-4 ring heteroatoms selected from N, NR^{f}, O, and S(O)ₚ, wherein said heterocycle is substituted with 0-3 R^{e};
   R^{b} is, independently at each occurrence, H, =O, F, Cl, Br, I, -(CH₂)ᵣ-OR^{c}, SR^{c}, -SF₅, CN, NO₂, CF₃, OCF₃, -(CH₂)ᵣ-NR¹²R¹³, -C(O)R^{c}, -(CH₂)ᵣ-C(O)OR^{c}, -(CH₂)ᵣ-C(O)NR¹²R¹³, -NR¹⁴C(O)R^{d}, -S(O)ₚNR¹²R¹³, -S(O)R^{d}, -S(O)₂R^{d}, C₁₋₄ haloalkyl, C₁₋₄ haloalkyloxy-, C₁₋₄ alkyloxy-, C₁₋₄ alkylthio-, C₁₋₄ alkyl-C(O)-, C₁₋₄ alkyl-O-C(O)-, C₁₋₄ alkyl-C(O)NH-, C₁₋₈ alkyl substituted with 0-2 R^{a}, C₂₋₈ alkenyl substituted with 0-2 R^{a}, C₂₋₈ alkynyl substituted with 0-2 R^{a}, -(CH₂)ᵣ-C₃₋₁₀ carbocycle substituted with 0-3 R^{e}, or-(CH₂)ᵣ-5- to 10-membered heterocycle comprising: carbon ring atoms and 1-4 ring heteroatoms selected from N, NR^{f}, O, and S(O)ₚ, wherein said heterocycle is substituted with 0-3 R^{e};
   R^{c} is, independently at each occurrence, H, -OP(O)(OEt)₂, C₁₋₈ alkyl substituted with 0-2 R^{e}, C₂₋₈ alkenyl substituted with 0-2 R^{e}, C₂₋₈ alkynyl substituted with 0-2 R^{e}, -(CR^{f}R^{f})ᵣ-C₃₋₈ cycloalkyl substituted with 0-2 R^{e}, -(CR^{f}R^{f})ᵣ-C₆₋₁₀ aryl substituted with 0-2 R^{e}, or-(CR^{f}R^{f})ᵣ-5- to 10-membered heterocycle comprising: carbon ring atoms and 1-4 ring heteroatoms selected from N, NR^{f}, O, and S(O)ₚ, wherein said heterocycle is substituted with 0-2 R^{e};
   R^{d} is, independently at each occurrence, CF₃, OH, C₁₋₄ alkoxy, C₁₋₆ alkyl, -(CH₂)ᵣ-C₃₋₁₀ carbocycle substituted with 0-2 R^{e}, or-(CH₂)ᵣ-5- to 10- membered heterocycle comprising: carbon ring atoms and 1-4 ring heteroatoms selected from N, NR^{f}, O, and S(O)ₚ, wherein said heterocycle is substituted with 0-2 R^{e};
   R^{e} is, independently at each occurrence, H, =O, -(CH₂)ᵣ-OR^{f}, F, Cl, Br, I, CN, NO₂, -(CH₂)ᵣ-NR¹²R¹³, -C(O)R^{f}, -(CH₂)ᵣ-C(O)OR^{f}, -NR¹⁴C(O)R^{f}, -(CH₂)ᵣ-C(O)NR¹²R¹³, -SO₂NR¹²R¹³, -NR¹⁴SO₂NR¹²R¹³, -NR¹⁴SO₂-C₁₋₄ alkyl, -NR¹⁴SO₂CF₃, -NR¹⁴SO₂-phenyl, -S(O)₂CF₃, -S(O)ₚ-OR^{h}, -(CF₂)ᵣCF₃, Si(Me)₃, Si(Me)₂(*t*-Bu), Si(C₁₋₄ alkyl)₃, C₁₋₈ alkyl substituted with 0-2 R^{g}, C₂₋₈ alkenyl substituted with 0-2 R^{g}, C₂₋₈ alkynyl substituted with 0-2 R^{g}, -(CH₂)ᵣ-C₃₋₈ cycloalkyl substituted with 0-2 R^{g}, -(CH₂)ᵣ-C₆₋₁₀ aryl substituted with 0-2 R^{g}, or-(CH²)ᵣ-5- to 10-membered heterocycle comprising: carbon ring atoms and 1-4 ring heteroatoms selected from N, NR^{f}, O, and S(O)ₚ, wherein said heterocycle is substituted with 0-2 R^{g};
   alternatively, two R^{e} groups on the same carbon atom combine to form a 3- to 7-membered carbocyclic or heterocyclic ring comprising carbon ring atoms and 0-2 ring heteroatoms selected from N, NR¹¹, O, and S(O)ₚ, 0-1 carbonyl and 0-3 double bonds, wherein said carbocyclic or heterocyclic ring is substituted with 0-2 R^{b};
   alternatively, two R^{e} groups within the same heterocycle combine to form a methylene bridge;
   R^{f} is, independently at each occurrence, H, C₁₋₆ alkyl, or -(CH₂)ₙ-phenyl;
   R^{g} is, independently at each occurrence, H, =O, OR^{f}, F, Cl, Br, I, CN, NO₂, -NR^{f}R^{f}. -C(O)R^{f}, -C(O)OR^{f}, -NR^{f}C(O)R^{f}, -C(O)NR^{f}R^{f}, -SO₂NR^{f}R^{f}, -NR^{f}SO₂NR^{f}R^{f}, -NR^{f}SO₂-C₁₋₄ alkyl, -NR^{f}SO₂CF₃, -NR^{f}SO₂-phenyl, -S(O)₂CF₃, -S(O)ₚ-C₁₋₄ alkyl, -S(O)ₚ-phenyl, -(CF₂)ᵣCF₃, C₁₋₆ alkyl, C₂₋₆ alkenyl, or C₂₋₆ alkynyl;
   R^{h} is, independently at each occurrence, C₁₋₆ alkyl substituted with 0-2 R^{g}, -(CH₂)ₙ-phenyl substituted with 0-2 R^{g}, or-(CH₂)ₙ-5- to 10-membered heterocycle comprising: carbon ring atoms and 1-4 ring heteroatoms selected from N, NR^{f}, O, and S(O)ₚ, wherein said heterocycle is substituted with 0-2 R^{g};
   R¹ is, independently at each occurrence, H, C₁₋₆ alkyl substituted. with 0-2 R^{g}, -(CH₂)ₙ-phenyl substituted with 0-2 R^{g}, or-(CH₂)ₙ-5- to 10-membered heterocycle comprising: carbon ring atoms and 1-4 ring heteroatoms selected from N, NR^{f}, O, and S(O)ₚ, wherein said heterocycle is substituted with 0-2 R^{g};
      n, at each occurrence, is selected from 0, 1, 2, 3, and 4;
      p, at each occurrence, is selected from 0, 1, and 2;
      r, at each occurrence, is selected from 0, 1, 2, 3, and 4;
      s, at each occurrence, is selected from 0, 1, 2, and 3; and
      t, at each occurrence, is selected from 1, 2, 3, and 4;
      for use in a method for treating and/or preventing a CNS disorder.
2. The compound for use according to aspect 1, wherein:
   X₂ is a bond, -CH₂-, -CH₂CH₂-, -CHMe-, -CH₂CHMe-, -CH₂CO-, 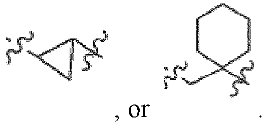
3. The compound for use according to aspect 1 or 2, wherein:
   Y is O, S, NH, -OCH₂-, -OCHMe-, -OCH(CO₂Me)-, -CH=CH-, or -CONH-.
4. The compound for use according to any one of aspects 1 to 3, wherein:
   W is O; and
   Y is O, S, or NH.
5. The compound for use according to any one of aspects 1 to 4, wherein:
   ring A is substituted with 0-5 R¹ and selected from: phenyl, pyridinyl, pyrimidinyl, furanyl, isoxazolyl, thiazolyl, thiadiazolyl, benzothiazolyl, indolyl, and benzimidazolyl.
6. The compound for use according to any one of aspects 1 to 5, wherein:
   ring A is substituted with 0-5 R¹ and selected from: 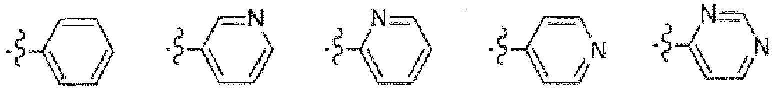 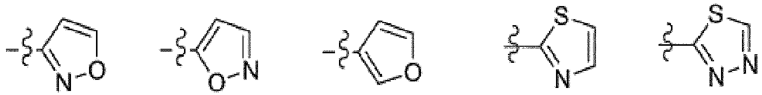 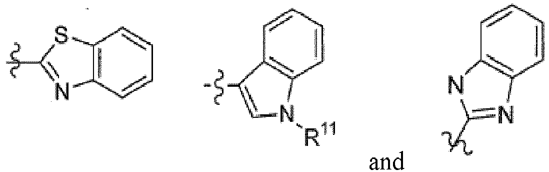
7. The compound for use according to any one of aspects 1 to 6, wherein:
   ring B is substituted with 0-3 R⁷ and selected from: 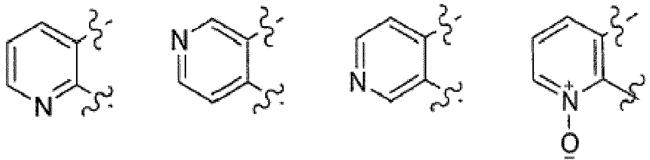 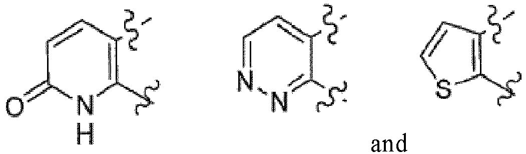
8. The compound for use according to any one of aspects 1 to 7, wherein:
   R¹ is, independently at each occurrence, F, Cl, Br, I, CF₃,-CF₂CF₃, OCF₃, -OCF₂CF₂H, -OCF₂CF₃, SiMe₃, -(CR^{f}R^{f})ᵣ-OR^{c}, SR^{c}, CN, NO₂, -(CR^{f}R^{f})ᵣ-NR¹²R¹³, -(CR^{f}R^{f})ᵣ-C(O)R^{c}, -(CR^{f}R^{f})ᵣ-CO₂R^{c}, -(CR^{f}R^{f})ᵣ-C(O)NR¹²R¹³, -OP(O)(OEt)₂, 4,4,5,5-tetramethyl-1,3,2-dioxaborolanyl, C₁₋₈ alkyl substituted with 0-2 R^{a}, C₂₋₈ alkenyl substituted with 0-2 R^{a}, C₂₋₈ alkynyl substituted with 0-2 R^{a}, -(CR^{f}R^{f})ᵣ-C₃₋₁₃ carbocycle substituted with 0-5 R^{b}, or-(CR^{f}R^{f})ᵣ-5- to 10-membered heterocycle comprising: carbon ring atoms and 1-4 ring heteroatoms selected from N, NR¹¹, O, and S(O)ₚ, wherein said heterocycle is substituted with 0-5 R^{b},
   alternatively, two R¹s on two adjacent carbon atoms are combined with the carbon atoms to which they attached, form a 5- to 7-membered carbocycle or heterocycle comprising: carbon ring atoms and 0-3 additional ring heteroatoms selected from N, NR¹¹, O, and S(O)ₚ, and 0-2 carbonyl groups, wherein said carbocycle or heterocycle is substituted with 0-4 R^{b}.
9. The compound for use according to any one of aspects 1 to 8, wherein:
   R⁶ is -(CH₂)ₙ-phenyl substituted with 0-3 R^{6a} or -(CH₂)ₙ-pyridyl substituted with 0-3 R^{6a}; and
   R^{6a} is, independently at each occurrence, F, Cl, Br, I, -(CRⁱRⁱ)ᵣ-OR^{c}, SR^{c}, CN, CF₃, OCF₃, -CF₂CF₃, -OCF₂CF₂H, -OCF₂CF₃, -NR¹²R¹³, -C(O)R^{c}, Si(Me)₃, Si(C₁₋₄ alkyl)₃, C₁₋₄ haloalkyl, C₁₋₄ haloalkyloxy-, C₁₋₄ alkyloxy-, C₁₋₄ alkylthio-, C₁-C₄ alkyl-C(O)-, C₁₋₄ alkyl-O-C(O)-, C₁₋₄ alkyl-C(O)NH-, C₁₋₈ alkyl substituted with 0-2 R^{a}, C₂₋₈ alkenyl substituted with 0-2 R^{a}, C₂₋₈ alkynyl substituted with 0-2 R^{a},-(CR^{f}R^{f})ᵣ-C₃₋₁₀ carbocycle substituted with 0-2 R^{e}, or-(CR^{f}R^{f})ᵣ-5- to 10- membered heterocycle comprising: carbon ring atoms and 1-4 ring heteroatoms selected from N, NR¹¹, O, and S(O)ₚ, wherein said heterocycle is substituted with 0-2 R^{e};
   alternatively, when two R^{6a} groups are attached to adjacent atoms, together with the atoms to which they are attached they form a 5- to 7-membered carbocyclic or heterocyclic ring comprising: carbon ring atoms and 0-2 ring heteroatoms selected from N, NR¹¹, O, and S(O)ₚ, 0-1 carbonyl and 0-3 ring double bonds, wherein said carbocyclic or heterocyclic ring is substituted with 0-2 R^{b}.
10. The compound for use according to any one of aspects 1 to 9, wherein:
   R¹¹ is, independently at each occurrence, H, C₁₋₄ alkoxy, C₁₋₈ alkyl substituted with 0-2 R^{a}, -C(O)(C₁₋₆ alkyl), -C(O)(CH₂)ₙ(C₃₋₆ cycloalkyl), -C(O)(CH₂)ₙphenyl, -C(O)O(C₁₋₈ alkyl), -C(O)O(CH₂)ₙ(C₃₋₆ cycloalkyl), -C(O)O(CH₂)ₙphenyl, -C(O)O(CH₂)₂₋₄(C₁₋₄ alkyl), -C(O)NH(C₁₋₆ alkyl), -S(O)₂(C₁₋₆ alkyl), -S(O)₂(CH₂)ₙphenyl, -(CR^{f}R^{f})ᵣ-C₃₋₁₀ carbocycle, or -(CR^{f}R^{f})ᵣ-5 to 10-membered heterocycle; wherein said alkyl, cycloalkyl, phenyl, and carbocycle are substituted with 0-2 R^{b}, and said heterocycle is substituted with 0-2 R^{b} and comprises: carbon ring atoms and 1-4 ring heteroatoms selected from N, NR^{f}, O, and S(O)ₚ.
11. The compound for use according to aspect 1, wherein:
   ring A is substituted with 0-5 R¹ and selected from: 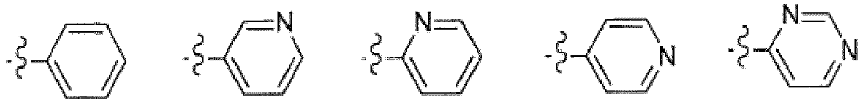 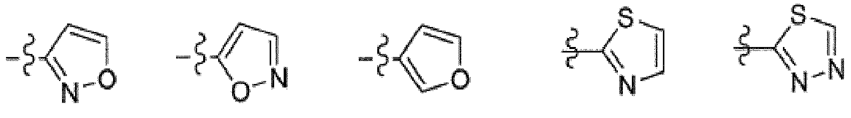 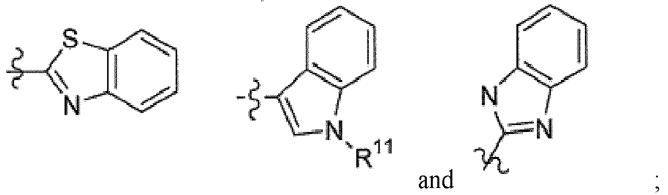
   ring B is substituted with 0-3 R⁷ and selected from: 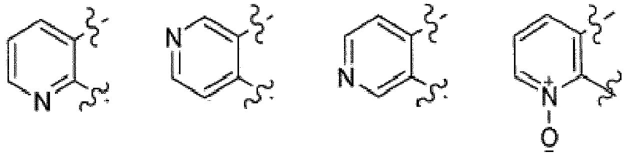 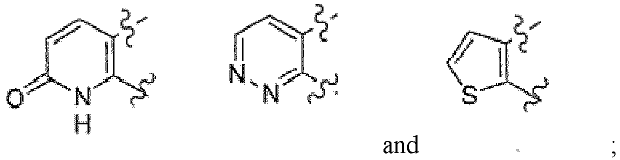
   W is O;
   X₂ is a bond, -CH₂-,-CH₂CH₂-, -CHMe-,-CH₂CHMe-, -CH₂CO-, 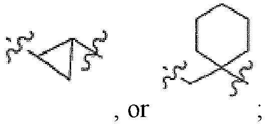
   Y is O, S, NH,-OCH₂-, -OCHMe-, -OCH(CO₂Me)-, -CH=CH-, or-CONH-;
   R¹ is, independently at each occurrence, F, Cl, Br, I, CF₃,-CF₂CF₃, OCF₃, -OCF₂CF₂H, -OCF₂CF₃, SiMe₃, -(CR^{f}R^{f})ᵣ-OR^{c}, SR^{c}, CN, NO₂,-(CR^{f}R^{f})ᵣ-NR¹²R¹³, -(CR^{f}R^{f})ᵣ-C(O)R^{c}, -(CR^{f}R^{f})r-CO₂R^{c}, -(CR^{f}R^{f})ᵣ-C(O)NR¹²R¹³, -OP(O)(OEt)₂, 4,4,5,5-tetramethyl-1,3,2-dioxaborolanyl, C₁₋₈ alkyl substituted with 0-2 R^{a}, C₂₋₈ alkenyl substituted with 0-2 R^{a}, C₂₋₈ alkynyl substituted with 0-2 R^{a}, -(CR^{f}R^{f})ᵣ-C₃₋₁₃ carbocycle substituted with 0-5 R^{b}, or-(CR^{f}R^{f})ᵣ-5- to 10-membered heterocycle comprising: carbon ring atoms and 1-4 ring heteroatoms selected from N, NR¹¹, O, and S(O)ₚ, wherein said heterocycle is substituted with 0-5 R^{b};
   alternatively, two R¹s on two adjacent carbon atoms are combined with the carbon atoms to which they attached, form a 5- to 7-membered carbocycle or heterocycle comprising: carbon ring atoms and 0-3 additional ring heteroatoms selected from N, NR¹¹, O, and S(O)ₚ, and 0-2 carbonyl groups, wherein said carbocycle or heterocycle is substituted with 0-4 R^{b};
   R⁶ is -(CH₂)ₙ-phenyl substituted with 0-3 R^{6a} or -(CH₂)ₙ-pyridyl substituted with 0-3 R^{6a};
   R^{6a} is, independently at each occurrence, F, Cl, Br, I,-(CRⁱRⁱ)ᵣ-OR^{c}, SR^{c}, CN, CF₃, OCF₃, -NR¹²R¹³, -C(O)R^{c}, Si(Me)₃, Si(C₁₋₄ alkyl)₃, C₁₋₄ haloalkyl, C₁₋₄ haloalkyloxy-, C₁₋₄ alkyloxy-, C₁₋₄ alkylthio-, C₁-C₄ alkyl-C(O)-, C₁₋₄ alkyl-O-C(O)-, C₁₋₄ alkyl-C(O)NH-, C₁₋₈ alkyl substituted with 0-2 R^{a}, C₂₋₈ alkenyl substituted with 0-2 R^{a}, C₂₋₈ alkynyl substituted with 0-2 R^{a}, -(CR^{f}R^{f})ᵣ-C₃₋₁₀ carbocycle substituted with 0-2 R^{e}, or -(CR^{f}R^{f})ᵣ-5- to 10-membered heterocycle comprising: carbon ring atoms and 1-4 ring heteroatoms selected from N, NR¹¹, O, and S(O)ₚ, wherein said heterocycle is substituted with 0-2 R^{e};
   alternatively, when two R^{6a} groups are attached to adjacent atoms, together with the atoms to which they are attached they form a 5- to 7-membered carbocyclic or heterocyclic ring comprising: carbon ring atoms and 0-2 ring heteroatoms selected from N, NR¹¹, O, and S(O)ₚ, 0-1 carbonyl and 0-3 ring double bonds, wherein said carbocyclic or heterocyclic ring is substituted with 0-2 R^{b}; and
   R¹¹ is, independently at each occurrence, H, C₁₋₄ allcoxy, C₁₋₈ alkyl substituted with 0-2 R^{a}, -C(O)(C₁₋₆ alkyl), -C(O)(CH₂)ₙ(C₃₋₆ cycloalkyl), -C(O)(CH₂)ₙphenyl, -C(O)O(C₁₋₈ alkyl), -C(O)O(CH₂)ₙ(C₃₋₆ cycloalkyl), -C(O)O(CH₂)ₙphenyl, -C(O)O(CH₂)₂₋₄(C₁₋₄ alkyl), -C(O)NH(C₁₋₆ alkyl), -S(O)₂(C₁₋₆ alkyl), -S(O)₂(CH₂)ₙphenyl, -(CR^{f}R^{f})ᵣ-C₃₋₁₀ carbocycle, or -(CR^{f}R^{f})ᵣ-5- to 10-membered heterocycle; wherein said alkyl, cycloalkyl, phenyl, and carbocycle are substituted with 0-2 R^{b}, and said heterocycle is substituted with 0-2 R^{b} and comprises: carbon ring atoms and 1-4 ring heteroatoms selected from N, NR^{f}, O, and S(O)ₚ.
12. A compound that (a) is of Formula (Ia): 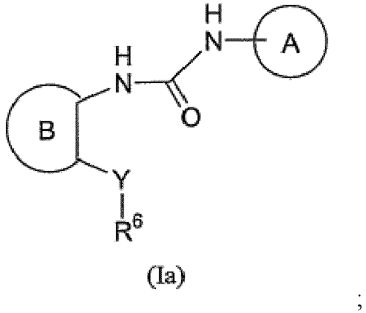 or (b) is a tautomer, N-oxide, pharmaceutically acceptable salt, or solvate thereof; wherein:
   ring A is 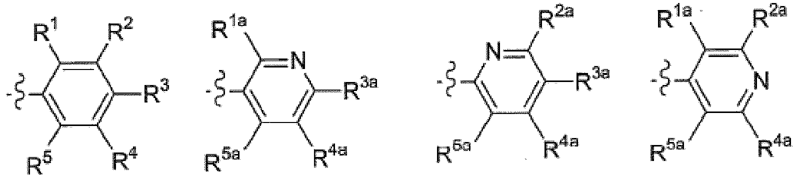 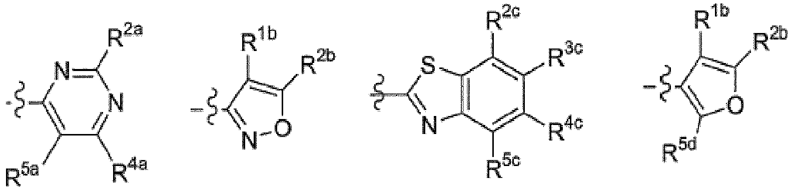 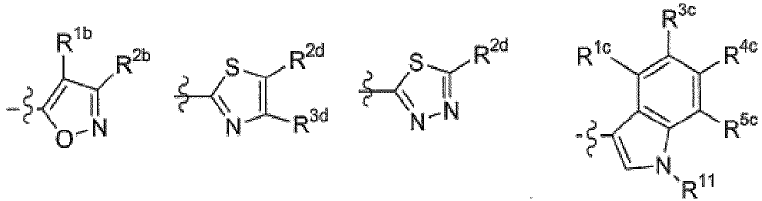 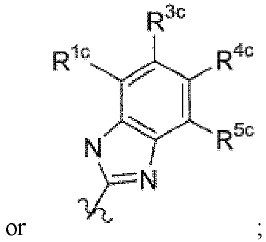
   ring B is 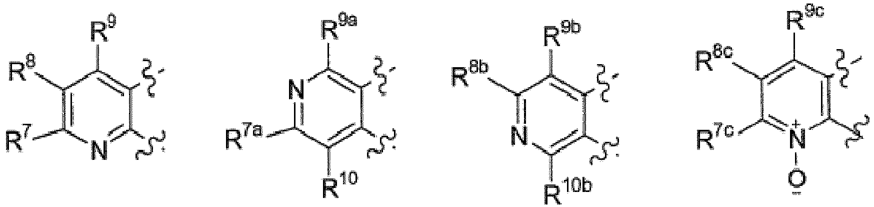 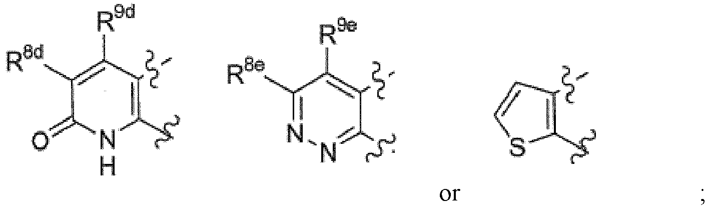
   R¹, R^{1a}, R^{1b}, and R^{1c} are, independently at each occurrence, H, F, Cl, Me, NH₂, or OH;
   R², R^{2a}, R^{2b}, R^{2c}, R^{2d} and R^{3d} are, independently at each occurrence, H, F, Cl, Br, Me, *t*-Bu, OMe, OBu, pentoxy, isopentoxy, neohexoxy, -O(CH₂)₂OMe, -O(CH₂)₂O(*i*-Pr), -O(CH₂)₈CO₂Me, -O(CH₂)₂C(Me)₂OMe, -O(CH₂)₂NMe₂, -OCH₂C(Me)₂CH₂NMe₂, -O(CH₂)₂OCOMe, -OCH(Et)CH₂OMe, -OCH(Me)CH₂O(*t*-Bu), NO₂, CF₃, OCF₃, 2-CH₂N(Me)₂-Ph, cyclopenoxy, cyclohexoxy, 4-Me-cyclohexoxy, cyclohexylmethoxy, cyclohexylethoxy, phenyl, phenoxy, benzoxy, 2-OMe-benzoxy, 3-OMe-benzoxy, 4-OMe-benzoxy, 4-Cl-benzoxy, 3-CN-benzoxy, 4-CN-benzoxy, 3-NMe₂-benzoxy, 2-CF₃-benzoxy, 3-OCF₃-benzoxy, 4-OCF₃-benzoxy, 4-CO₂Me-benzoxy, 4-NHCOMe-benzoxy, 4-Ph-benzoxy, (2-naphthyl)methoxy, (1-Bn-pyrrolidin-3-yl)oxy, (1-Bn-pyrrolidin-3-yl)methoxy, (furan-2-yl)methoxy, (furan-3-yl)methoxy, (tetrahydrofuran-2-yl)methoxy, (tetrahydrofuran-3-yl)methoxy, (thien-3-yl)methoxy, (IH-pyrrol-1-yl)ethoxy, (2-Bu-1H-imidazol-4-yl)methoxy, (1-Ph-1H-1,2,3-triazol-4-yl)methoxy, 1-Bn-piperidin-3-oxy, 1-Bn-piperidin-4-oxy, (4-Bn-morpholin-2-yl)methoxy, (pyridin-2-yl)methoxy, (pyridin-3-yl)methoxy, (pyridin-4-yl)methoxy, (pyridin-2-yl)ethoxy, (pyridin-4-yl)ethoxy, or -OCH(Et)(pyridine-4-yl);
   R³, R^{3a}, R^{3c}, R⁴, R^{4a}, R^{4c}, R⁵, R^{5a}, R^{5c}, and R^{5d}, are, independently at each occurrence, H, F, Cl, Br, -OCH(Me)CH₂O-*t*-Bu, CF₃, OCHF₂, OCF₃, -O(CH₂)₂OMe, -O(CH₂)₃NMe₂, -O(CH₂)₄NMe₂, -OCH(Et)CH₂OMe, CN, NH₂, NMe₂,-CH₂NMe₂, NEt₂, -NHPh, -N(Me)Ph, -NH(4-OMe-Ph), -NH(2-CF₃-Ph), -CH(Me)NHCH(Me)Ph, -CH(Me)N(Me)(3-CF₃-Bn), -CH(Me)N(Me)(furan-2-ylmethyl), -CH(Me)N(Me)(thien-2-ylmethyl), -CH(Me)OH, -CH(Me)O(*i*-Pr), -CH(Me)O(*i*-Bu), -CH(Me)O(3-CF₃-Bn), -CH(Me)O(4-CF₃-Bn), -CH(Me)O(1-Bn-pyrrolidin-3-ylmethyl), -CH(Me)OCH₂C(Me)₂CH₂NMe₂, -CH(Me)OBn, -CH(Me)O(4-*i*-Pr-Bn), -CH(Me)O(4-OPh-Bn), -CH(Me)O(3,5-diCl-Bn), -CH(Me)OCH₂(1-Bn-piperidin-4-yl), -CH₂NHBn, -CH₂NH(4-CF₃-Bn), -CH₂N(Me)Bn, -CH(Me)NHCH₂-pyridin-2-yl, -CH(Me)NHCH₂-pyridin-4-yl, -CH(Me)NHCH₂(6-Cl-pyridin-3-yl), -CH(Me)N(Me)(*i*-Bu), -CH(Me)N(Me)Bn, -CH(Me)N(Me)(4-OMe-Bn), -CH(Me)N(Me)(4-F-Bn), -CH(Me)N(Me)(3-Cl-Bn), -CH(Me)N(Me)(4-Cl-Bn), -CH(Me)N(Me)(3,4-diCl-Bn), -CH(Me)N(Me)CH₂CH₂Ph, -CH(Me)N(Me)CH₂-pyridin-2-yl, -CH(Me)N(Me)CH₂-pyridin-3-yl, -CH(Me)N(Me)CH₂-pyridin-4-yl, -CH(Me)N(Me)CH₂-furan-2-yl, -CH(Me)N(Me)CH₂-thien-2-yl, -CH(Me)N(Me)CH₂-(5-Me-thien-2-yl), -CH(Me)N(Me)CH₂-(5-Cl-thien-2-yl), -CH(Me)N(Et)Bn, -CH(Me)N(Et)(4-Me-Bn), -CH(Me)N(Et)(2-Cl-Bn), -CH(Me)N(Bn)CH₂CN, -CH(Me)N(Bn)CH₂CH₂OH, -CH(Me)N(Bn)CH₂CO₂Me, -CH(Me)N(Bn)CH₂CONMe₂, -CH(Me)N(Bn)CH₂CON(Me)(Bn), -CH(Me)-isoindolin-2-yl, -CH(Me)-(1,2,3,4-tetrahydroisoquinolin-2-yl), -CH(Me)(4-Bn-piperazin-1-yl), -C(CF₃)₂OH, -COMe, CO₂Et, -CH₂CO₂Me, -C(Me)₂CO₂Me, -O(CH₂)₅CO₂Et, -O(CH₂)₈CO₂Me, -O(CH₂)₂C(Me)₂OMe, -O(CH₂)₂OCOMe, -OCH₂C(Me)₂CH₂NMe₂, Ph, 2-CH₂OH-Ph, 2-CH₂N(Me)₂-Ph, 3-CH₂N(Me)₂-Ph, 4-CH₂N(Me)₂-Ph, 2-((3-OH-pyrrolidin-1-yl)methyl)-Ph, phenoxy, Bn, benzoxy, 4-Cl-benzoxy, 2-OMe-benzoxy, 3-OMe-benzoxy, 2-OMe-benzoxy, 3-CN-benzoxy, 4-CN-benzoxy, 3-NMe₂-benzoxy, 4-CO₂Me-benzoxy, 3-CF₃-benzoxy, 3-OCF₃-benzoxy, 4-OCF₃-benzoxy, 4-Ph-benzoxy, 2,4-diF-benzoxy, (2-naphthyl)methoxy, cyclohexylmethoxy, cyclohexylethoxy, cyclopentoxy, 3-Me-cyclopentoxy, cyclohexoxy, 4-Me-cyclohexoxy, 4-CO₂Et-cyclohexoxy, 1-Bn-pyrrolidin-3-oxy, (1-Bn-pyrrolidin-3-yl)methoxy, (furan-2-yl)methoxy, (furan-3-yl) methoxy, (tetrahydrofuran-3-yl)methoxy, (thien-3-yl)methoxy, thiazol-2-yl, 1H-pyrazol-1-yl, 3-CO₂Et-5-Me-1H-pyrazol-1-yl, 4-CO₂Et-5-Me-1H-pyrazol-1-yl, 5-CO₂Et-3-Me-1H-pyrazol-1-yl, (2-Bu-1H-imidazol-4-yl)methoxy, 1H-1,2,4-triazol-1-yl, (1-Ph-1H-1,2,3-triazol-4-yl)methoxy, 2-(1H-pyrrol-1-yl)-ethoxy, 1-piperidinyl, 1-Bn-piperazin-4-yl, (2,2-dimethyl-1,3-dioxolan-4-yl)-methoxy, 1-Bn-piperidin-3-oxy, 1-Bn-piperidin-4-oxy, (1-(*i*-Bu)-piperidin-4-yl)methoxy, (1-isopentyl-piperidin-4-yl)methoxy, (1-CO₂(*t*-Bu)-piperidin-4-yl)methoxy, (1-CO₂Bn-piperidin-4-yl)methoxy, (1-Bn-piperidin-4-yl)methoxy, (1-phenethyl-piperidin-4-yl)methoxy, (1-(4-phenylbutyl)-piperidin-4-yl)methoxy, (1-cyclohexylmethyl-piperidin-4-yl)methoxy, (1-((pyridin-2-yl)methyl)-piperidin-4-yl)methoxy, (1-((pyridin-4-yl)methyl)-piperidin-4-yl)methoxy, (1-((1,3-dioxolan-2-yl)methyl)piperidin-4-yl)methoxy, N-morpholinyl, (pyridin-2-yl)methoxy, (pyridin-3 -yl)methoxy, (pyridin-4-yl)methoxy, (pyridin-4-yl)ethoxy, (4-Bn-morpholin-2-yl)methoxy, 4,4,5,5-tetramethyl-1,3,2-dioxaborolanyl, -OP(O)(OEt)₂, C₁₋₆ alkyl, C₁₋₆ alkoxy, or C₃₋₆ cycloalkyl optionally substituted with the group selected from: -CO₂Me, -CH₂OH, and - CH₂OMe;
   alternatively, R¹ + R², R² + R³, R³ + R⁴, R⁴ + R⁵, R^{1a} + R^{2a},
   R^{2a} + R^{3a}, R^{3a} + R^{4a}, R^{4a} + R^{5a}, R^{1b} + R^{2b} R^{1c} + R^{3c}, R^{2c} + R^{3c}, R^{2d} + R^{3d}, R^{3c} + R^{4c}, or R^{4c} + R^{5c}, combine with the carbon atoms to which they attached, form 5- to 10-membered carbocyclic or heterocyclic ring comprising: carbon ring atoms and 0-3 ring heteroatoms selected from N, NR¹¹, O, and S(O)ₚ, 0-1 carbonyl group, and additional 0-3 double bonds, wherein said carbocyclic or heterocyclic ring is substituted with 0-2 R^{b};
   R⁶ is -(CH₂)ₙ-phenyl substituted with 0-3 R^{6a} or -(CH₂)ₙ-pyridyl substituted with 0-3 R^{6a};
   R^{6a} is, independently at each occurrence, H, F, Cl, Br, I, CN,-C(Me)₂CN, C₁₋₈ alkyl, C₂₋₈ alkenyl, OH, SMe, S(*i*-Pr), -C(Me)₂OMe, -C(Me)₂OEt, -C(Me)₂OPr, -CHMeO(CH₂)₂OMe, -C(Me)₂OBu, -C(Me)₂O(CH₂)₂OMe, -C(Me)(OMe)CH₂OMe, -C(Me)₂O(CH₂)₂N(*i*-Bu)₂, -C(Me)₂O(CH₂)₂S(*i*-Bu), -C(Me)₂O(CH₂)₂S(O)(*i*-Bu), -C(Me)₂O(CH₂)₂S(furan-2-ylmethyl), -C(Me)₂O(CH₂)₂S(pyridin-2-yl), -C(Me)₂O(CH₂)₂S(O)₂(pyridin-2-yl), -C(Me)₂CH₂OSi(Me)₂(*t*-Bu), -C(Me)₂O(CH₂)₂Si(Me)₂(*t*-Bu), -C(Et)₂OH, -C(Pr)₂OH, -C(CH₂CH=CH₂)₂OH, -C(CH₂CH=CH₂)₂OMe, -C(Et)₂OMe, -C(Et)₂OEt, -C(Et)₂OPr, COMe, COPh, CO₂Me, CO₂Et, -NH(*i*-Bu), -CH=CHCO₂(*t*-Bu), -OCH₂CO₂(*t*-Bu), CF₃, OCF₃, C₁₋₄ alkyloxy, C₃₋₇ cycloalkyl, C₃₋₇ cycloalkenyl, Ph, Bn, naphthyl, 1-pyrrolidinyl, 5-isoxazolyl, N-morpholinyl, 4-Bn-piperazinyl, 1-piperidinyl, 1-Bn-piperidin-4-yl, 1-*i*-Bu-piperidin-4-yl, 1-neopentyl-piperidin-4-yl, 1-COPh-piperidin-4-yl, -SiMe₃, 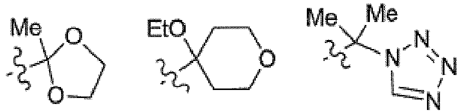 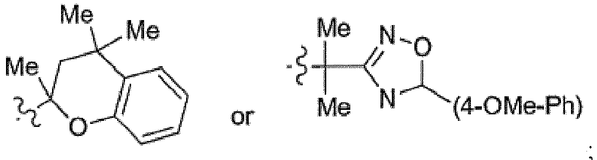
   alternatively, when two R^{6a} groups are attached to adjacent atoms, together with the atoms to which they are attached they form a 5- to 7-membered carbocyclic or heterocyclic ring comprising: carbon ring atoms and 0-2 ring heteroatoms selected from N, NR¹¹, O, and S(O)ₚ, 0-1 carbonyl and 0-3 ring double bonds, wherein said carbocyclic or heterocyclic ring is substituted with 0-2 R^{b};
   R⁷, R^{7a}, and R^{7c} are, independently at each occurrence, H, Me, Cl, Br, CN, OH, OMe, SMe, NHMe, NMe₂, CO₂Me, imidazol-1-yl, or-CH₂NH(CO)H;
   R⁸, R^{8b}, R^{8c}, R^{8d}, and R^{8e} are, independently at each occurrence, H, Me, Cl, Br, CN, or CF₃;
   R⁹, R^{9a}, R^{9b}, R^{9c}, R^{9d}, and R^{9e} are, independently at each occurrence, H or Me;
   R¹⁰ and R^{10a} are, independently at each occurrence, H or Me;
   R¹¹ is, independently at each occurrence, H, C₁₋₆ alkyl, OMe, -C(O)(C₁₋₆ alkyl),-C(O)phenyl, -C(O)benzyl, -C(O)O(C₁₋₆ alkyl),-C(O)Obenzyl, -S(O)₂(C₁₋₆ alkyl), -S(O)₂phenyl, -S(O)₂benzyl, cyclohexylmethyl, phenyl, benzyl, phenethyl, phenylpropyl, -CH₂CH(Me)Ph, 1H-pyrrol-2-ylmethyl, 1-Me-pyrrol-2-ylmethyl, thieny-2-ylmethyl, furan-2-ylmethyl, furan-3-ylmethyl, 2-F-Bn, 2-OH-Bn, 2-CN-Bn, 3-CN-Bn, 4-CN-Bn, 4-OMe-Bn, 4-CO₂Me-Bn, 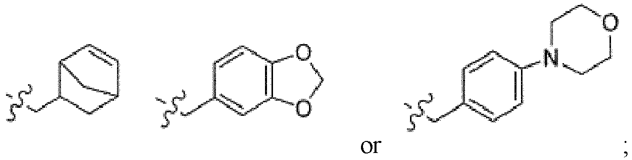
   Y is O, S, or NH;
   R^{b} is, independently at each occurrence, H, F, Cl, Br, C₁₋₄ alkyl, OH, CO₂H, NH₂, CF₃, OCF₃, C₁₋₄ alkyloxy, C₃₋₇ cycloalkyl, phenyl, or benzyl;
   n, at each occurrence, is selected from 0, 1, and 2;
   p, at each occurrence, is selected from 0, 1, and 2;
   for use in a method for treating and/or preventing a CNS disorder.
13. The compound for use according to aspect 12, wherein:
   R¹, R^{1a}, R^{1b}, and R^{1c} are, independently at each occurrence, H, F, Cl, or OH;
   R², R^{2a}, R^{2b}, R^{2c}, R^{2d} and R^{3d} are, independently at each occurrence, H, F, Cl, Br, Me, *t*-Bu, OMe, OBu, pentoxy, isopentoxy, neohexoxy,-O(CH₂)₂OMe, -O(CH₂)₂O(*i*-Pr), -O(CH₂)₈CO₂Me, -O(CH₂)₂C(Me)₂OMe, -O(CH₂)₂NMe₂, -OCH₂C(Me)₂CH₂NMe₂, -O(CH₂)₂OCOMe, -OCH(Et)CH₂OMe, -OCH(Me)CH₂O(*t*-Bu), NO₂, CF₃, OCF₃, 2-CH₂N(Me)₂-Ph, cyclopenoxy, cyclohexoxy, 4-Me-cyclohexoxy, cyclohexylmethoxy, cyclohexylethoxy, phenyl, phenoxy, benzoxy, 2-OMe-benzoxy, 3-OMe-benzoxy, 4-OMe-benzoxy, 4-Cl-benzoxy, 3-CN-benzoxy, 4-CN-benzoxy, 3-NMe₂-benzoxy, 2-CF₃-benzoxy, 3-OCF₃-benzoxy, 4-OCF₃-benzoxy, 4-CO₂Me-benzoxy, 4-NHCOMe-benzoxy, 4-Ph-benzoxy, (2-naphthyl)methoxy, (1-Bn-pyrrolidin-3-yl)oxy, (1-Bn-pyrrolidin-3-yl)methoxy, (furan-2-yl)methoxy, (furan-3-yl)methoxy, (tetrahydrofuran-2-yl) methoxy, (tetrahydrofuran-3-yl)methoxy, (thien-3-yl)methoxy, (1H-pyrrol-1-yl)ethoxy, (2-Bu-1H-imidazol-4-yl)methoxy, (1-Ph-1H-1,2,3-triazol-4-yl)methoxy, 1-Bn-piperidin-3-oxy, 1-Bn-piperidin-4-oxy, (4-Bn-morpholin-2-yl)methoxy, (pyridin-2-yl)methoxy, (pyridin-3-yl)methoxy, (pyridin-4-yl)methoxy, (pyridin-2-yl)ethoxy, (pyridin-4-yl) ethoxy, or -OCH(Et)(pyridine-4-yl);
   R³, R^{3a}, R^{3c}, and R^{4c} are, independently at each occurrence, H, F, Cl, Br, -OCH(Me)CH₂O-*t*-Bu, CF₃, OCHF₂, OCF₃, -O(CH₂)₂OMe, -O(CH₂)₃NMe₂, -O(CH₂)₄NMe₂, -OCH(Et)CH₂OMe, CN, NH₂, NMe₂,-CH₂NMe₂, NEt₂,-NHPh, -N(Me)Ph, -NH(4-OMe-Ph), -NH(2-CF₃-Ph), -CH(Me)NHCH(Me)Ph, -CH(Me)N(Me)(3-CF₃-Bn), -CH(Me)N(Me)(furan-2-ylmethyl), -CH(Me)N(Me)(thien-2-ylmethyl), -CH(Me)OH, -CH(Me)O(*i*-Pr), -CH(Me)O(*i*-Bu), -CH(Me)O(3-CF₃-Bn), -CH(Me)O(4-CF₃-Bn), -CH(Me)O(1-Bn-pyrrolidin-3-ylmethyl), -C(Me)₂OH, -C(Me)₂CH₂OH, -C(CF₃)₂OH, -COMe, CO₂Et, -CH₂CO₂Me, -C(Me)₂CO₂Me, -O(CH₂)₅CO₂Et, -O(CH₂)₈CO₂Me, -O(CH₂)₂C(Me)₂OMe, -O(CH₂)₂OCOMe, -OCH₂C(Me)₂CH₂NMe₂, Ph, 2-CH₂OH-Ph, 2-CH₂N(Me)₂-Ph, 3-CH₂N(Me)₂-Ph, 4-CH₂N(Me)₂-Ph, 2-((3-OH-pyrrolidin-1-yl)methyl)-Ph, phenoxy, Bn, benzoxy, 4-Cl-benzoxy, 2-OMe-benzoxy, 3-OMe-benzoxy, 4-OMe-benzoxy, 3-CN-benzoxy, 4-CN-benzoxy, 3-NMe₂-benzoxy, 4-CO₂Me-benzoxy, 3-CF₃-benzoxy, 3-OCF₃-benzoxy, 4-OCF₃-benzoxy, 4-Ph-benzoxy, 2,4-diF-benzoxy, (2-naphthyl)methoxy, cyclohexylmethoxy, cyclohexylethoxy, cyclopentoxy, 3-Me-cyclopentoxy, cyclohexoxy, 4-Me-cyclohexoxy, 4-CO₂Et-cyclohexoxy, 1-Bn-pyrrolidin-3-oxy, (1-Bn-pyrrolidin-3-yl)methoxy, (furan-2-yl)methoxy, (furan-3-yl)methoxy, (tetrahydrofuran-3-yl)methoxy, (thien-3-yl)methoxy, thiazol-2-yl, 1H-pyrazol-1-yl, 3-CO₂Et-5-Me-1H-pyrazol-1-yl, 4-CO₂Et-5-Me-1H-pyrazol-1-yl, 5-CO₂Et-3-Me-1H-pyrazol-1-yl, (2-Bu-1H-imidazol-4-yl)methoxy, 1H-1,2,4-triazol-1-yl, (1-Ph-1H-1,2,3-triazol-4-yl)methoxy, 2-(1H-pyrrol-1-yl)-ethoxy, 1-piperidinyl, 1-Bn-piperazin-4-yl, (2,2-dimethyl-1,3-dioxolan-4-yl)-methoxy, 1-Bn-piperidin-3-oxy, 1-Bn-piperidin-4-oxy, (1-(*i*-Bu)-piperidin-4-yl)methoxy, (1-isopentyl-piperidin-4-yl)methoxy, (1-CO₂(*t*-Bu)-piperidin-4-yl)methoxy, (1-CO₂Bn-piperidin-4-yl)methoxy, (1-Bn-piperidin-4-yl)methoxy, (1-phenethyl-piperidin-4-yl)methoxy, (1-(4-phenylbutyl)-piperidin-4-yl)methoxy, (1-cyclohexylmethyl-piperidin-4-yl)methoxy, (1-((pyridin-2-yl)methyl)-piperidin-4-yl)methoxy, (1-((pyridin-4-yl)methyl)-piperidin-4-yl)methoxy, (1-((1,3-dioxolan-2-yl)methyl)piperidin-4-yl)methoxy, N-morpholinyl, (pyridin-2-yl)methoxy, (pyridin-3 -yl)methoxy, (pyridin-4-yl)methoxy, (pyridin-4-yl)ethoxy, (4-Bn-morpholin-2-yl)methoxy,
   4,4,5,5-tetramethyl-1,3,2-dioxaborolanyl, -OP(O)(OEt)₂, C₁₋₆ alkyl, C₁₋₆ alkoxy, or C₃₋₆ cycloalkyl optionally substituted with the group selected from: - CO₂Me,
   -CH₂OH, and-CH₂OMe; and
   R⁴, R^{4a}, R⁵, R^{5a}, R^{5c}, and R^{5d}, are, independently at each occurrence, H, F, Cl, Me, or OMe;
   alternatively, R¹ + R², R² + R³, R³ + R⁴, R⁴ + R⁵, R^{1a} + R^{2a},
   R^{2a} + R^{3a}, R^{3a} + R^{4a}, R^{4a} + R^{5a}, R^{1b} + R^{2b}, R^{1c} + R^{3c}, R^{2c} + R^{3c}, R^{2d} + R^{3d},
   R^{3c} + R^{4c}, or R^{4c} + R^{5c}, combine with the carbon atoms to which they attached, form 5- to 10-membered carbocyclic or heterocyclic ring comprising: carbon ring atoms and 0-3 ring heteroatoms selected from N, NR¹¹, 0, and S(O)ₚ, 0-1 carbonyl group, and additional 0-3 double bonds, wherein said carbocyclic or heterocyclic ring is substituted with 0-2 R^{b}.
14. The compound for use according to aspect 12, wherein:
   ring A is 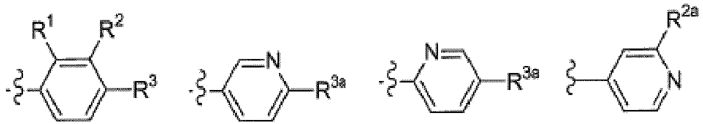 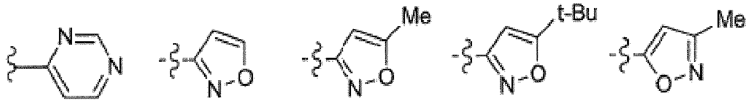 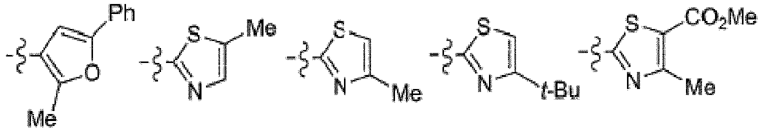 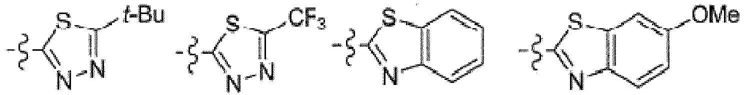 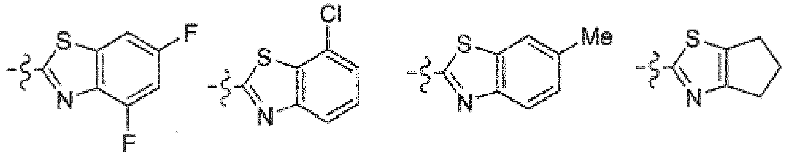 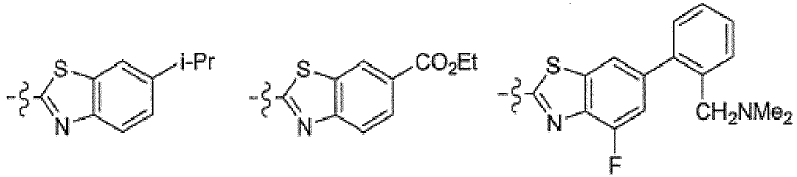 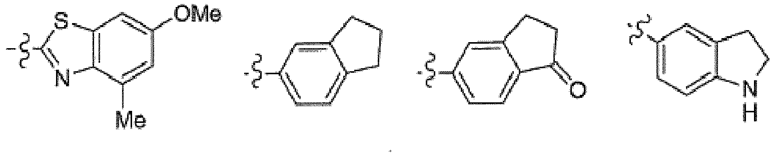 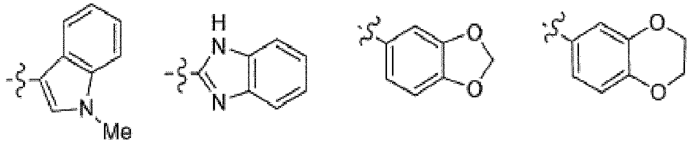 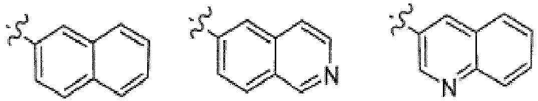 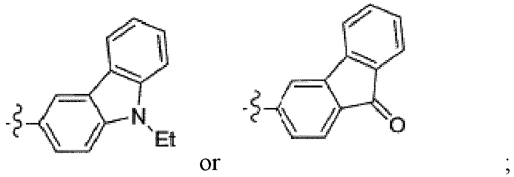
   R¹ is H, or F;
   R² is H, F, Cl, Br, Me, *t*-Bu, OMe, OBu, pentoxy, isopentoxy, neohexoxy, -O(CH₂)₂OMe, -O(CH₂)₂O(*i*-Pr), -O(CH₂)₈CO₂Me, -O(CH₂)₂C(Me)₂OMe, -O(CH₂)₂NMe₂, -OCH₂C(Me)₂CH₂NMe₂, -O(CH₂)₂OCOMe, -OCH(Et)CH₂OMe, -OCH(Me)CH₂O(*t*-Bu), NO₂, CF₃, OCF₃, 2-CH₂N(Me)₂-Ph, cyclopenoxy, cyclohexoxy, 4-Me-cyclohexoxy, cyclohexylmethoxy, cyclohexylethoxy, phenyl, phenoxy, benzoxy, 2-OMe-benzoxy, 3-OMe-benzoxy, 4-OMe-benzoxy, 4-Cl-benzoxy, 3-CN-benzoxy, 4-CN-benzoxy, 3-NMe₂-benzoxy, 2-CF₃-benzoxy, 3-OCF₃-benzoxy, 4-OCF₃-benzoxy, 4-CO₂Me-benzoxy, 4-NHCOMe-benzoxy, 4-Ph-benzoxy, (2-naphthyl)methoxy, (1-Bn-pyrrolidin-3-yl)oxy, (1-Bn-pyrrolidin-3-yl)methoxy, (furan-2-yl)methoxy, (furan-3-yl)methoxy, (tetrahydrofuran-2-yl)methoxy, (tetrahydrofuran-3 -yl)methoxy, (thien-3 -yl)methoxy, (1H-pyrrol-1-yl)ethoxy, (2-Bu-1H-imidazol-4-yl)methoxy, (1-Ph-1H-1,2,3-triazol-4-yl)methoxy, 1-Bn-piperidin-3-oxy, 1-Bn-piperidin-4-oxy, (4-Bn-morpholin-2-yl)methoxy, (pyridin-2-yl)methoxy, (pyridin-3-yl)methoxy, (pyridin-4-yl)methoxy, (pyridin-2-yl)ethoxy, (pyridin-4-yl)ethoxy, or -OCH(Et)(pyridine-4-yl);
   R^{2a} is F, Cl, Br, Me, or *t*-Bu;
   R³ is H, F, Cl, Br, Me, Et, Pr, Bu, *t*-Bu, OMe, OEt, OPr, O-*i*-Pr, OBu, O-*t*-Bu, pentoxy, isopentoxy, neohexoxy, -OCH(Me)CH₂O-*t*-Bu, CF₃, OCHF₂, OCF₃, -O(CH₂)₂OMe, -O(CH₂)₃NMe₂,-O(CH₂)₄NMe₂, -OCH(Et)CH₂OMe, CN, NH₂, NMe₂, -CH₂NMe₂, NEt₂, -NHPh,-N(Me)Ph, -NH(4-OMe-Ph), -NH(2-CF₃-Ph), -CH(Me)NHCH(Me)Ph, -CH(Me)N(Me)(3-CF₃-Bn), -CH(Me)N(Me)(furan-2-ylmethyl), -CH(Me)N(Me)(thien-2-ylmethyl), -CH(Me)OH, -CH(Me)O(*i*-Pr), -CH(Me)O(*i*-Bu), -CH(Me)O(3-CF₃-Bn), -CH(Me)O(4-CF₃-Bn), -CH(Me)O(1-Bn-pyrrolidin-3-ylmethyl), -C(Me)₂OH, -C(Me)₂CH₂OH, -C(CF₃)₂OH, -COMe, CO₂Et, -CH₂CO₂Me, -C(Me)₂CO₂Me, -O(CH₂)₅CO₂Et, -O(CH₂)₈CO₂Me, -O(CH₂)₂C(Me)₂OMe, -O(CH₂)₂OCOMe, -OCH₂C(Me)₂CH₂NMe₂, Ph, 2-CH₂OH-Ph, 2-CH₂N(Me)₂-Ph, 3-CH₂N(Me)₂-Ph, 4-CH₂N(Me)₂-Ph, 2-((3-OH-pyrrolidin-1-yl)methyl)-Ph, phenoxy, Bn, benzoxy, 4-Cl-benzoxy, 2-OMe-benzoxy, 3-OMe-benzoxy, 4-OMe-benzoxy, 3-CN-benzoxy, 4-CN-benzoxy, 3-NMe₂-benzoxy, 4-CO₂Me-benzoxy, 3-CF₃-benzoxy, 3-OCF₃-benzoxy, 4-OCF₃-benzoxy, 4-Ph-benzoxy, 2,4-diF-benzoxy, (2-naphthyl)methoxy, cyclohexylmethoxy, cyclohexylethoxy, cyclopentoxy, 3-Me-cyclopentoxy, cyclohexoxy, 4-Me-cyclohexoxy, 4-CO₂Et-cyclohexoxy, 1-Bn-pyrrolidin-3-oxy, (1-Bn-pyrrolidin-3-yl)methoxy, (furan-2-yl)methoxy, (furan-3-yl)methoxy, (tetrahydrofuran-3-yl)methoxy, (thien-3-yl)methoxy, thiazol-2-yl, 1H-pyrazol-1-yl, 3-CO₂Et-5-Me-1H-pyrazol-1-yl, 4-CO₂Et-5-Me-1H-pyrazol-1-yl, 5-CO₂Et-3-Me-1H-pyrazol-1-yl, (2-Bu-1H-imidazol-4-yl)methoxy, 1H-1,2,4-triazol-1-yl, (1-Ph-1H-1,2,3-triazol-4-yl)methoxy, 2-(1H-pyrrol-1-yl)-ethoxy, 1-piperidinyl, 1-Bn-piperazin-4-yl, (2,2-dimethyl-1,3-dioxolan-4-yl)-methoxy, 1-Bn-piperidin-3-oxy, 1-Bn-piperidin-4-oxy, (1-(*i*-Bu)-piperidin-4-yl)methoxy, (1-isopentyl-piperidin-4-yl)methoxy, (1-CO₂(*t*-Bu)-piperidin-4-yl)methoxy, (1-CO₂Bn-piperidin-4-yl)methoxy, (1-Bn-piperidin-4-yl)methoxy, (1-phenethyl-piperidin-4-yl)methoxy, (1-(4-phenylbutyl)-piperidin-4-yl)methoxy, (1-cyclohexylmethyl-piperidin-4-yl)methoxy, (1-((pyridin-2-yl)methyl)-piperidin-4-yl)methoxy, (1-((pyridin-4-yl)methyl)-piperidin-4-yl)methoxy, (1-((1,3-dioxolan-2-yl)methyl)piperidin-4-yl)methoxy, N-morpholinyl, (pyridin-2-yl)methoxy, (pyridin-3 -yl)methoxy, (pyridin-4-yl)methoxy, (pyridin-4-yl)ethoxy, (4-Bn-morpholin-2-yl)methoxy, 1-CH₂OH-cyclopropyl, 1-CO₂Me-cyclopropyl, 1-CH₂OMe-cyclopropyl, 1-CO₂Me-cyclobutyl, 1-CO₂Me-cyclopentyl, cyclohexyl, 1-CO₂Me-cyclohexyl, 4,4,5,5-tetramethyl-1,3,2-dioxaborolanyl, or -OP(O)(OEt)₂;
   R^{3a}, is Me, Cl, CF₃,-NHPh, -NH(2-CF₃-Ph), -NH(2-*t*-Bu-Ph), 2-*t*-Bu-phenoxy, or 2-CF₃-phenoxy;
   R⁶ is 2-Me-Ph, 3-Me-Ph, 2-Et-Ph, 3-Et-Ph, 2-Pr-Ph, 2-*i*-Pr-Ph, 3-*i*-Pr-Ph, 2-*i*-Bu-Ph, 2-*t*-Bu-Ph, 3-*t*-Bu-Ph, 2-vinyl-Ph, 2-isopropenyl-Ph, 3-isopropenyl-Ph, 3-Br-Ph, 2-I-Ph, 2-SMe-Ph, 2-S(*i*-Pr)-Ph, 2-C(Me)₂CN-Ph, 2-CF₃-Ph, 3-CF₃-Ph, 2-OCF₃-Ph, 3-OCF₃-Ph, 3-Ph-Ph, 2-Bn-Ph, 2-SiMe₃-Ph, 3-SiMe₃-Ph, 2-C(Me)₂OMe-Ph, 2-C(Me)₂OEt-Ph, 2-C(Me)₂OPr-Ph, 2-CH(Me)O(CH₂)₂OMe-Ph, 2-C(Me)₂O(CH₂)₂OMe-Ph, 2-C(Et)₂OH-Ph, 2-C(Et)₂OMe-Ph, 2-C(Et)₂OEt-Ph, 2-C(Et)₂OPr-Ph, 3-COPh-Ph, 2-CO₂Et-Ph, 3-CO₂Et-Ph, 2-NH(*i*-Bu)-Ph, 2-cyclopropyl-Ph, 2-cyclopentyl-Ph, 2,3-dimethoxy-Ph, 2,3-diCl-Ph, 2,6-diMe-Ph, 2-Me-5-F-Ph, 2-*i*-Pr-5-Me-Ph, 2-*t*-Bu-4-Me-Ph, 2-*t*-Bu-5-Me-Ph, 2-*t*-Bu-6-CN-Ph, 2-F-3-CF₃-Ph, 2-F-5-CF₃-Ph, 2-Cl-5-CF₃-Ph, 2-COMe-3-F-Ph, 2-CO₂Me-3-F-Ph, 2-CF₃-Bn, 1-naphthyl, 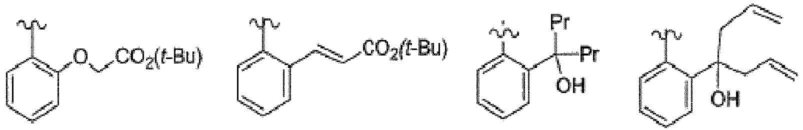 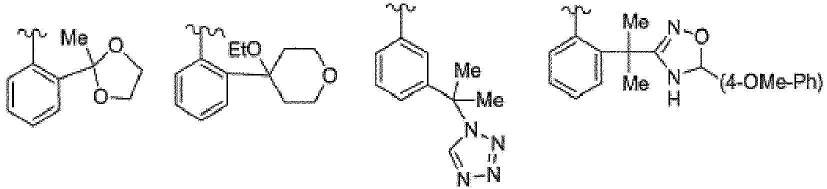 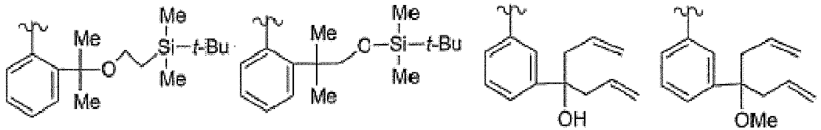 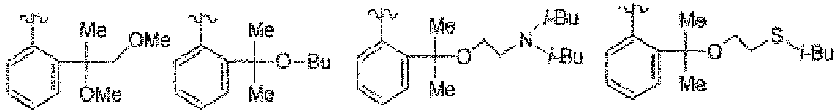 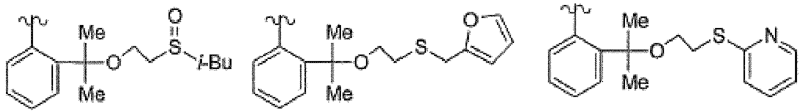 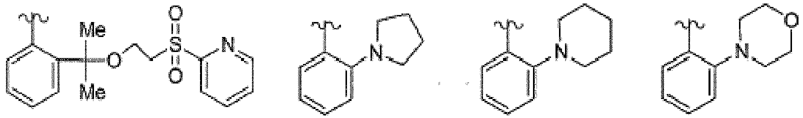 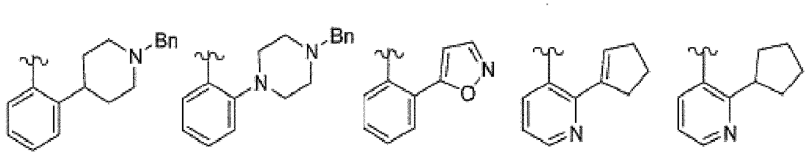 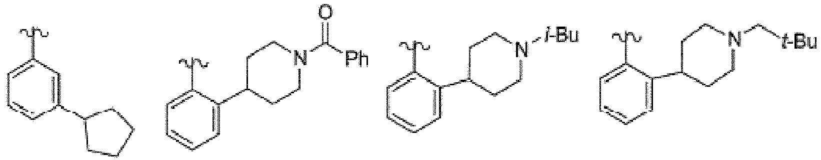 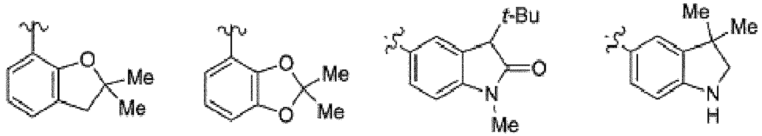 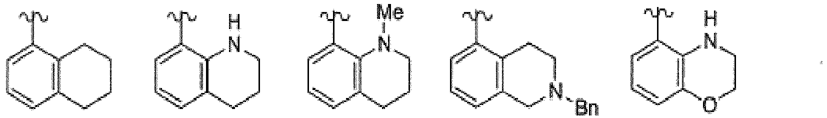 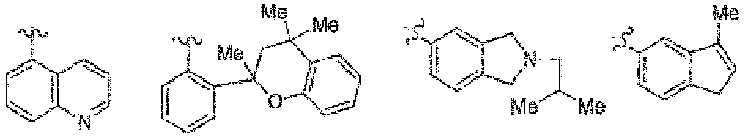 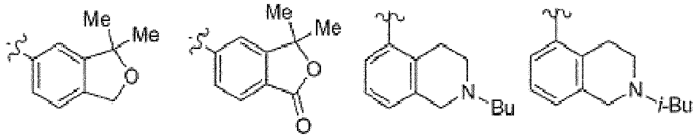 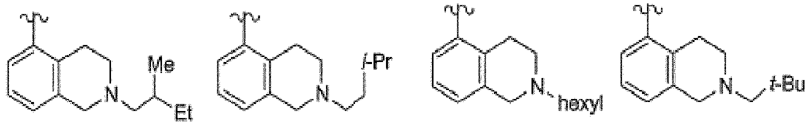 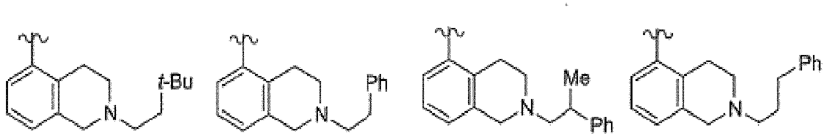 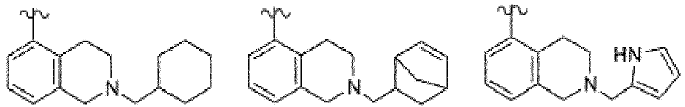 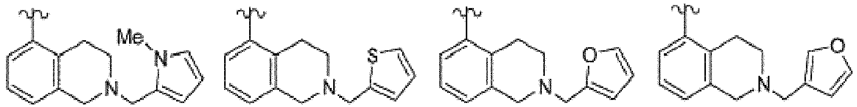 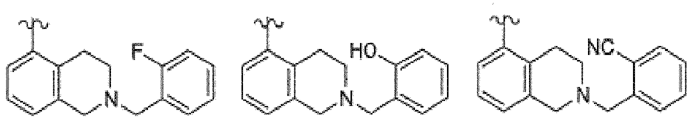 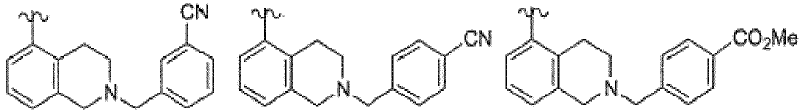 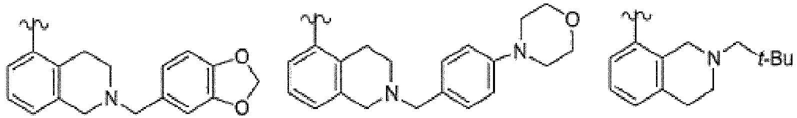 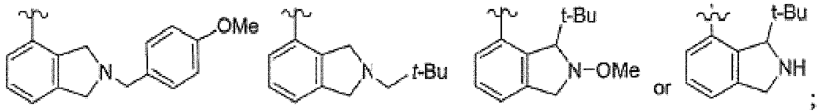
   ring B is 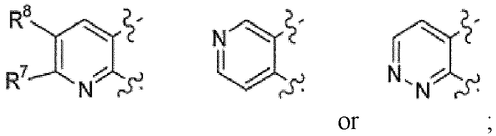
   R⁷ is H, Me, Cl, Br, CN, OH, OMe, SMe, NHMe, NMe₂, CO₂Me, imidazol-1-yl, or-CH₂NH(CO)H;
   R⁸ is H, Me, Cl, Br, CN, or CF₃; and
   Y is O, S, or NH.
15. The compound for use according to aspect 12, wherein:
   ring A is 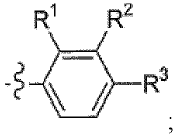
   ring B is 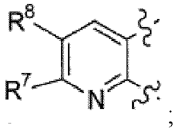 and
   Y is O.
16. A compound that (a) is of Formula (IIb): 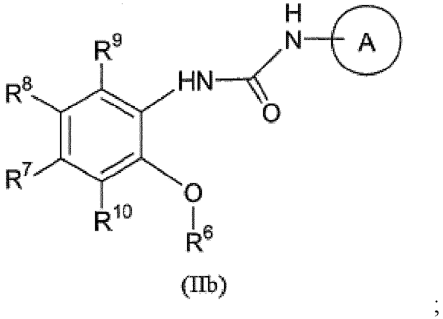 or (b) is a tautomer, N-oxide, pharmaceutically acceptable salt, or solvate thereof; wherein:
   ring A is 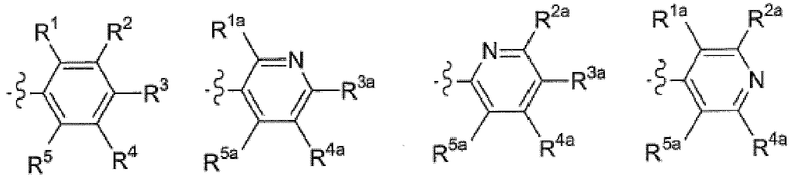 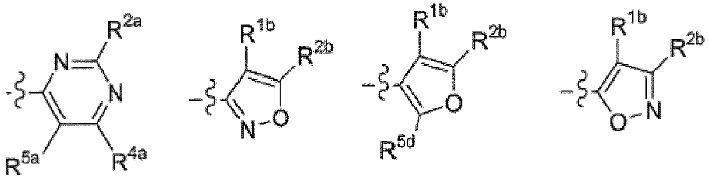 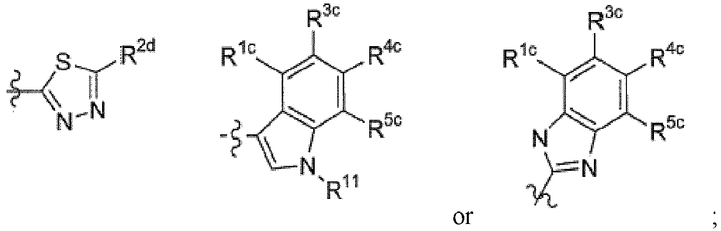
   R¹, R^{1a}, R^{1b}, and R^{1c} are, independently at each occurrence, H, F, Cl, or OH;
   R², R^{2a} R^{2b}, R^{2c}, R^{2d} and R^{3d} are, independently at each occurrence, H, F, Cl, Br, Me, *t*-Bu, OMe, OBu, pentoxy, isopentoxy, neohexoxy, -O(CH₂)₂OMe, -O(CH₂)₂O(*i*-Pr), -O(CH₂)₈CO₂Me, -O(CH₂)₂C(Me)₂OMe, -O(CH₂)₂NMe₂, -OCH₂C(Me)₂CH₂NMe₂, -O(CH₂)₂OCOMe, -OCH(Et)CH₂OMe, -OCH(Me)CH₂O(*t*-Bu), NO₂, CF₃, OCF₃, 2-CH₂N(Me)₂-Ph, cyclopenoxy, cyclohexoxy, 4-Me-cyclohexoxy, cyclohexylmethoxy, cyclohexylethoxy, phenyl, phenoxy, benzoxy, 2-OMe-benzoxy, 3-OMe-benzoxy, 4-OMe-benzoxy, 4-Cl-benzoxy, 3-CN-benzoxy, 4-CN-benzoxy, 3-NMe₂-benzoxy, 2-CF₃-benzoxy, 3-OCF₃-benzoxy, 4-OCF₃-benzoxy, 4-CO₂Me-benzoxy, 4-NHCOMe-benzoxy, 4-Ph-benzoxy, (2-naphthyl)methoxy, (1-Bn-pyrrolidin-3-yl)oxy, (1-Bn-pyrrolidin-3-yl)methoxy, (furan-2-yl)methoxy, (furan-3-yl)methoxy, (tetrahydrofuran-2-yl)methoxy, (tetrahydrofuran-3-yl)methoxy, (thien-3-yl)methoxy, (1H-pyrrol-1-yl)ethoxy, (2-Bu-1H-imidazol-4-yl)methoxy, (1-Ph-1H-1,2,3-triazol-4-yl)methoxy, 1-Bn-piperidin-3-oxy, 1-Bn-piperidin-4-oxy, (4-Bn-morpholin-2-yl)methoxy, (pyridin-2-yl)methoxy, (pyridin-3-yl)methoxy, (pyridin-4-yl)methoxy, (pyridin-2-yl)ethoxy, (pyridin-4-yl)ethoxy, or -OCH(Et)(pyridine-4-yl);
   R³, R^{3a}, R^{3c}, and R^{4c} are, independently at each occurrence, H, F, Cl, Br, -OCH(Me)CH₂O-*t*-Bu, CF₃, OCHF₂, OCF₃, -O(CH₂)₂OMe, -O(CH₂)₃NMe₂, -O(CH₂)₄NMe₂, -OCH(Et)CH₂OMe, CN, NH₂, NMe₂,-CH₂NMe₂, NEt₂,-NHPh, -N(Me)Ph, -NH(4-OMe-Ph), -NH(2-CF₃-Ph), -CH(Me)NHCH(Me)Ph, -CH(Me)N(Me)(3-CF₃-Bn), -CH(Me)N(Me)(furan-2-ylmethyl), -CH(Me)N(Me)(thien-2-ylmethyl), -CH(Me)OH, -CH(Me)O(*i*-Pr), -CH(Me)O(*i*-Bu), -CH(Me)O(3-CF₃-Bn), -CH(Me)O(4-CF₃-Bn), -CH(Me)O(1-Bn-pyrrolidin-3-ylmethyl), -C(CF₃)₂OH,-COMe, CO₂Et, -CH₂CO₂Me, -C(Me)₂CO₂Me, -O(CH₂)₅CO₂Et, -O(CH₂)₈CO₂Me, -O(CH₂)₂C(Me)₂OMe, -O(CH₂)₂OCOMe, -OCH₂C(Me)₂CH₂NMe₂, Ph, 2-CH₂OH-Ph, 2-CH₂N(Me)₂-Ph, 3-CH₂N(Me)₂-Ph, 4-CH₂N(Me)₂-Ph, 2-((3-OH-pyrrolidin-1-yl)methyl)-Ph, phenoxy, Bn, benzoxy, 4-Cl-benzoxy, 2-OMe-benzoxy, 3-OMe-benzoxy, 4-OMe-benzoxy, 3-CN-benzoxy, 4-CN-benzoxy, 3-NMe₂-benzoxy, 4-CO₂Me-benzoxy, 3-CF₃-benzoxy, 3-OCF₃-benzoxy, 4-OCF₃-benzoxy, 4-Ph-benzoxy, 2,4-diF-benzoxy, (2-naphthyl)methoxy, cyclohexylmethoxy, cyclohexylethoxy, cyclopentoxy, 3-Me-cyclopentoxy, cyclohexoxy, 4-Me-cyclohexoxy, 4-CO₂Et-cyclohexoxy, 1-Bn-pyrrolidin-3-oxy, (1-Bn-pyrrolidin-3-yl)methoxy, (furan-2-yl)methoxy, (furan-3 -yl)methoxy, (tetrahydrofuran-3 -yl)methoxy, (thien-3 -yl)methoxy, thiazol-2-yl, 1H-pyrazol-1-yl, 3-CO₂Et-5-Me-1H-pyrazol-1-yl, 4-CO₂Et-5-Me-1H-pyrazol-1-yl, 5-CO₂Et-3-Me-1H-pyrazol-1-yl, (2-Bu-1H-imidazol-4-yl)methoxy, 1H-1,2,4-triazol-1-yl, (1-Ph-1H-1,2,3-triazol-4-yl)methoxy, 2-(1H-pyrrol-1-yl)-ethoxy, 1-piperidinyl, 1-Bn-piperazin-4-yl, (2,2-dimethyl-1,3-dioxolan-4-yl)-methoxy, 1-Bn-piperidin-3-oxy, 1-Bn-piperidin-4-oxy, (1-(*i*-Bu)-piperidin-4-yl)methoxy, (1-isopentyl-piperidin-4-yl)methoxy, (1-CO₂(*t*-Bu)-piperidin-4-yl)methoxy, (1-CO₂Bn-piperidin-4-yl)methoxy, (1-Bn-piperidin-4-yl)methoxy, (1-phenethyl-piperidin-4-yl)methoxy, (1-(4-phenylbutyl)-piperidin-4-yl)methoxy, (1-cyclohexylmethyl-piperidin-4-yl)methoxy, (1-((pyridin-2-yl)methyl)-piperidin-4-yl)methoxy, (1-((pyridin-4-yl)methyl)-piperidin-4-yl)methoxy, (1-((1,3-dioxolan-2-yl)methyl)piperidin-4-yl)methoxy, N-morpholinyl, (pyridin-2-yl)methoxy, (pyridin-3 -yl)methoxy, (pyridin-4-yl)methoxy, (pyridin-4-yl)ethoxy, (4-Bn-morpholin-2-yl)methoxy, 4,4,5,5-tetramethyl-1,3,2-dioxaborolanyl, -OP(O)(OEt)₂, C₁₋₆ alkyl, C₁₋₆ alkoxy, or C₃₋₆ cycloalkyl optionally substituted with the group selected from: -CO₂Me, -CH₂OH, and -CH₂OMe; and
   R⁴, R^{4a}, R⁵, R^{5a}, R^{5c}, and R^{5d}, are, independently at each occurrence, H, F, Cl, Me, or OMe;
   alternatively, R¹ + R², R² + R³, R³ + R⁴, R⁴ + R⁵, R^{1a} + R^{2a}, R^{2a} + R^{3a}, R^{3a}, + R^{4a}, R^{4a}, + R^{5a}, R^{1b} + R^{2b}, R^{1c} + R^{3c}, R^{2c} + R^{3c}, R^{2d} + R^{3d}, R^{3c} + R^{4c}, or R^{4c} + R^{5c}, combine with the carbon atoms to which they attached, form 5- to 10- membered carbocyclic or heterocyclic ring comprising: carbon ring atoms and 0-3 ring heteroatoms selected from N, NR¹¹, O, and S(O)ₚ, 0-1 carbonyl group, and additional 0-3 double bonds, wherein said carbocyclic or heterocyclic ring is substituted with 0-2 R^{b};
   R⁶ is -(CH₂)ₙ-phenyl substituted with 0-3 R^{6a} or -(CH₂)ₙ-pyridyl substituted with 0-3 R^{6a};
   R^{6a} is, independently at each occurrence, H, F, Cl, Br, I, CN, -C(Me)₂CN, C₁₋₈ alkyl, C₂₋₈ alkenyl, OH, SMe, S(*i*-Pr), -C(Me)₂OMe, -C(Me)₂OEt, -C(Me)₂OPr, -CHMeO(CH₂)₂OMe, -C(Me)₂OBu, -C(Me)₂O(CH₂)₂OMe, -C(Me)(OMe)CH₂OMe, -C(Me)₂O(CH₂)₂N(*i*-Bu)₂, -C(Me)₂O(CH₂)₂S(*i*-Bu), -C(Me)₂O(CH₂)₂S(O)(*i*-Bu), -C(Me)₂O(CH₂)₂S(furan-2-ylmethyl), -C(Me)₂O(CH₂)₂S(pyridin-2-yl), -C(Me)₂O(CH₂)₂S(O)₂(pyridin-2-yl), -C(Me)₂CH₂OSi(Me)₂(*t*-Bu), -C(Me)₂O(CH₂)₂Si(Me)₂(*t*-Bu), -C(Et)₂OH, -C(Pr)₂OH, -C(CH₂CH=CH₂)₂OH, -C(CH₂CH=CH₂)₂OMe, -C(Et)₂OMe, -C(Et)₂OEt, -C(Et)₂OPr, COMe, COPh, CO₂Me, CO₂Et, -NH(*i*-Bu), -CH=CHCO₂(*t*-Bu), -OCH₂CO₂(*t*-Bu), CF₃, OCF₃, C₁₋₄ alkyloxy, C₃₋₇ cycloalkyl, C₃₋₇ cycloalkenyl, Ph, Bn, naphthyl, 1-pyrrolidinyl, 5-isoxazolyl, N-morpholinyl, 4-Bn-piperazinyl, 1-piperidinyl, 1-Bn-piperidin-4-yl, 1-*i*-Bu-piperidin-4-yl, 1-neopentyl-piperidin-4-yl, 1-COPh-piperidin-4-yl, -SiMe₃, 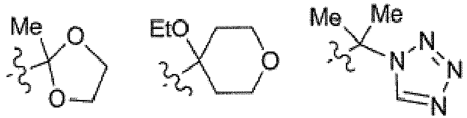 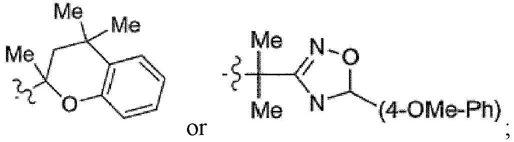
   alternatively, when two R^{6a} groups are attached to adjacent atoms, together with the atoms to which they are attached they form a 5- to 7-membered carbocyclic or heterocyclic ring comprising: carbon ring atoms and 0-2 ring heteroatoms selected from N, NR¹¹, O, and S(O)ₚ, 0-1 carbonyl and 0-3 ring double bonds, wherein said carbocyclic or heterocyclic ring is substituted with 0-2 R^{b};
   R⁷ is H, Me, Cl, Br, CN, OH, OMe, SMe, NHMe, NMe₂, CO₂Me, imidazol-1-yl, or-CH₂NH(CO)H;
   R⁸ is H, Me, Cl, Br, or CN;
   R⁹ is H or Me;
   R¹⁰ is H or Me;
   R¹¹ is, independently at each occurrence, H, C₁₋₆ alkyl, OMe, -C(O)(C₁₋₆ alkyl),-C(O)phenyl, -C(O)benzyl, -C(O)O(C₁₋₆ alkyl), -C(O)Obenzyl, -S(O)₂(C₁₋₆ alkyl), -S(O)₂phenyl, -S(O)₂benzyl, cyclohexylmethyl, phenyl, benzyl, phenethyl, phenylpropyl, -CH₂CH(Me)Ph, 1H-pyrrol-2-ylmethyl, 1-Me-pyrrol-2-ylmethyl, thieny-2-ylmethyl, furan-2-ylmethyl, furan-3-ylmethyl, 2-F-Bn, 2-OH-Bn, 2-CN-Bn, 3-CN-Bn, 4-CN-Bn, 4-OMe-Bn, 4-CO₂Me-Bn, 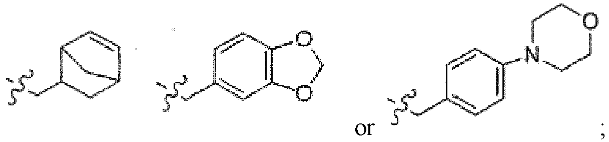
   R^{b} is, independently at each occurrence, H, F, Cl, Br, C₁₋₄ alkyl, OH, CO₂H, NH₂, CF₃, OCF₃, C₁₋₄ alkyloxy, C₃₋₇ cycloalkyl, phenyl, or benzyl;
   n, at each occurrence, is selected from 0, 1, and 2;
   p, at each occurrence, is selected from 0, 1, and 2;
   for use in a method for treating and/or preventing a CNS disorder.
17. The compound for use according to aspect 16, wherein:
   ring A is 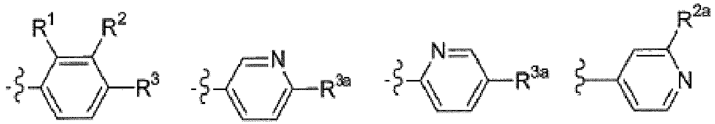 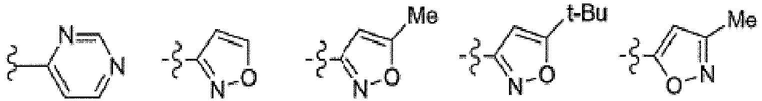 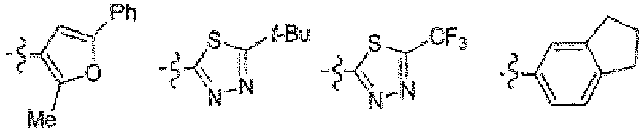 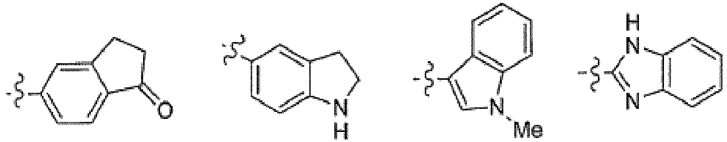 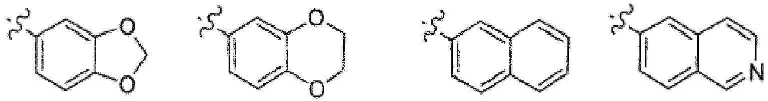 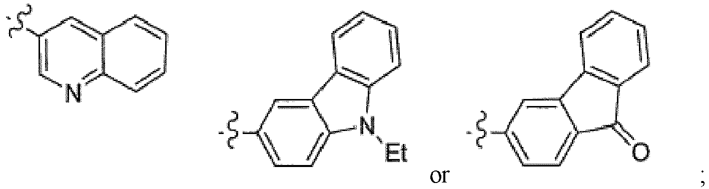
   R¹ is H or F;
   R² is H, F, Cl, Br, Me, *t*-Bu, OMe, OBu, pentoxy, isopentoxy, neohexoxy, -O(CH₂)₂OMe, -O(CH₂)₂O(*i*-Pr), -O(CH₂)₈CO₂Me, -O(CH₂)₂C(Me)₂OMe, -O(CH₂)₂NMe₂, -OCH₂C(Me)₂CH₂NMe₂, -O(CH₂)₂OCOMe, -OCH(Et)CH₂OMe, -OCH(Me)CH₂O(*t*-Bu), NO₂, CF₃, OCF₃, 2-CH₂N(Me)₂-Ph, cyclopenoxy, cyclohexoxy, 4-Me-cyclohexoxy, cyclohexylmethoxy, cyclohexylethoxy, phenyl, phenoxy, benzoxy, 2-OMe-benzoxy, 3-OMe-benzoxy, 4-OMe-benzoxy, 4-Cl-benzoxy, 3-CN-benzoxy, 4-CN-benzoxy, 3-NMe₂-benzoxy, 2-CF₃-benzoxy, 3-OCF₃-benzoxy, 4-OCF₃-benzoxy, 4-CO₂Me-benzoxy, 4-NHCOMe-benzoxy, 4-Ph-benzoxy, (2-naphthyl)methoxy, (1-Bn-pyrrolidin-3-yl)oxy, (1-Bn-pyrrolidin-3-yl)methoxy, (furan-2-yl)methoxy, (furan-3-yl)methoxy, (tetrahydrofuran-2-yl)methoxy, (tetrahydrofuran-3-yl)methoxy, (thien-3-yl) methoxy, (1H-pyrrol-1-yl)ethoxy, (2-Bu-1H-imidazol-4-yl)methoxy, (1-Ph-1H-1,2,3-triazol-4-yl)methoxy, 1-Bn-piperidin-3-oxy, 1-Bn-piperidin-4-oxy, (4-Bn-morpholin-2-yl)methoxy, (pyridin-2-yl)methoxy, (pyridin-3-yl)methoxy, (pyridin-4-yl)methoxy, (pyridin-2-yl)ethoxy, (pyridin-4-yl)ethoxy, or -OCH(Et)(pyridine-4-yl);
   R^{2a} is F, Cl, Br, Me, or *t*-Bu;
   R³ is H, F, Cl, Br, Me, Et, Pr, Bu, *t*-Bu, OMe, OEt, OPr, O-*i*-Pr, OBu, O-*t*-Bu, pentoxy, isopentoxy, neohexoxy, -OCH(Me)CH₂O-*t*-Bu, CF₃, OCHF₂, OCF₃, -O(CH₂)₂OMe, -O(CH₂)₃NMe₂,-O(CH₂)₄NMe₂, -OCH(Et)CH₂OMe, CN, NH₂, NMe₂,-CH₂NMe₂, NEt₂,-NHPh, -N(Me)Ph, -NH(4-OMe-Ph), -NH(2-CF₃-Ph), -CH(Me)NHCH(Me)Ph, -CH(Me)N(Me)(3-CF₃-Bn), -CH(Me)N(Me)(furan-2-ylmethyl), -CH(Me)N(Me)(thien-2-ylmethyl), -CH(Me)OH, -CH(Me)O(*i*-Pr), -CH(Me)O(*i*-Bu), -CH(Me)O(3-CF₃-Bn), -CH(Me)O(4-CF₃-Bn), -CH(Me)O(1-Bn-pyrrolidin-3-ylmethyl), -C(Me)₂OH, -C(Me)₂CH₂OH, -C(CF₃)₂OH, -COMe, CO₂Et,-CH₂CO₂Me, -C(Me)₂CO₂Me, -O(CH₂)₅CO₂Et, -O(CH₂)₈CO₂Me, -O(CH₂)₂C(Me)₂OMe, -O(CH₂)₂OCOMe, -OCH₂C(Me)₂CH₂NMe₂, Ph, 2-CH₂OH-Ph, 2-CH₂N(Me)₂-Ph, 3-CH₂N(Me)₂-Ph, 4-CH₂N(Me)₂-Ph, 2-((3-OH-pyrrolidin-1-yl)methyl)-Ph, phenoxy, Bn, benzoxy, 4-Cl-benzoxy, 2-OMe-benzoxy, 3-OMe-benzoxy, 4-OMe-benzoxy, 3-CN-benzoxy, 4-CN-benzoxy, 3-NMe₂-benzoxy, 4-CO₂Me-benzoxy, 3-CF₃-benzoxy, 3-OCF₃-benzoxy, 4-OCF₃-benzoxy, 4-Ph-benzoxy, 2,4-diF-benzoxy, (2-naphthyl)methoxy, cyclohexylmethoxy, cyclohexylethoxy, cyclopentoxy, 3-Me-cyclopentoxy, cyclohexoxy, 4-Me-cyclohexoxy, 4-CO₂Et-cyclohexoxy, 1-Bn-pyrrolidin-3-oxy, (1-Bn-pyrrolidin-3-yl)methoxy, (furan-2-yl)methoxy, (furan-3-yl)methoxy, (tetrahydrofuran-3-yl)methoxy, (thien-3-yl)methoxy, thiazol-2-yl, 1H-pyrazol-1-yl, 3-CO₂Et-5-Me-1H-pyrazol-1-yl, 4-CO₂Et-5-Me-1H-pyrazol-1-yl, 5-CO₂Et-3-Me-1H-pyrazol-1-yl, (2-Bu-1H-imidazol-4-yl)methoxy, 1H-1,2,4-triazol-1-yl, (1-Ph-1H-1,2,3-triazol-4-yl)methoxy, 2-(1H-pyrrol-1-yl)-ethoxy, 1-piperidinyl, 1-Bn-piperazin-4-yl, (2,2-dimethyl-1,3-dioxolan-4-yl)-methoxy, 1-Bn-piperidin-3-oxy, 1-Bn-piperidin-4-oxy, (1-(*i*-Bu)-piperidin-4-yl)methoxy, (1-isopentyl-piperidin-4-yl)methoxy, (1-CO₂(*t*-Bu)-piperidin-4-yl)methoxy, (1-CO₂Bn-piperidin-4-yl)methoxy, (1-Bn-piperidin-4-yl)methoxy, (1-phenethyl-piperidin-4-yl)methoxy, (1-(4-phenylbutyl)-piperidin-4-yl)methoxy, (1-cyclohexylmethyl-piperidin-4-yl)methoxy, (1-((pyridin-2-yl)methyl)-piperidin-4-yl)methoxy, (1-((pyridin-4-yl)methyl)-piperidin-4-yl)methoxy, (1-((1,3-dioxolan-2-yl)methyl)piperidin-4-yl)methoxy, N-morpholinyl, (pyridin-2-yl)methoxy, (pyridin-3 -yl)methoxy, (pyridin-4-yl)methoxy, (pyridin-4-yl)ethoxy, (4-Bn-morpholin-2-yl)methoxy, 1-CH₂OH-cyclopropyl, 1-CO₂Me-cyclopropyl, 1-CH₂OMe-cyclopropyl, 1-CO₂Me-cyclobutyl, 1-CO₂Me-cyclopentyl, cyclohexyl, 1-CO₂Me-cyclohexyl, 4,4,5,5-tetramethyl-1,3,2-dioxaborolanyl, or -OP(O)(OEt)₂;
   R^{3a}, is Me, Cl, CF₃,-NHPh, -NH(2-CF₃-Ph), -NH(2-*t*-Bu-Ph), 2-*t*-Bu-phenoxy, or 2-CF₃-phenoxy;
   R⁶ is 2-Me-Ph, 3-Me-Ph, 2-Et-Ph, 3-Et-Ph, 2-Pr-Ph, 2-*i*-Pr-Ph, 3-*i*-Pr-Ph, 2-*i*-Bu-Ph, 2-*t*-Bu-Ph, 3-*t*-Bu-Ph, 2-vinyl-Ph, 2-isopropenyl-Ph, 3-isopropenyl-Ph, 3-Br-Ph, 2-I-Ph, 2-SMe-Ph, 2-S(*i*-Pr)-Ph, 2-C(Me)₂CN-Ph, 2-CF₃-Ph, 3-CF₃-Ph, 2-OCF₃-Ph, 3-OCF₃-Ph, 3-Ph-Ph, 2-Bn-Ph, 2-SiMe₃-Ph, 3-SiMe₃-Ph, 2-C(Me)₂OMe-Ph, 2-C(Me)₂OEt-Ph, 2-C(Me)₂OPr-Ph, 2-CH(Me)O(CH₂)₂OMe-Ph, 2-C(Me)₂O(CH₂)₂OMe-Ph, 2-C(Et)₂OH-Ph, 2-C(Et)₂OMe-Ph, 2-C(Et)₂OEt-Ph, 2-C(Et)₂OPr-Ph, 3-COPh-Ph, 2-CO₂Et-Ph, 3-CO₂Et-Ph, 2-NH(*i*-Bu)-Ph, 2-cyclopropyl-Ph, 2-cyclopentyl-Ph, 2,3-dimethoxy-Ph, 2,3-diCl-Ph, 2,6-diMe-Ph, 2-Me-5-F-Ph, 2-*i*-Pr-5-Me-Ph, 2-*t*-Bu-4-Me-Ph, 2-*t*-Bu-5-Me-Ph, 2-*t*-Bu-6-CN-Ph, 2-F-3-CF₃-Ph, 2-F-5-CF₃-Ph, 2-Cl-5-CF₃-Ph, 2-COMe-3-F-Ph, 2-CO₂Me-3-F-Ph, 2-CF₃-Bn, 1-naphthyl, 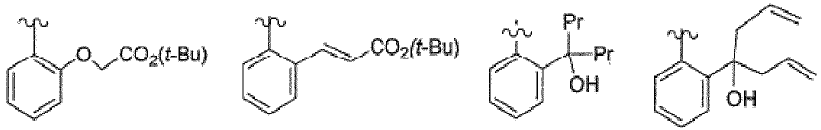 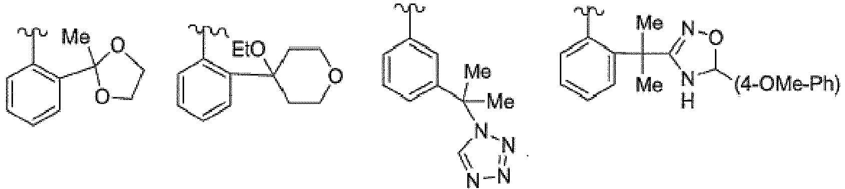 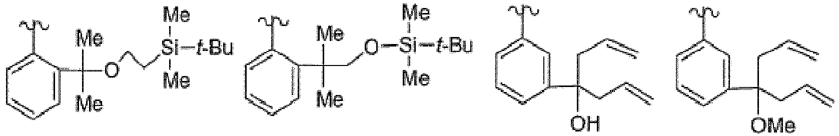 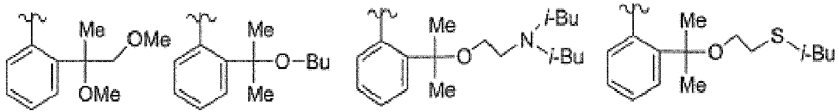 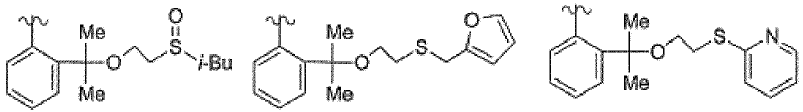 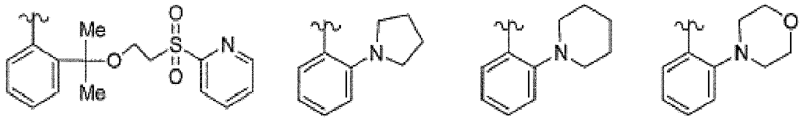 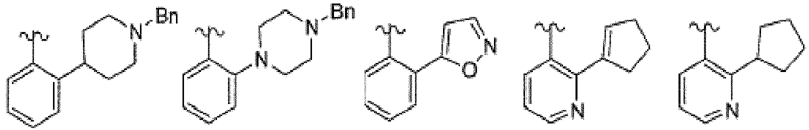 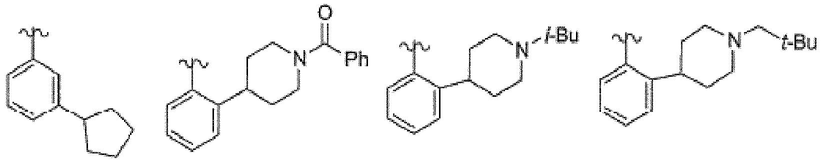 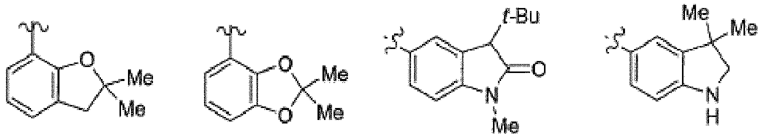 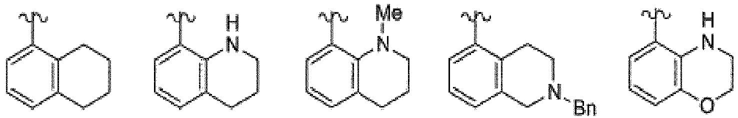 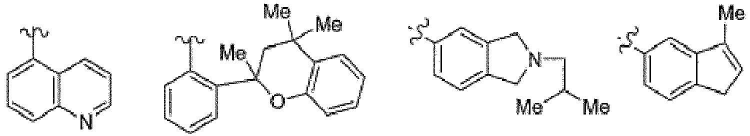 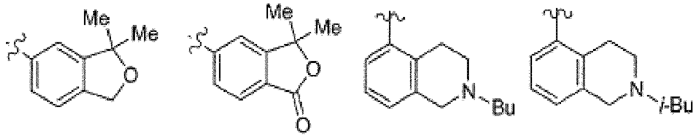 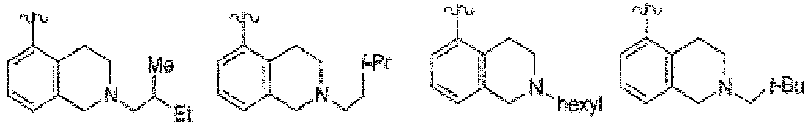 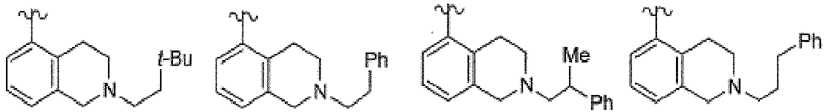 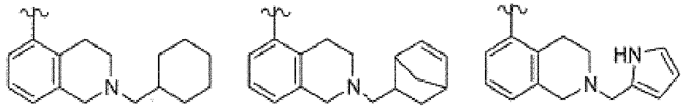 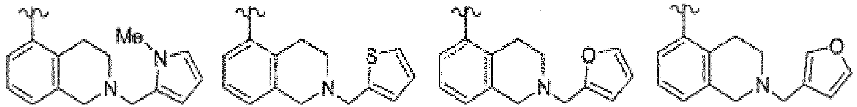 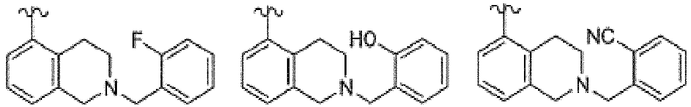 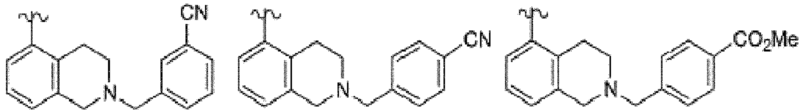 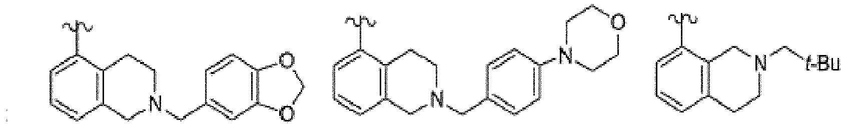 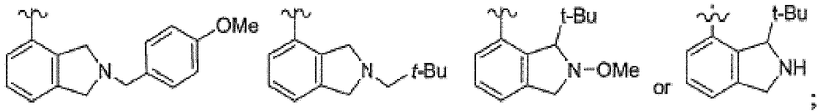
   R⁷ is H, Me, Cl, Br, CN, OH, OMe, SMe, NHMe, NMe₂, CO₂Me, imidazol-1-yl, or -CH₂NH(CO)H; and
   R⁸ is H, Me, Cl, Br, or CN.
18. A compound that (a) is of Formula (IIc): 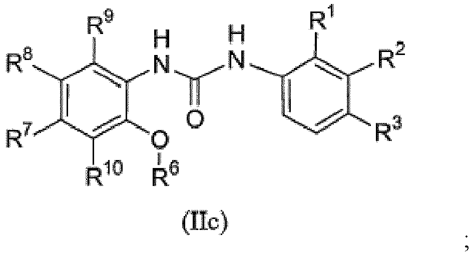 or (b) is a tautomer, N-oxide, pharmaceutically acceptable salt, or solvate thereof; wherein:
   R¹ is H or F;
   R² is H or F;
   R³ is H, Me, *t*-Bu, F, OCF₃, or O-*t*-Bu;
   R⁶ is 2-*t*-Bu-Ph, 3-CF₃-Ph, 3-I-Ph, 3-CH₂OMe-Ph, 2-Me-5-F-Ph, Bn, 4-t-Bu- Bn, 4-Br-Bn, 4-OCF₃-Bn, -CH(Me)Ph, or -CH(CO₂Me)Ph;
   R⁷ is H or CN;
   R⁸ is H, CN, CO₂Me;
   R⁹ is H; and
   R¹⁰ is H, Me, NH₂, or-CH₂OMe;
   for use in a method for treating and/or preventing a CNS disorder.
19. A compound that (a) is selected from the group:
   1-(4-*tert*-butylphenyl)-3-(2-(3-isopropylphenoxy)pyridin-3-yl)urea;
   1-(2-(2-(*tert*-butyl)phenoxy)pyridin-3-yl)-3-(4-(trifluoromethoxy)phenyl)urea;
   1-(2-(2-*tert*-butylphenoxy)pyridin-3-yl)-3-(4-cyclohexylphenyl)urea;
   1-(4-*tert*-butoxyphenyl)-3-(2-(2-*tert*-butylphenoxy)pyridin-3-yl)urea;
   1-(2,4-difluoro-phenyl)-3-[2-(2-trifluoromethyl-phenoxy)-pyridin-3-yl]-urea;
   1-(2,4-difluoro-phenyl)-3-[2-(3-ethyl-phenoxy)-pyridin-3-yl]-urea;
   1-[2-(biphenyl-3-yloxy)-pyridin-3-yl]-3-(2,4-difluoro-phenyl)-urea;
   1-(2,4-difluoro-phenyl)-3-[2-(2-isopropyl-phenoxy)-pyridin-3-yl]-urea;
   1-[2-(3-trifluoromethyl-phenoxy)-pyridin-3-yl]-3-(3-trifluoromethyl-phenyl)-urea;
   1-(3-chloro-4-methyl-phenyl)-3-[2-(3-trifluoromethyl-phenoxy)-pyridin-3-yl]-urea;
   1-(4-chloro-3-trifluoromethyl-phenyl)-3-[2-(3-trifluoromethyl-phenoxy)-pyridin-3-yl]-urea;
   1-naphthalen-2-yl-3-[2-(3-trifluoromethyl-phenoxy)-pyridin-3-yl]-urea;
   1-(3-chloro-4-fluoro-phenyl)-3-[2-(3-trifluoromethyl-phenoxy)-pyridin-3-yl]-urea;
   1-biphenyl-4-yl-3-[2-(3-trifluoromethyl-phenoxy)-pyridin-3-yl]-urea:
   1-(4-*tert*-butyl-phenyl)-3-[2-(3-trifluoromethyl-phenoxy)-pyridin-3-yl]-urea;
   1-(3,4-difluoro-phenyl)-3-[2-(3-trifluoromethyl-phenoxy)-pyridin-3-yl]-urea;
   1-(4-benzyl-phenyl)-3-[2-(3-trifluoromethyl-phenoxy)-pyridin-3-yl]-urea;
   1-biphenyl-4-yl-3-[2-(2-isopropyl-phenoxy)-pyridin-3-yl]-urea;
   1-[2-(2-isopropyl-phenoxy)-pyridin-3-yl]-3-(4-trifluoromethoxy-phenyl)-urea;
   1-(4-*tert*-butyl-phenyl)-3-[2-(2-isopropyl-phenoxy)-pyridin-3-yl]-urea;
   1-biphenyl-4-yl-3-[2-(2-*tert*-butyl-phenoxy)-pyridin-3-yl]-urea;
   1-[2-(2-*tert*-butyl-phenoxy)-pyridin-3-yl]-3-(4-*tert*-butyl-phenyl)-urea;
   1-[2-(2-*tert*-butyl-phenoxy)-pyridin-3-yl]-3-*p*-tolyl-urea;
   1-[2-(3-*tert*-butyl-phenoxy)-pyridin-3-yl]-3-(4-*tert*-butyl-phenyl)-urea;
   1-[2-(2-*tert*-butyl-phenoxy)-pyridin-3-yl]-3-(4-chloro-2-fluoro-phenyl)-urea;
   1-[2-(2-*tert*-butyl-phenoxy)-pyridin-3-yl]-3-(2-fluoro-4-methyl-phenyl)-urea;
   1-[2-(2-*tert*-butyl-phenylsulfanyl)-pyridin-3-yl]-3-*p*-tolyl-urea;
   1-[2-(2-*tert*-butyl-phenoxy)-pyridin-3-yl]-3-(4-dimethylamino-phenyl)-urea;
   1-[2-(2-propyl-phenoxy)-pyridin-3-yl]-3-(4-trifluoromethoxy-phenyl)-urea;
   1-[2-(2-*tert*-butyl-phenoxy)-pyridin-3-yl]-3-(4-trifluoromethyl-phenyl)-urea;
   1-[2-(2-*tert*-butyl-phenoxy)-pyridin-3-yl]-3-(4-methoxy-phenyl)-urea;
   1-[2-(2-*tert*-butyl-phenoxy)-pyridin-3-yl]-3-(4-phenoxy-phenyl)-urea;
   1-[2-(2-*tert*-butyl-phenoxy)-pyridin-3-yl]-3-(4-isopropoxy-phenyl)-urea;
   1-[2-(2-trifluoromethoxy-phenoxy)-pyridin-3-yl]-3-(4-trifluoromethoxy-phenyl)-urea;
   1-(4-dimethylamino-phenyl)-3-[2-(2-trifluoromethoxy-phenoxy)-pyridin-3-yl]-urea;
   1-(4-*tert*-butyl-phenyl)-3-[2-(2-trifluoromethoxy-phenoxy)-pyridin-3-yl]-urea;
   1-(4-*tert*-butoxy-phenyl)-3-[2-(2-trifluoromethoxy-phenoxy)-pyridin-3-yl]-urea;
   1-[2-(2-*tert*-butyl-phenoxy)-pyridin-3-yl]-3-isoquinolin-7-yl-urea;
   1-[2-(2-*tert*-butyl-phenoxy)-pyridin-3-yl]-3-(4-phenylamino-phenyl)-urea;
   1-[2-(2-*tert*-butyl-phenoxy)-pyridin-3-yl]-3-(9-ethyl-9*H*-carbazol-3-yl)-urea;
   1-[2-(2-*tert*-butyl-phenoxy)-pyridin-3-yl]-3-(4-diethylamino-phenyl)-urea;
   1-[2-(2-*tert*-butyl-phenoxy)-pyridin-3-yl]-3-(4-morpholin-4-yl-phenyl)-urea;
   1-(2,4-difluoro-phenyl)-3-[2-(3-trifluoromethyl-phenoxy)-pyridin-3-yl]-urea;
   methyl 2-(4-(3-(2-(2-*tert*-butylphenoxy)pyridin-3-yl)ureido)phenyl)acetate;
   methyl 2-(4-(3-(2-(2-*tert*-butylphenoxy)pyridin-3-yl)ureido)phenyl)-2-methylpropanoate;
   1-(2-(2-*tert*-butylphenoxy)pyridin-3-yl)-3-(6-(2-*tert*-butylphenoxy)pyridin-3-yl)urea;
   1-(2-(2-*tert*-butylphenoxy)pyridin-3-yl)-3-(6-(2-(trifluoromethyl)phenoxy)pyridin-3-yl)urea
   1-(2-(2-*tert*-butylphenoxy)pyridin-3-yl)-3-(6-(2-*tert*-butylphenylamino)pyridin-3-yl)urea;
   1-(2-(2-*tert*-butylphenoxy)pyridin-3-yl)-3-(6-(2-(trifluoromethyl)phenylamino)pyridin-3-yl)urea;
   1-(2-(2-*tert*-butylphenoxy)pyridin-3-yl)-3-(6-(phenylamino)pyridin-3-yl)urea;
   1-(5-*tert*-buylisoxazol-3-yl)-3-(2-(2-*tert*-butylphenoxy)pyridin-3-yl)urea;
   1-(2-(2-*tert*-buylphenoxy)pyridin-3-yl)-3-(6-(trifluoromethyl)pyridin-3-yl)urea;
   ethyl 1-(3-(3-(2-(2-*tert*-butylphenoxy)pyridin-3-yl)ureido)phenyl)-5-methyl-1H-pyrazole-3-carboxylate;
   1-(2-(2-*tert*-butylphenoxy)pyridin-3-yl)-3-(2-fluoro-(2'-N,N-dimethylaminomethylphenyl))urea;
   1-(2-(2-*tert*-butylphenoxy)pyridin-3-yl)-3-(2-fluoro-4-morpholinophenyl)urea;
   1-(4-*tert*-butoxyphenyl)-3-(2-(2-*tert*-butylphenoxy)-6-chloropyridin-3-yl)urea;
   1-(4-*tert*-butoxyphenyl)-3-(2-(2-*tert*-butylphenoxy)-6-cyanopyridin-3-yl)urea;
   1-[2-(2-*tert*-butylphenoxy)-6-methoxypyridin-3-yl]-3-(4-trifluoromethoxyphenyl)urea;
   1-(2-(2-*tert*-butylphenoxy)-6-(methylthio)pyridin-3-yl)-3-(4-*tert*-butylphenyl)urea;
   1-(2-(2-*tert*-butylphenoxy)-6-(methylamino)pyridin-3-yl)-3-(4-(trifluoromethoxy)phenyl)urea;
   1-(4-*tert*-butoxyphenyl)-3-(2-(2-*tert*-butylphenoxy)-5-methylpyridin-3-yl)urea;
   1-(2-(2-*tert*-butylphenoxy)-5-chloropyridin-3-yl)-3-(4-(trifluoromethoxy)phenyl)urea;
   1-(2-(2-*tert*-butylphenoxy)-5-cyanopyridin-3-yl)-3-(4-*tert*-butylphenyl)urea;
   1-[2-(2-*tert*-Butyl-phenoxy)-6-chloro-pyridin-3-yl]-3-(4-*tert*-butyl-phenyl)-urea;
   1-[6-bromo-2-(2-*tert*-butyl-phenoxy)-pyridin-3-yl]-3-(4-trifluoromethoxy-phenyl)-urea;
   1-[2-(2-*tert*-butyl-phenoxy)-6-methylamino-pyridin-3-yl]-3-*p*-tolyl-urea;
   1-[2-(2-*tert*-butyl-phenoxy)-6-cyano-pyridin-3-yl]-3-(4-dimethylamino-phenyl)-urea;
   1-[2-(2-*tert*-butyl-phenoxy)-6-cyano-pyridin-3-yl]-3-(4-*tert*-butyl-phenyl)-urea;
   1-[2-(2-*tert*-butyl-phenoxy)-6-cyano-pyridin-3-yl]-3-(4-trifluoromethoxy-phenyl)-urea;
   1-[2-(2-*tert*-butyl-phenoxy)-6-methylsulfanyl-pyridin-3-yl]-3-(4-dimethylaminophenyl)-urea;
   1-[2-(2-*tert*-butyl-phenoxy)-5-methyl-pyridin-3-yl]-3-(4-tert-butyl-phenyl)-urea;
   1-(4-*tert*-butoxy-phenoxy)-3-[2-(2-*tert*-butyl-phenoxy)-5-methyl-pyridin-3-yl]-urea;
   1-(4-*tert*-butoxy-phenoxy)-3-[2-(2-*tert*-butyl-phenoxy)-6-methoxy-pyridin-3-yl]-urea;
   1-[2-(2-*tert*-butyl-phenoxy)-5-chloro-pyridin-3-yl]-3-*p*-tolyl-urea;
   1-(2-(2,2-dimethyl-2,3-dihydrobenzofuran-7-yloxy)pyridin-3-yl)-3-(4-(trifluoromethoxy)phenyl)urea;
   1-(2-(2,2-dimethylbenzo[d][1,3]dioxol-4-yloxy)pyridin-3-yl)-3-(4-(trifluoromethoxy)phenyl)urea;
   1-(4-*tert*-butyl-phenyl)-3-[2-(2,2-dimethyl-2,3-dihydro-benzofuran-7-yloxy)-pyridin-3-yl]-urea;
   1-(4-dimethylamino-phenyl)-3-[2-(2,2-dimethyl-2,3-dihydro-benzofuran-7-yloxy)-pyridin-3-yl]-urea;
   1-(4-*tert*-butoxy-phenyl)-3-[2-(2,2-dimethyl-2,3-dihydro-benzofuran-7-yloxy)-pyridin-3-yl]-urea;
   1-(4-*tert*-butoxy-phenyl)-3-[2-(2,2-dimethyl-benzo[1,3]dioxol-4-yloxy)-pyridin-3-yl]-urea;
   1-(4-*tert*-butyl-phenyl)-3-[2-(2,2-dimethyl-benzo[1,3]dioxol-4-yloxy)-pyridin-3-yl]-urea;
   1-(4-*tert*-butylphenyl)-3-(2-(2-*tert*-butylphenylamino)pyridin-3-yl)urea;
   1-(4-(2-*tert*-butylphenoxy)pyridin-3-yl)-3-(4-(trifluoromethoxy)phenyl)urea;
   1-(4-(2-*tert*-butylphenoxy)pyridin-3-yl)-3-(4-*tert*-butylphenyl)urea;
   1-(4-butoxyphenyl)-3-(2-(2-*tert*-butylphenoxy)pyridin-3-yl)urea;
   1-[2-(2-*tert*-butyl-phenoxy)-pyridin-3-yl]-3-(4-cyclopentyloxy-phenyl)-urea;
   1-[2-(2-*tert*-butyl-phenoxy)-pyridin-3-yl]-3-[4-(3,3-dimethyl-butoxy)-phenyl]-urea;
   1-[2-(2-*tert*-butyl-phenoxy)-pyridin-3-yl]-3-[4-(2-cyclohexyl-ethoxy)-phenyl]-urea;
   1-[4-(2-*tert*-butoxy-1-methyl-ethoxy)-phenyl]-3-[2-(2-*tert*-butyl-phenoxy)-pyridin-3-yl]-urea;
   1-[2-(2-*tert*-butyl-phenoxy)-pyridin-3-y1]-3-[4-(3-methoxy-benzyloxy)-phenyl]-urea;
   1-[2-(2-*tert*-butyl-phenoxy)-pyridin-3-yl]-3-[4-(4-trifluoromethoxy-benzyloxy)-phenyl]-urea;
   1-[4-(1-benzyl-piperidin-4-ylmethoxy)-phenyl]-3-[2-(2-*tert*-butyl-phenoxy)-pyridin-3-yl]-urea;
   1-[2-(2-*tert*-butyl-phenoxy)-pyridin-3-yl]-3-(4-propoxy-phenyl)-urea;
   1-[2-(2-*tert*-butyl-phenoxy)-pyridin-3-yl]-3-[4-(3-methyl-butoxy)-phenyl]-urea;
   1-[2-(2-*tert*-butyl-phenoxy)-pyridin-3-yl]-3-[4-(3-methyl-cyclopentyloxy)-phenyl]-urea;
   1-[2-(2-*tert*-butyl-phenoxy)-pyridin-3-yl]-3-[4-(2,4-difluoro-benzyloxy)-phenyl]-urea;
   1-(4-{3-[2-(2-*tert*-butyl-phenoxy)-pyridin-3-yl]-ureido}-phenyl)-cyclopropanecarboxylicacidmethylester;
   1-(4-{3-[2-(2-*tert*-butyl-phenoxy)-pyridin-3-yl]-ureido}-phenyl)-cyclobutanecarboxylic acid methyl ester;
   1-(4-{3-[2-(2-*tert*-butyl-phenoxy)-pyridin-3-yl]-ureido}-phenyl)-cyclopentanecarboxylic acid methyl ester;
   1-(4-{3-[2-(2-*tert*-butyl-phenoxy)-pyridin-3-yl]-ureido}-phenyl)-cyclohexanecarboxylic acid methyl ester;
   1-[2-(2-*tert*-butyl-phenoxy)-pyridin-3-yl]-3-[4-(1-methoxymethyl-cyclopropyl)-phenyl]-urea;
   1-(4-trifluoromethoxy-phenyl)-3-[2-(2-trimethylsilanyl-phenoxy)-pyridin-3-yl]-urea;
   1-(4-trifluoromethoxy-phenyl)-3-[2-(3-trimethylsilanyl-phenoxy)-pyridin-3-yl]-urea;
   1-(4-*tert*-butyl-phenyl)-3-[2-(2-trimethylsilanyl-phenoxy)-pylidin-3-yl]-urea;
   1-[2-(2-cyclopentyl-phenoxy)-pyridin-3-yl]-3-(4-trifluoromethoxy-phenyl)-urea;
   1-[2-(2-*tert*-butyl-phenoxy)-pyridin-3-yl]-3-(4-piperidin-1-yl-phenyl)-urea;
   1-[4-(4-benzyl-piperazin-l-yl)-phenyl]-3-[2-(2-*tert*-butyl-phenoxy)-pyridin-3-yl]-urea;
   1-[4-(1-benzyl-piperidin-4-ylmethoxy)-2-fluoro-phenyl]-3-[2-(2-*tert*-butyl-phenoxy)-pyridin-3-yl]-urea;
   1-[2-(2-*tert*-butyl-phenoxy)-pyridin-3-yl]-3-[4-(3-dimethylamino-2,2-dimethyl-propoxy)-2-fluoro-phenyl]-urea;
   1-[2-(2-*tert*-butyl-phenoxy)-pyridin-3-yl]-3-[4-(3-dimethylamino-2,2-dimethyl-propoxy)-phenyl]-urea;
   1-[2-(2-*tert*-butyl-phenoxy)-pyridin-3-yl]-3-(6-methoxy-benzothiazol-2-yl)-urea;
   1-[2-(2-*tert*-butyl-phenoxy)-pyridin-3-yl]-3-(2-fluoro-4-isopropoxy-phenyl)-urea;
   1-(3-bromo-4-trifluoromethoxy-phenyl)-3-[2-(2-*tert*-butyl-phenoxy)-pyridin-3-yl]-urea;
   1-[2-(2-*tert*-butyl-phenoxy)-pyridin-3-yl]-3-(4-ethoxy-2-fluoro-phenyl)-urea;
   1-[2-(2-benzyl-phenoxy)-pyridin-3-yl]-3-(4-trifluoromethoxy-phenyl)-urea;
   1-[2-(2-*tert*-butyl-phenoxy)-pyridin-3-yl]-3-(2-fluoro-4-methoxy-phenyl)-urea;
   2-(4-{3-[2-(2-*tert*-butyl-phenoxy)-pyridin-3-yl]-ureido}-phenyl)-5-methyl-2*H-*pyrazole-3-carboxylicacidethylester;
   5-(3-(2-(2-*tert*-butylphenoxy)pyridin-3-yl)ureido)-2-(trifluoromethoxy)benzoate;
   1-(4-(1H-pyrazol-1-yl)phenyl)-3-(2-(2-*tert*-butylphenoxy)pyridin-3-yl)urea;
   ethyl1-(4-(3-(2-(2-*tert*-butylphenoxy)pyridin-3-yl)ureido)phenyl)-5-methyl-1H-pyrazole-4-carboxylate;
   1-[2-(2-*tert*-butyl-phenoxy)-pyridin-3-yl]-3-(3'-dimethylaminomethyl-3-fluorobiphenyl-4-yl)-urea;
   1-(2-(2-(piperidin-1-yl)phenoxy)pyridin-3-yl)-3-(4-(trifluoromethoxy)phenyl)urea;
   1-(2-(2-(pyrrolidin-1-yl)phenoxy)pyridin-3-yl)-3-(4-(trifluoromethoxy)phenyl)urea;
   1-[2-(2-*tert*-butyl-phenoxy)-pyridin-3-yl]-3-(4'-dimethylaminomethyl-3-fluorobiphenyl-4-yl)-urea;
   1-(2-(2-(isopropylthio)phenoxy)pyridin-3-yl)-3-(4-(trifluoromethoxy)phenyl)urea;
   1-(2-(2-iodophenoxy)pyridin-3-yl)-3-(4-(trifluoromethoxy)phenyl)urea;
   1-[2-(2-*tert*-butyl-phenoxy)-pyridin-3-yl]-3-(3-fluoro-2'-hydroxymethyl-biphenyl-4-yl)-urea;
   1-(2-(2-methoxyphenoxy)pyridin-3-yl)-3-(3-(trifluoromethoxy)phenyl)urea;
   1-(2-(3-benzoylphenoxy)pyridin-3-yl)-3-(3-(trifluoromethoxy)phenyl)urea;
   1-(2-(2-*tert*-butyl-6-cyanophenoxy)pyridin-3-yl)-3-(3-(trifluoromethoxy)phenyl)urea;
   1-(2-(2-(4-hydroxyheptan-4-yl)phenoxy)pyridin-3-yl)-3-(3-(trifluoromethoxy)-phenyl)urea;
   1-(2-(2-*tert*-butylphenoxy)pyridin-3-yl)-3-(indolin-5-yl)urea;
   1-(2-(2-*tert*-butylphenoxy)pyridin-3-yl)-3-(9-oxo-9H-fluoren-3-yl)urea;
   1-(2-(2-(1-benzylpiperidin-4-yl)phenoxy)pyridin-3-yl)-3-(4-(trifluoromethoxy)phenyl)urea;
   1-(2-(2-*tert*-butylphenoxy)pyridin-3-yl)-3-(1-oxo-2,3 -dihydro-1H-inden-5-yl)urea;
   1-(2-(2-(4-hydroxyhepta-1,6-dien-4-yl)phenoxy)pyridin-3-yl)-3-(4-(trifluoromethoxy)phenyl)urea;
   4-(3-(2-(2-*tert*-butylphenoxy)pyridin-3-yl)ureido)-3-fluorophenyl diethyl phosphate;
   1-(2-(2-(4-benzylpiperazin-1-yl)phenoxy)pyridin-3-yl)-3-(4-(trifluoromethoxy)phenyl)urea;
   1-(2-(2-*tert*-butylphenoxy)pyridin-3-yl)-3-(4-(1-(hydroxymethyl)cyclopropyl)-phenyl) urea;
   1-(2-(5-fluoro-2-methylphenoxy)pyridin-3-yl)-3-(4-(trifluoromethoxy)phenyl)urea;
   1-(4-aminophenyl)-3-(2-(2-*tert*-butylphenoxy)pyridin-3-yl)urea;
   1-(2-(2-(2-(5-(4-methoxyphenyl)-4,5-dihydro-1,2,4-oxadiazol-3-yl)propan-2-yl)phenoxy)pyridin-3-yl)-3-(4-(trifluoromethoxy)phenyl)urea;
   1-(2-(2-morpholinophenoxy)pyridin-3-yl)-3-(4-(trifluoromethoxy)phenyl)urea;
   1-(2-(2-*tert*-butylphenoxy)pyridin-3-yl)-3-(4-((dimethylamino)methyl)-2-fluorophenyl) urea;
   1-[2-(2-*tert*-butyl-phenoxy)-pyridin-3-yl]-3-[3-fluoro-2'-((S)-3-hydroxy-pyrrolidin-1-ylmethyl)-biphenyl-4-yl]-urea;
   1-(2-(2,3-dimethoxyphenoxy)pyridin-3-yl)-3-(4-(trifluoromethoxy)phenyl)urea;
   1-(2-(quinolin-5-yloxy)pyridin-3-yl)-3-(4-(trifluoromethoxy)phenyl)urea;
   1-(2-(2-*tert*-butylphenoxy)pyridin-3-yl)-3-(4-(4,4,5,5-tetramethyl-1,3,2-dioxaborolan-2-yl)phenyl)urea;
   1-[2-(2-*tert-*Butyl-phenoxy)-pyridin-3-yl]-3-(2'-dimethylaminomethyl-biphenyl-3-yl)-urea;
   1-(2-(2-tert-butylphenoxy)pyridin-3-yl)-3-(4-(1-hydroxyethyl)phenyl)urea;
   1-(2-(2-(2-ethoxypropan-2-yl)phenoxy)pyridin-3-yl)-3-(4-(trifluoromethoxy)phenyl)urea;
   1-(2-(2-(3-ethoxypentan-3-yl)phenoxy)pyridin-3-yl)-3-(4-(trifluoromethoxy)phenyl)urea;
   1-(2-(2-(2-methoxypropan-2-yl)phenoxy)pyridin-3-yl)-3-(4-(trifluoromethoxy)-phenyl)urea;
   1-(2-(2-(3-methoxypentan-3-yl)phenoxy)pyridin-3-yl)-3-(4-(trifluoromethoxy)-phenyl)urea;
   1-(2-(2-(2-propoxypropan-2-yl)phenoxy)pyridin-3-yl)-3-(4-(trifluoromethoxy)-phenyl)urea;
   1-(2-(3-(2-ethoxypropan-2-yl)phenoxy)pyridin-3-yl)-3-(4-(trifluoromethoxy)phenyl)urea;
   1-(2-(2-(2-(2-methoxyethoxy)propan-2-yl)phenoxy)pyridin-3-yl)-3-(4-(trifluoromethoxy)phenyl)urea;
   1-(2-(2-(1-(2-methoxyethoxy)ethyl)phenoxy)pyridin-3-yl)-3-(4-(trifluoromethoxy)phenyl)urea;
   1-(2-(2-(2-cyanopropan-2-yl)phenoxy)pyridin-3-yl)-3-(4-(trifluoromethoxy)phenyl)urea;
   1-(4-(trifluoromethoxy)phenyl)-3-(2-(2-vinylphenoxy)pyridin-3-yl)urea;
   1-(2-(2-(prop-1-en-2-yl)phenoxy)pyridin-3-yl)-3-(4-(trifluoromethoxy)phenyl)urea;
   1-(2-(2-cyclopropylphenoxy)pyridin-3-yl)-3-(4-(trifluoromethoxy)phenyl)urea;
   (*E*)-*tert*-butyl 3-(2-(3-(3-(4-(trifluoromethoxy)phenyl)ureido)pyridin-2-yloxy) phenyl)acrylate;
   1-(2-(2-*tert*-butylphenoxy)pyridin-3-yl)-3-(4-(methyl(phenyl)amino)phenyl)urea;
   1-(2-(2-*tert*-butylphenoxy)pyridin-3-yl)-3-(3-(cyclohexyloxy)phenyl)urea;
   1-(2-(2-*tert*-butylphenoxy)pyridin-3-yl)-3-(4-(2-(trifluoromethyl)phenylamino)-phenyl)urea;
   1-(2-(2-*tert*-butylphenoxy)pyridin-3-yl)-3-(4-(4-methylcyclohexyloxy)phenyl)urea;
   1-(2-(2-*tert*-butylphenoxy)pyridin-3-yl)-3-(4-(cyclohexylmethoxy)phenyl)urea;
   ethyl 6-(4-(3-(2-(2-*tert*-butylphenoxy)pyridin-3-yl)ureido)phenoxy)hexanoate;
   1-(2-(2-*tert*-butylphenoxy)pyridin-3-yl)-3-(3-(isopentyloxy)phenyl)urea;
   9-(4-{3-[2-(2-*tert*-butyl-phenoxy)-pyridin-3-yl]-ureido}-phenoxy)-nonanoic acid methyl ester;
   1-(2-(2-*tert*-butylphenoxy)pyridin-3-yl)-3-(4-(3-methoxy-3-methylbutoxy)phenyl)urea;
   methyl 4-((4-(3-(2-(2-*tert*-butylphenoxy)pyridin-3-yl)ureido)phenoxy)methyl)benzoate;
   1-(2-(2-*tert*-butylphenoxy)pyridin-3-yl)-3-(4-((1-(cyclohexylmethyl)piperidin-4-yl)methoxy)-2-fluorophenyl)urea;
   ethyl 4-(4-(3-(2-(2-*tert*-butylphenoxy)pyridin-3-yl)ureido)phenoxy)-cyclohexanecarboxylate;
   1-(2-(2-*tert*-butylphenoxy)pyridin-3-yl)-3-(3-(4-methylcyclohexyloxy)phenyl)urea;
   1-(2-(2-*tert*-butylphenoxy)pyridin-3-yl)-3-(3-(cyclohexylmethoxy)phenyl)urea;
   1-(2-(2-*tert*-butylphenoxy)pyiidin-3-yl)-3-(4-((2,2-dimethyl-1,3-dioxolan-4-yl)methoxy)-2-fluorophenyl)urea;
   1-(4-(1-benzylpiperidin-3-yloxy)phenyl)-3-(2-(2-*tert*-butylphenoxy)pyridin-3-yl)urea;
   1-(4-(2-(1H-pyrrol-1-yl)ethoxy)phenyl)-3-(2-(2-*tert-*butylphenoxy)pyridin-3-yl)urea;
   (S)-1-(4-(1-benzylpiperidin-3-yloxy)phenyl)-3-(2-(2-*tert*-butylphenoxy)pyridin-3-yl)urea;
   *tert-*butyl 4-((4-(3-(2-(2-*tert*-butylphenoxy)pyridin-3-yl)ureido)-3-fluorophenoxy)methyl)-piperidine-1-carboxylate;
   benzyl 4-((4-(3-(2-(2-*tert-*butylphenoxy)pyridin-3-yl)ureido)phenoxy)methyl)piperidine-1-carboxylate;
   1-(2-(2-*tert*-butylphenoxy)pyridin-3-yl)-3-(4-(1-(cyclohexylmethyl)piperidin-4-yloxy)phenyl)urea;
   1-(2-(2-*tert*-butylphenoxy)pyridin-3-yl)-3-(4-(4-methoxyphenylamino)phenyl)urea;
   1-(4-(1-benzylpyrrolidin-3-yloxy)phenyl)-3-(2-(2-*tert*-butylphenoxy)pyridin-3-yl)urea;
   methyl 4-((3-(3-(2-(2-*tert*-butylphenoxy)pyridin-3-yl)ureido)phenoxy)methyl)benzoate;
   (S)-1-(4-(1-benzylpyrrolidin-3-yloxy)phenyl)-3-(2-(2-*tert*-butylphenoxy)pyridin-3-yl)urea;
   1-(4-((4-benzylmorpholin-2-yl)methoxy)phenyl)-3-(2-(2-*tert-*butylphenoxy)pyridin-3 -yl)urea;
   9-(3-{3-[2-(2-*tert*-butyl-phenoxy)-pyridin-3-yl]-ureido}-phenoxy)-nonanoic acid methyl ester;
   1-(2-(2-*tert*-butylphenoxy)pyridin-3-yl)-3-(3-(3-methoxy-3-methylbutoxy)phenyl)urea;
   1-(4-(1-benzylpiperidin-4-yloxy)phenyl)-3-(2-(2-*tert*-butylphenoxy)pyridin-3-yl)urea;
   1-(2-(2-*tert*-butylphenoxy)pyridin-3-yl)-3-(3-((1-phenyl-1H-1,2,3-triazol-4-yl )methoxy)phenyl)urea;
   1-(4-((1-benzylpyrrolidin-3-yl)methoxy)phenyl)-3-(2-(2-*tert*-butylphenoxy)pyridin-3-yl)urea;
   (R)-1-(3-(1-benzylpiperidin-3-yloxy)phenyl)-3-(2-(2-*tert*-butylphenoxy)pyridin-3-yl)urea;
   1-(3-(2-(1H-pyrrol-1-yl)ethoxy)phenyl)-3-(2-(2-*tert*-butylphenoxy)pyridin-3-yl)urea;
   1-(2-(2-*tert-*butylphenoxy)pyridin-3-yl)-3-(4-((1-phenethylpiperidin-4-yl)methoxy)phenyl)urea;
   1-[3-(1-benzyl-piperidin-3-yloxy)-phenyl]-3-[2-(2*-tert*-butyl-phenoxy)-pyridin-3-yl]-urea;
   1-[2-(2-*tert-*butyl-phenoxy)-pyridin-3-yl]-3-[4-(1-phenyl-1*H*-[1,2,3]triazol-4-ylmethoxy)-phenyl]-urea;
   1-[3-(1-benzyl-piperidin-4-yloxy)-phenyl]-3-[2-(2-*tert*-butyl-phenoxy)-pyridin-3-yl]-urea;
   1-[3-(4-benzyl-morpholin-2-ylmethoxy)-phenyl]-3-[2-(2-*tert*-butyl-phenoxy)-pyridin-3-yl]-urea;
   1-[3-(1-benzyl-pyrrolidin-3-yloxy)-phenyl]-3-[2-(2-*tert*-butyl-phenoxy)-pyridin-3-yl]-urea;
   1-[3-((*S*)-1-benzyl-pyrrolidin-3-yloxy)-phenyl]-3-[2-(2-*tert*-butyl-phenoxy)-pyridin-3-yl]-urea;
   1-[2-(2-*tert-*butyl-phenoxy)-pyridin-3-yl]-3-[4-(1-pyridin-4-ylmethyl-piperidin-4-ylmethoxy)-phenyl]-urea;
   1-[2-(2-*tert*-butyl-phenoxy)-pyridin-3-yl]-3-{2-fluoro-4-[1-(3-methyl-butyl)-piperidin-4-ylmethoxy]-phenyl}-urea;
   acetic acid 2-(3-{3-[2-(2-*tert*-butyl-phenoxy)-pyridin-3-yl]-ureido}-phenoxy)-ethyl ester;
   1-[2-(2-*tert*-butyl-phenoxy)-pyridin-3-yl]-3-[4-(1-pyridin-2-ylmethyl-piperidin-4-ylmethoxy)-phenyl]-urea;
   1-[3-(1-benzyl-pyrrolidin-3-ylmethoxy)-phenyl]-3-[2-(2-*tert*-butyl-phenoxy)-pyridin-3-yl]-urea;
   1-[3-(2-butyl-1H-imidazol-4-ylmethoxy)-phenyl]-3-[2-(2-*tert*-butyl-phenoxy)-pyridin-3-yl]-urea;
   1-[2-(2-*tert*-butyl-phenoxy)-pyridin-3-yl]-3-(4-{1-[methyl-(3-trifluoromethylbenzyl)-amino]-ethyl}-phenyl)-urea;
   1-[2-(2-*tert*-butyl-phenoxy)-pyridin-3-yl]-3-{4-[1-(methyl-thiophen-2-ylmethylamino)-ethyl]-phenyl)-urea;
   1-[2-(2-*tert*-butyl-phenoxy)-pyridin-3-yl]-3-[2-fluoro-4-(1-isobutyl-piperidin-4-ylmethoxy)-phenyl]-urea;
   1-[2-(2-*tert*-butyl-phenoxy)-pyridin-3-yl]-3-[4-(1-isobutyl-piperidin-4-ylmethoxy)-phenyl]-urea;
   1-[2-(2-*tert*-butyl-phenoxy)-pyridin-3-yl]-3-(4,6-difluoro-benzothiazol-2-yl)-urea;
   1-[2-(2-*tert*-butyl-phenoxy)-pyridin-3-yl]-3-(6-isopropyl-benzothiazol-2-yl)-urea;
   1-[2-(2-*tert*-butyl-phenoxy)-pyridin-3-yl]-3-(6-methyl-benzothiazol-2-yl)-urea;
   2-{3-[2-(2-*tert*-butyl-phenoxy)-pyridin-3-yl]-ureido}-benzothiazole-6-carboxylic acid ethyl ester;
   1-[2-(2-*tert*-butyl-phenoxy)-pyridin-3-yl]-3-(4-chloro-benzothiazol-2-yl)-urea;
   1-[2-(2-*tert*-butyl-phenoxy)-pyridin-3-yl]-3-(6-methoxy-4-methyl-benzothiazol-2-yl)-urea;
   1-[2-(2-*tert*-butyl-phenoxy)-pyridin-3-yl]-3-[6-(2-dimethylaminomethyl-phenyl)-4-fluoro-benzothiazol-2-yl]-urea;
   1-[2-(2-*tert*-butyl-phenoxy)-pyridin-3-yl]-3-(2-methyl-5-phenyl-furan-3-yl)-urea;
   1-[5-cyano-2-(2,2-dimethyl-2,3-dihydro-benzofuran-7-yloxy)-pyridin-3-yl]-3-(4-trifluoromethoxy-phenyl)-urea;
   1-[2-(5,6,7,8-tetrahydro-naphthalen-1-yloxy)-pyridin-3-yl]-3-(4-trifluoromethoxyphenyl)-urea;
   1-[2-(1,2,3,4-tetrahydro-quinolin-8-yloxy)-pyridin-3-yl]-3-(4-trifluoromethoxyphenyl)-urea;
   1-[2-(1-methyl-1,2,3,4-tetrahydro-quinolin-8-yloxy)-pyridin-3-yl]-3-(4-trifluoromethoxy-phenyl)-urea;
   1-[2-(3,3-dimethyl-2,3-dihydro-1*H*-indol-4-yloxy)-pyridin-3-yl]-3-(4-trifluoromethoxy-phenyl)-urea;
   1-[2-(3-*tert*-butyl-1-methyl-2-oxo-2,3-dihydro-1*H*-indol-4-yloxy)-pyridin-3-yl]-3-(4-trifluoromethoxy-phenyl)-urea;
   1-[2-(2-benzyl-1,2,3,4-tetrahydro-isoquinolin-5-yloxy)-pyridin-3-yl]-3-(4-trifluoromethoxy-phenyl)-urea;
   1-[2-(1,2,3,4-tetrahydro-quinolin-5-yloxy)-pyridin-3-yl]-3-(4-trifluoromethoxyphenyl)-urea;
   1-[2-(3,4-dihydro-2*H*-benzo[1,4]oxazin-5-yloxy)-pyridin-3-yl]-3-(4-trifluoromethoxy-phenyl)-urea;
   1-[3-(2-*tert*-butyl-phenoxy)-pyridazin-4-yl]-3-(4-*tert*-butyl-phenyl)-urea;
   1-[2-(2-*tert*-butyl-phenoxy)-pyridin-3-yl]-3-(2,4-dichloro-phenyl)-thiourea;
   1-[2-(2-*tert*-butyl-phenoxy)-pyridin-3-yl]-3-(2-methyl-4-trifluoromethoxy-phenyl)-thiourea;
   1-[2-(2-*tert*-butyl-phenoxy)-pyridin-3-yl]-3-(3-*tert*-butyl-phenyl)-thiourea;
   1-[2-(2-*tert*-butyl-phenoxy)-pyridin-3-yl]-3-*p*-tolyl-thiourea;
   1-[2-(2-*tert*-butyl-phenoxy)-pyridin-3-yl]-3-(4-methoxy-phenyl)-thiourea;
   1-[2-(2-*tert*-butyl-phenoxy)-pyridin-3-yl]-3-(2,4-dimethyl-phenyl)-thiourea;
   1-[2-(2-*tert-*butyl-phenoxy)-pyridin-3-yl]-3-[2-(4-chloro-phenyl)-ethyl]-urea;
   1-[2-(4-bromo-phenyl)-ethyl]-3-[2-(2-*tert*-butyl-phenoxy)-pyridin-3-yl]-urea;
   1-[2-(2-*tert*-butyl-phenoxy)-pyridin-3-yl]-3-[2-(1-methyl-1*H*-indol-3-yl)-ethyl]-urea;
   1-[2-(3,4-bis-benzyloxy-phenyl)-ethyl]-3-[2-(2-*tert*-butyl-phenoxy)-pyridin-3-yl]-urea;
   1-[2-(2-*tert*-butyl-phenoxy)-pyridin-3-yl]-3-[2-(3-fluoro-phenyl)-ethyl]-urea;
   1-(2-benzo[1,3]dioxol-5-yl-ethyl)-3-[2-(2-*tert*-butyl-phenoxy)-pyridin-3-yl]-urea;
   1-[2-(2-*tert*-butyl-phenoxy)-pyridin-3-yl]-3-[2-(4-methoxy-phenyl)-ethyl]-urea;
   1-[2-(2-*tert*-butyl-phenoxy)-pyridin-3-yl]-3-[2-(2,5-dimethoxy-phenyl)-ethyl]-urea;
   1-[2-(2-*tert*-butyl-phenoxy)-pyridin-3-yl]-3-((S)-2-phenyl-propyl)-urea;
   1-[2-(4-bromo-phenyl)-2-oxo-ethyl]-3-[2-(2-*tert*-butyl-phenoxy)-pyridin-3-yl]-urea;
   1-[2-(2-*tert*-butyl-phenoxy)-pyridin-3-yl]-3-[2-(4-methoxy-phenyl)-2-oxo-ethyl]-urea;
   1-[2-(2-*tert*-butyl-phenoxy)-pyridin-3-yl]-3-(3-chloro-4-methoxy-benzyl)-urea;
   1-[2-(2-*tert*-butyl-phenoxy)-pyridin-3-yl]-3-(1-phenyl-cyclohexylmethyl)-urea;
   1-(4-*tert*-butyl-phenyl)-3-[2-(2-trifluoromethyl-benzyloxy)-pyridin-3-yl]-urea;
   1-[2-(2-*tert*-butyl-phenoxy)-3-methyl-phenyl]-3-(4-*tert*-butyl-phenyl)-urea;
   1-[2-(3-iodo-phenoxy)-phenyl]-3-(4-trifluoromethoxy-phenyl)-urea;
   1-[2-(5-fluoro-2-methyl-phenoxy)-phenyl]-3-(4-trifluoromethoxy-phenyl)-urea;
   1-[3-amino-2-(2-*tert*-butyl-phenoxy)-phenyl]-3-(4-*tert*-butyl-phenyl)-urea;
   1-[2-(1-phenyl-ethoxy)-phenyl]-3-(4-trifluoromethoxy-phenyl)-urea;
   1-(2-benzyloxy-pyridin-3-yl)-3-(4-*tert*-butyl-phenyl)-urea;
   1-(2-benzyl oxy-phenyl)-3-(4-trifluoromethoxy-phenyl)-urea;
   1-[2-(3-methoxymethyl-phenoxy)-phenyl]-3-(4-trifluoromethoxy-phenyl)-urea;
   phenyl-{2-[3-(4-trifluoromethoxy-phenyl)-ureido]-phenoxy}-acetic acid ethyl ester;
   1-[2-(4-*tert*-butyl-benzyloxy)-phenyl]-3-(4-trifluoromethoxy-phenyl)-urea;
   1-[2-(4-bromo-benzyloxy)-phenyl]-3-(4-trifluoromethoxy-phenyl)-urea;
   1-[2-(4-trifluoromethoxy-benzyloxy)-phenyl]-3-(4-trifluoromethoxy-phenyl)-urea;
   1-{2-[(*E*)-2-(4-fluoro-phenyl)-vinyl]-phenyl}-3-(4-trifluoromethoxy-phenyl)-urea;
   1-[2-((*E*)-styryl)-phenyl]-3-(4-trifluoromethoxy-phenyl)-urea;
   1-{2-[(*E*)-2-(4-chloro-phenyl)-vinyl]-phenyl}-3-(4-trifluoromethoxy-phenyl)-urea;
   *N*-(3-*tert*-butyl-phenyl)-2-[3-(4-trifluoromethoxy-phenyl)-ureido]-benzamide;
   1-(2-(2-isopropyl-5-methylphenoxy)pyridin-3-yl)-3-p-tolylurea;
   1-(4-bromophenyl)-3-(2-(3-ethylphenoxy)pyridin-3-yl)urea;
   1-(2-(2-isopropyl-5-methylphenoxy)pyridin-3-yl)-3-(4-(trifluoromethoxy)phenyl)urea;
   1-[2-(biphenyl-3-yloxy)-pyridin-3-yl]-3-(4-trifluoromethoxy-phenyl)-urea;
   1-(2-(2-isopropyl-5-methyl phenoxy)-6-methoxypyridin-3 -yl)-3 -(4-(trifluoromethoxy)phenyl)urea;
   1-(2,4-difluorophenyl)-3-(2-(2-ethylphenoxy)pyridin-3-yl)urea;
   1-[2-(biphenyl-3-yloxy)-pyridin-3-yl]-3-(4-bromo-phenyl)-urea;
   1-biphenyl-4-yl-3-[2-(3-*tert*-butyl-phenoxy)-pyridin-3-yl]-urea;
   1-(3,4-dichlorophenyl)-3-(2-(3-ethylphenoxy)pyridin-3-yl)urea;
   1-(4-bromophenyl)-3-(2-(2-ethylphenoxy)pyridin-3-yl)urea;
   1-(3,4-dimethylphenyl)-3-(2-(3-ethylphenoxy)pyridin-3-yl)urea;
   1-(3,4-dichlorophenyl)-3-(2-(2-isopropylphenoxy)pyridin-3-yl)urea;
   1-[2-(biphenyl-3-yloxy)-pyridin-3-yl]-3-(4-*tert*-butyl-phenyl)-urea;
   1-[2-(biphenyl-2-yloxy)-pyridin-3-yl]-3-(2,4-difluoro-phenyl)-urea;
   1-(2-(2-sec-butylphenoxy)-6-methoxypyridin-3-yl)-3-(4-(trifluoromethoxy)phenyl)urea;
   1-(2-(2-*tert*-butyl-5-methylphenoxy)-6-methoxypyridin-3-yl)-3-p-tolylurea;
   1-(2-(2-isopropyl-5-methylphenoxy)-6-methoxypyridin-3-yl)-3-p-tolylurea;
   1-(2,4-difluorophenyl)-3-(2-(3-(trifluoromethoxy)phenoxy)pyridin-3-yl)urea;
   1-(2,4-difluorophenyl)-3-(2-(3-(trifluoromethyl)phenoxy)phenyl)urea;
   1-(2,4-difluorophenyl)-3-(2-(2-fluoro-3-(trifluoromethyl)phenoxy)pyridin-3-yl)urea;
   1-(2,4-difluorophenyl)-3-(2-(naphthalen-1-yloxy)pyridin-3-yl)urea;
   1-(2-(3-bromophenoxy)pyridin-3-yl)-3-(2,4-difluorophenyl)urea;
   1-(2-(2-chloro-5-(trifluoromethyl)phenoxy)pyridin-3-yl)-3-(2,4-difluorophenyl)urea;
   1-(2-(2-*tert*-butylphenoxy)pyridin-3-yl)-3-(5,6-dihydro-4H-cyclopenta[d]thiazol-2-yl)urea;
   ethyl 2-(3-(2-(2-*tert*-butylphenoxy)pyridin-3-yl)ureido)-4-methylthiazole-5-carboxylate;
   1-(2-(2-*tert*-butylphenoxy)pyridin-3-yl)-3-(4,5-dimethylthiazol-2-yl)urea;
   1-(2-fluoro-4-methylphenyl)-3-(2-(3-(trifluoromethyl)phenoxy)pyridin-3-yl)urea;
   1-(4-chloro-2-fluorophenyl)-3-(2-(3-(trifluoromethyl)phenoxy)pyridin-3-yl)urea;
   1-(2-(2-*tert*-butylphenoxy)pyridin-3-yl)-3-(2,3-dihydrobenzo[b][1,4]dioxin-6-yl)urea;
   1-(2-(2-*tert-*butylphenoxy)pyridin-3-yl)-3-(6-chloropyridin-3-yl)urea;
   1-(2-(2-*tert*-butylphenylamino)pyridin-3-yl)-3-(4-phenoxyphenyl)urea;
   1-(2-(2-*tert*-butylphenylamino)pyridin-3-yl)-3-p-tolylurea;
   1-(2-(2-*tert*-butylphenoxy)pyridin-3-yl)-3-(5-methylisoxazol-3-yl)urea;
   1-(2-(2-*tert*-butylphenoxy)pyridin-3-yl)-3-((2S)-2-phenylcyclopropyl)urea;
   1-(1H-benzo[d]imidazol-2-yl)-3-(2-(2-*tert*-butylphenoxy)pyridin-3-yl)urea;
   1-(2-(2-*tert*-butylphenylamino)pyiidin-3-yl)-3-m-totylurea;
   1-(2-(3-*tert-*butylphenoxy)pyridin-3-yl)-3-(3-chloro-4-methylphenyl)urea;
   1-(2-(2-*tert*-butylphenoxy)pyridin-3-yl)-3-(3,4-dimethylphenyl)urea;
   1-(4-bromophenyl)-3-(2-(2-*tert*-butylphenoxy)pyridin-3-yl)urea;
   1-(2-(2-*ter*t-butylphenoxy)pyridin-3-yl)-3-(4-*tert*-butylphenyl)urea;
   1-(2-(2-*tert*-butylphenoxy)pyridin-3-yl)-3-(3-chloro-4-methylphenyl)urea;
   1-(4-bromophenyl)-3-(2-(2-isopropylphenoxy)pyridin-3-yl)urea;
   1-(2-(3-isopropylphenoxy)pyridin-3-yl)-3-(4-(trifluoromethoxy)phenyl)urea;
   1 -(2-(2-isopropylphenoxy)pyridin-3-yl)-3-p-tolylurea;
   1-(2-(2-*tert*-butylphenoxy)pyridin-3-yl)-3-(3,4-dichlorophenyl)urea;
   1-(2-(2-propylphenoxy)pyridin-3-yl)-3-p-tolylurea;
   1-(3,4-dimethylphenyl)-3-(2-(2-isopropylphenoxy)pyridin-3-yl)urea;
   1-(2-(2-*tert*-butylphenoxy)pyridin-3-yl)-3-(2,4-difluorophenyl)urea;
   1-(3-chloro-4-methylphenyl)-3-(2-(2-isopropylphenoxy)pyridin-3-yl)urea;
   1-(2-(2-sec-butylphenoxy)pyridin-3-yl)-3-p-tolylurea;
   1-(2-(2-ethylphenoxy)pyridin-3-yl)-3-(4-(trifluoromethoxy)phenyl)urea;
   1-biphenyl-4-yl-3-[2-(3-isopropyl-phenoxy)-pyridin-3-yl]-urea;
   1-(2-(3-*tert-*butylphenoxy)pyridin-3-yl)-3-p-tolylurea;
   1-(2-(3-*tert*-butylphenoxy)pyridin-3-yl)-3-(3,4-dichiorophenyl)urea;
   1-(4-bromophenyl)-3-(2-(3-*tert*-butylphenoxy)pyridin-3-yl)urea;
   1-(3,4-dimethylphenyl)-3-(2-(2-ethylphenoxy)pyridin-3-yl)urea;
   1-(2-(3-*tert*-butylphenoxy)pyridin-3-yl)-3-(2,4-difluorophenyl)urea;
   1-(2-(2-*tert*-butylphenoxy)-6-methoxypyridin-3-yl)-3-(4-(trifluoromethoxy)phenyl)urea;
   1-(4-*tert*-butylphenyl)-3-(2-(3-ethylphenoxy)pyridin-3-yl)urea;
   1-biphenyl-4-yl-3-[2-(3-ethyl-phenoxy)-pyridin-3-yl]-urea;
   1-(3,4-dichlorophenyl)-3-(2-(3-isopropylphenoxy)pyridin-3-yl)urea;
   1-[2-(biphenyl-3-yloxy)-pyridin-3-yl]-3-p-tolyl-urea;
   1-(2-(3-*tert-*butylphenoxy)pyridin-3-yl)-3-(3,4-dimethylphenyl)urea;
   1-(2-(2-sec-butylphenoxy)pyridin-3-yl)-3-(4-(trifluoromethoxy)phenyl)urea;
   1-(2-(3-ethylphenoxy)pyridin-3-yl)-3-p-tolylurea;
   1-(2,4-difluorophenyl)-3-(2-(3-isopropylphenoxy)pyridin-3-yl)urea;
   1-biphenyl-4-yl-3-[2-(2-ethyl-phenoxy)-pyridin-3-yl]-urea;
   1-(2-(3-ethylphenoxy)pyridin-3-yl)-3-(4-(trifluoromethoxy)phenyl)urea;
   1-(3,4-dimethylphenyl)-3-(2-(3-isopropylphenoxy)pyridin-3-yl)urea;
   1-(4-t*ert*-butylphenyl)-3-(2-(2-ethylphenoxy)pyridin-3-yl)urea;
   1-(3-chloro-4-methylphenyl)-3-(2-(2-ethylphenoxy)pyridin-3-yl)urea;
   1-(2-(2-*tert*-butyl-5-methylphenoxy)-6-methoxypyridin-3-yl)-3-(4-(trifluoromethoxy)phenyl)urea;
   1-(6-methoxy-2-(2-propylphenoxy)pyridin-3-yl)-3-(4-(trifluoromethoxy)phenyl)urea;
   1-(2,4-difluorophenyl)-3-(2-(2-(trifluoromethoxy)phenoxy)pyridin-3-yl)urea;
   1-biphenyl-4-yl-3-[2-(biphenyl-3-yloxy)-pyridin-3-yl]-urea;
   1-biphenyl-4-yl-3-[2-(2-trifluoromethoxy-phenoxy)-pyridin-3-yl]-urea;
   1-(3-chloro-4-methylphenyl)-3-(2-(3-ethylphenoxy)pyridin-3-yl)urea;
   1-(3,4-dimethylphenyl)-3-(2-(2-(trifluoromethoxy)phenoxy)pyridin-3-yl)urea;
   1-(2-(2-ethylphenoxy)pyridin-3-yl)-3-p-tolylurea;
   1-(2-(2-*tert*-butylphenylamino)pyridin-3-yl)-3-(4-(trifluoromethoxy)phenyl)urea;
   1-(2-(2-*tert*-butylphenoxy)pyridin-3-yl)-3-(5-methylthiazol-2-yl)urea;
   1-cyclohexyl-3-(2-(3-(trifluoromethyl)phenoxy)pyridin-3-yl)urea;
   1-(2,4-difluorophenyl)-3-(2-(7-methyl-2,3-dihydro-1H-inden-4-yloxy)pyridin-3-yl)urea;
   1-(2-(2-*tert*-butylphenoxy)-6-methoxypyridin-3-yl)-3-(4-*tert*-butylphenyl)urea;
   1-(2-(2-*tert*-butylphenoxy)-6-methoxypyridin-3-yl)-3-p-tolylurea;
   1-(4-methyl-2-(3 -(trifluoromethyl)phenoxy)pyridin-3 -yl)-3 -p-tolylurea;
   1-biphenyl-4-yl-3-[2-(2-*tert*-butyl-phenoxy)-6-methoxy-pyridin-3-yl]-urea;
   1-biphenyl-4-yl-3-[2-(2-*tert*-butyl-phenoxy)-4-methyl-pyridin-3-yl]-urea;
   1-[2-(2-*tert*-butyl-phenoxy)-1-oxy-pyridin-3-yl]-3-(4-*tert*-butyl-phenyl)-urea;
   1-(6-(2-*tert*-butylphenoxy)-2-chloropyridin-3-yl)-3-(4-*tert*-butylphenyl)urea;
   1-(2-(2-*tert*-butylphenoxy)-6-hydroxypyridin-3-yl)-3-(4-*tert*-butylphenyl)urea;
   1-(2-(2-*tert*-butylphenoxy)-6-hydroxypyridin-3-yl)-3-(4-(trifluoromethoxy)phenyl)urea;
   1-(6-bromo-2-(2-*tert-*butylphenoxy)pyridin-3-yl)-3-p-tolylurea;
   1-(6-bromo-2-(2-*tert*-butylphenoxy)pyridin-3-yl)-3-(4-*tert*-butylphenyl)urea;
   1-(2-(2-*tert*-butylphenoxy)-6-chloropyridin-3-yl)-3-p-tolylurea;
   1-(2-(2-*tert*-butylphenoxy)-6-chloropyridin-3-yl)-3-(4-(trifluoromethoxy)phenyl)urea;
   1-(2-(2-*tert*-butylphenoxy)-6-chloropyridin-3-yl)-3-(4-(trifluoromethyl)phenyl)urea;
   1-(6-bromo-2-(2-*tert*-butylphenoxy)pyridin-3-yl)-3-(4-(trifluoromethyl)phenyl)urea;
   1-(2-(2-*tert*-butylphenoxy)-6-(dimethylamino)pyridin-3-yl)-3-p-tolylurea;
   1-(2-(2-*tert*-butylphenoxy)-6-(dimethylamino)pyridin-3-yl)-3-(4-(trifluoromethoxy)phenyl)urea;
   1-(4-bromo-2-fluorophenyl)-3-(2-(2-*tert*-butylphenoxy)-6-chloropyridin-3-yl)urea;
   1-(4-bromo-2-fluorophenyl)-3-(2-(2-*tert*-butylphenoxy)-6-oxo-1,6-dihydropyridin-3-yl)urea;
   1-(4-bromo-2-fluorophenyl)-3-(3-(2-*tert*-butylphenoxy)pyridin-4-yl)urea;
   1-(4-bromo-2-fluorophenyl)-3-(2-(2-(trifluoromethyl)phenoxy)pyridin-3-yl) urea;
   1-(4-bromo-2-fluorophenyl)-3-(2-(2-*tert*-butylphenoxy)-6-methoxypyridin-3-yl) urea;
   1-(4-bromo-2-fluorophenyl)-3-(2-(2-*tert-*butylphenoxy)pyridin-3-yl)urea;
   1-(3-(2-*tert*-butylphenoxy)pyridin-4-yl)-3-(4-(dimethylamino)phenyl)urea;
   1-(2-(2-*tert*-butylphenoxy)-6-cyanopyridin-3-yl)-3-(4-chlorophenyl)urea;
   1-(3-(2-*tert*-butylphenoxy)pyridin-4-yl)-3-(4-chlorophenyl)urea;
   1-(3-(2-*tert*-butylphenoxy)pyridin-4-yl)-3-(4-*tert*-butylphenyl)urea;
   1-(2-(2-*tert*-butylphenoxy)-6-(1H-imidazol-1-yl)pyridin-3-yl)-3-(4-(trifluoromethoxy)phenyl)urea;
   1-(2-(2-*tert*-butylphenoxy)-6-(1H-imidazol-1-yl)pyridin-3-yl)-3-(4-*tert-*butylphenyl)urea;
   1-(2-(2-*tert*-butylphenoxy)-6-(1H-imidazol-1-yl)pyridin-3-yl)-3-(4-chlorophenyl)urea;
   methyl 6-(2-*tert*-butylphenoxy)-5-(3-(4-*tert*-butylphenyl)ureido)picolinate
   methyl 6-(2-*tert*-butylphenoxy)-5-(3-(4-(trifluoromethoxy)phenyl)ureido)picolinate;
   methyl 6-(2-*tert*-butylphenoxy)-5-(3-(4-chlorophenyl)ureido)picolinate;
   methyl 2-(4-(3-(2-(2-*tert*-butylphenoxy)pyridin-3-yl)ureido)phenoxy)-2-methylpropanoate;
   1-(2-(2-*tert-*butylphenoxy)pyridin-3-yl)-3-(4-(1,1,1,3,3,3-hexafluoro-2-hydroxypropan-2-yl)phenyl)urea;
   1-(2-(2-*tert*-butylphenoxy)pyridin-3-yl)-3-(4-(1-hydroxy-2-methylpropan-2-yl)phenyl)urea;
   1-(2-(2-*tert*-butylphenoxy)pyridin-3-yl)-3-(2-(4-chlorophenyl)propyl)urea;
   1-(2-(2-*tert-*butylphenoxy)pyridin-3-yl)-3-(2-phenylpropyl)urea;
   1-(2-(2,2-dimethyl-2,3-dihydrobenzofuran-7-yloxy)-5-methylpyridin-3-yl)-3-(4-(trifluoromethoxy)phenyl)urea;
   1-(4-*tert*-butoxyphenyl)-3-(2-(2,2-dimethyl-2,3-dihydrobenzofuran-7-yloxy)-5-methylpyridin-3 -yl)urea;
   1-(4-*tert*-butoxyphenyl)-3-(2-(2-*tert*-butylphenoxy)thiophen-3-yl)urea;
   1-(4-*tert-*butoxyphenyl)-3-(2-(2-(trifluoromethyl)phenoxy)pyridin-3-yl)urea;
   1-(4-*tert*-butoxyphenyl)-3-(3-(2-*tert-*butylphenoxy)pyridin-4-yl)urea;
   1-(4-*tert*-butoxyphenyl)-3-(4-(2-*tert*-butylphenoxy)pyridin-3-yl)urea;
   1-(4-*tert*-butylphenyl)-3-(2-(2,2-dimethyl-2,3-dihydrobenzofuran-7-yloxy)-5-methylpyridin-3 -yl)urea;
   1-(4-*tert-b*utoxyphenyl)-3-(2-(2-*tert*-butylphenoxy)-5-(trifluoromethyl)pyridin-3-yl)urea;
   1-(6-bromo-2-(2,2-dimethyl-2,3-dihydrobenzofuran-7-yloxy)pyridin-3-yl)-3-(4-*tert*-butoxyphenyl)urea;
   1-(6-bromo-2-(2,2-dimethyl-2,3-dihydrobenzofuran-7-yloxy)pyridin-3-yl)-3-p-tolylurea;
   1-(6-bromo-2-(2,2-dimethyl-2,3-dihydrobenzofuran-7-yloxy)pyridin-3-yl)-3-(4-*tert*-butylphenyl)urea;
   1-(6-bromo-2-(2,2-dimethyl-2,3-dihydrobenzofuran-7-yloxy)pyridin-3-yl)-3-(4-(trifluoromethoxy)phenyl)urea;
   1-(6-bromo-2-(2-(trifluoromethoxy)phenoxy)pyridin-3-yl)-3-(4-*tert-*butoxyphenyl)urea;
   1-(6-bromo-2-(2-(trifluoromethoxy)phenoxy)pyridin-3-yl)-3-p-tolylurea;
   1-(6-bromo-2-(2-(trifluoromethoxy)phenoxy)pyridin-3-yl)-3-(4-(trifluoromethoxy)phenyl)urea;
   1-(4-*tert-*butoxyphenyl)-3-(5-chloro-2-(2,2-dimethyl-2,3-dihydrobenzofuran-7-yloxy)pyridin-3-yl)urea;
   1-(4-*tert*-butylphenyl)-3-(5-chloro-2-(2,2-dimethyl-2,3-dihydrobenzofuran-7-yloxy)pyridin-3-yl)urea;
   1-(5-chloro-2-(2,2-dimethyl-2,3-dihydrobenzofuran-7-yloxy)pyridin-3-yl)-3-(4-(trifluoromethoxy)phenyl)urea;
   1-(4-*tert*-butoxyphenyl)-3-(2-(2-*tert-*butylphenoxy)-5-chloropyridin-3-yl)urea;
   1-(2-(2-*tert*-butylphenoxy)-5-chloropyridin-3-yl)-3-(4-*tert*-butylphenyl)urea;
   1-(5-bromo-2-(2,2-dimethyl-2,3-dihydrobenzofuran-7-yloxy)pyridin-3-yl)-3-(4-(trifluoromethoxy)phenyl)urea;
   1-(5-bromo-2-(2-*tert*-butylphenoxy)pyridin-3-yl)-3-p-tolylurea;
   1-(5-bromo-2-(2-*tert-*butylphenoxy)pyridin-3-yl)-3-(4-*tert*-butylphenyl)urea;
   1-(5-bromo-2-(2-*tert*-butylphenoxy)pyridin-3-yl)-3-(4-(trifluoromethoxy)phenyl)urea;
   1-(4-*tert*-butoxyphenyl)-3-(2-(2-*tert-*butylphenoxy)-5-cyanopyridin-3-yl)urea;
   1-(2-(2-*tert*-butylphenoxy)-5-cyanopyridin-3-yl)-3-p-tolylurea;
   1-(2-(2-*tert*-butylphenoxy)-5-cyanophenyl)-3-p-tolylurea;
   1-(2-(2-*tert*-butylphenoxy)-5-cyanophenyl)-3-(4-(trifluoromethoxy)phenyl)urea;
   1-(6-bromo-2-(2-(trifluoromethoxy)phenoxy)pyridin-3-yl)-3-(4-*tert-*butylphenyl)urea;
   1-(4-*tert*-butoxyphenyl)-3-(6-cyano-2-(2,2-dimethyl-2,3-dihydrobenzofuran-7-yloxy)pyridin-3 -yl)urea;
   1-(6-cyano-2-(2,2-dimethyl-2,3-dihydrobenzofuran-7-yloxy)pyridin-3-yl)-3-p-tolylurea;
   1-(4-*tert*-butylphenyl)-3-(6-cyano-2-(2,2-dimethyl-2,3-dihydrobenzofuran-7-yloxy)pyridin-3-yl)urea;
   1-(6-cyano-2-(2,2-dimethyl-2,3-dihydrobenzofuran-7-yloxy)pyridin-3-yl)-3-(4-(trifluoromethoxy)phenyl)urea;
   1-(4-*tert*-butoxyphenyl)-3-(6-cyano-2-(2-(trifluoromethoxy)phenoxy)pyridin-3-yl)urea;
   1-(6-cyano-2-(2-(trifluoromethoxy)phenoxy)pyridin-3-yl)-3 -p-tolylurea;
   1-(6-cyano-2-(2-(trifluoromethoxy)phenoxy)pyridin-3-yl)-3-(4-(trifluoromethoxy)phenyl)urea;
   1-(5-chloro-2-(2,2-dimethyl-2,3-dihydrobenzofuran-7-yloxy)pyridin-3-yl)-3-p-tolylurea;
   1-(5-bromo-2-(2,2-dimethyl-2,3-dihydrobenzofuran-7-yloxy)pyridin-3-yl)-3-(4-*tert*-butoxyphenyl)urea;
   1-(5-bromo-2-(2,2-dimethyl-2,3-dihydrobenzofuran-7-yloxy)pyridin-3-yl)-3-p-tolylurea;
   1-(5-bromo-2-(2,2-dimethyl-2,3-dihydrobenzofuran-7-yloxy)pyridin-3-yl)-3-(4-*tert*-butylphenyl)urea;
   1-(5-bromo-2-(2-*tert*-butylphenoxy)pyridin-3-yl)-3-(4-*tert*-butoxyphenyl)urea;
   1-(5-cyano-2-(2,2-dimethyl-2,3-dihydrobenzofuran-7-yloxy)pyridin-3-yl)-3-p-tolylurea;
   1-(4-*tert*-butylphenyl)-3-(5-cyano-2-(2,2-dimethyl-2,3-dihydrobenzofuran-7-yloxy)pyridin-3-yl)urea;
   1-(2-(2-*tert-*butylphenoxy)-5-cyanopyridin-3-yl)-3-(4-(trifluoromethoxy)phenyl)urea;
   1-(4-*tert*-butoxyphenyl)-3-(2-(2-*tert*-butylphenoxy)-5-cyanophenyl)urea;
   1-(2-(2-*tert-*butylphenoxy)-5-cyanophenyl)-3-(4-*tert-*butylphenyl)urea;
   1-(2-(2-*tert*-butylphenoxy)-6-(formamidomethyl)pyridin-3-yl)-3-(4-*tert-*butylphenyl)urea;
   *tert*-butyl 2-(2-(3-(3-(4-*tert*-butylphenyl)ureido)pyridin-2-yloxy)phenoxy)acetate;
   *tert*-butyl 2-(2-(3-(3-(4-(trifluoromethoxy)phenyl)ureido)pyridin-2-yloxy)phenoxy)acetate;
   methyl 4-(2-*tert*-butylphenoxy)-3-(3-p-tolylureido)benzoate;
   methyl 4-(2-*ter*t-butylphenoxy)-3-(3-(4-(trifluoromethoxy)phenyl)ureido)benzoate;
   1-(2-(2-*tert*-butylphenoxy)-5-(trifluoromethyl)pyridin-3-yl)-3-p-tolylurea;
   1-(2-(2-*tert*-butylphenoxy)-5-(trifluoromethyl)pyridin-3-yl)-3-(4-*tert-*butylphenyl)urea;
   1-(2-(2-*tert*-butylphenoxy)-5-(trifluoromethyl)pyridin-3-yl)-3-(4-(trifluoromethoxy)phenyl)urea;
   1-[2-(2-*tert*-Butyl-phenoxy)-pyridin-3-yl]-3-[2-(3-methoxy-phenyl)-ethyl]-urea;
   1-[2-(2-*tert-*Butyl-phenoxy)-pyridin-3-yl]-3-[2-(4-phenoxy-phenyl)-ethyl]-urea;
   1-(2-(benzo[d][1,3]dioxol-5-yl)ethyl)-3-(2-(2-*tert*-butylphenoxy)pyridin-3-yl)urea;
   1-(4-*tert*-butylphenyl)-3-(6-cyano-2-(2-(trifluoromethoxy)phenoxy)pyridin-3-yl)urea;
   1-(5-cyano-2-(2,2-dimethyl-2,3-dihydrobenzofuran-7-yloxy)pyridin-3-yl)-3-(4-(trifluoromethoxy)phenyl)urea;
   1-(3-chloro-4-methoxybenzyl)-3-(2-(2-*tert*-butylphenoxy)pyridin-3-yl)urea;
   (S)-1-(2-(2-*tert*-butylphenoxy)pyridin-3-yl)-3-(2-phenylpropyl)urea;
   1-(2-(2-*tert*-butylphenoxy)pyridin-3-yl)-3-(4-(2-methoxyethoxy)phenyl)urea;
   1-(2-(2-*tert*-butylphenoxy)pyridin-3-yl)-3-(4-(pentyloxy)phenyl)urea;
   1-(2-(2-*tert*-butylphenoxy)pyridin-3-yl)-3-(4-(furan-2-ylmethoxy)phenyl)urea;
   1-(2-(2-*tert*-butylphenoxy)pyridin-3-yl)-3-(4-(furan-3-ylmethoxy)phenyl)urea;
   1-(2-(2-*tert*-butylphenoxy)pyridin-3-yl)-3-(4-((tetrahydrofaran-3-yl)methoxy)phenyl)urea;
   1-(2-(2-*tert*-butylphenoxy)pyridin-3-yl)-3-(4-(1-methoxybutan-2-yloxy)phenyl)urea;
   1-(2-(2-*tert*-butylphenoxy)pyridin-3-yl)-3-(4-(pyridin-2-ylmethoxy)phenyl)urea;
   1-(2-(2-*tert*-butylphenoxy)pyridin-3-yl)-3-(4-(pyridin-3-ylmethoxy)phenyl)urea;
   1-(2-(2-*tert*-butylphenoxy)pyridin-3-yl)-3-(4-(pyridin-4-ylmethoxy)phenyl)urea;
   1-(2-(2-*tert*-butylphenoxy)pyridin-3-yl)-3-(4-(thiophen-3-ylmethoxy)phenyl)urea;
   1-(2-(2-*tert*-butylphenoxy)pyridin-3-yl)-3-(4-(2-(pyridin-2-yl)ethoxy)phenyl)urea;
   1-(2-(2-*tert*-butylphenoxy)pyridin-3-yl)-3-(4-(1-(pyridin-4-yl)ethoxy)phenyl)urea;
   1-(2-(2-*tert*-butylphenoxy)pyridin-3-yl)-3-(4-(2-(pyridin-4-yl)ethoxy)phenyl)urea;
   1-(2-(2-*tert*-butylphenoxy)pyridin-3-yl)-3-(4-(2-(piperidin-1-yl)ethoxy)phenyl)urea;
   1-(4-(4-cyanobenzyloxy)phenyl)-3-(2-(2-*tert*-butylphenoxy)pyridin-3-yl)urea;
   1-(4-(3-cyanobenzyloxy)phenyl)-3-(2-(2-*tert*-butylphenoxy)pyridin-3-yl)urea;
   1-(4-(2-methoxybenzyloxy)phenyl)-3-(2-(2-*tert*-butylphenoxy)pyridin-3-yl)urea;
   1-(4-(4-methoxybenzyloxy)phenyl)-3-(2-(2-*tert*-butylphenoxy)pyridin-3-yl)urea;
   1-(4-(4-chlorobenzyloxy)phenyl)-3-(2-(2-*tert*-butylphenoxy)pyridin-3-yl)urea;
   1-(4-(3-(dimethylamino)benzyloxy)phenyl)-3-(2-(2-*tert*-butylphenoxy)pyridin-3-yl)urea;
   1-(2-(2-*tert*-butylphenoxy)pyridin-3-yl)-3-(4-(naphthalen-2-ylmethoxy)phenyl)urea;
   1-(4-(3-aminopropoxy)phenyl)-3-(2-(2-*tert*-butylphenoxy)pyridin-3-yl)urea;
   1-(4-(2-(trifluoromethyl)benzyloxy)phenyl)-3-(2-(2-*tert*-butylphenoxy)pyridin-3-yl)urea;
   1-[4-(Biphenyl-4-ylmethoxy)-phenyl]-3-[2-(2-*tert*-butyl-phenoxy)-pyridin-3-yl]-urea;
   1-(4-(4-aminobutoxy)phenyl)-3-(2-(2-*tert*-butylphenoxy)pyridin-3-yl)urea;
   1-(4-(3-(trifluoromethoxy)benzyloxy)phenyl)-3-(2-(2-*tert*-butylphenoxy)pyridin-3-yl)urea;
   1-(3-butoxyphenyl)-3-(2-(2-tert-butylphenoxy)pyridin-3-yl)urea;
   1-(2-(2-*tert*-butylphenoxy)pyridin-3-yl)-3-(3-(2-methoxyethoxy)phenyl)urea;
   1-(2-(2-*tert*-butylphenoxy)pyridin-3-yl)-3-(3-(cyclopentyloxy)phenyl)urea;
   1-(2-(2-*tert*-butylphenoxy)pyridin-3-yl)-3-(3-(pentyloxy)phenyl)urea;
   1-(2-(2-*tert*-butylphenoxy)pyridin-3-yl)-3-(3-(furan-2-ylmethoxy)phenyl)urea;
   1-(2-(2-*tert*-butylphenoxy)pyridin-3-yl)-3-(3-(furan-3-ylmethoxy)phenyl)urea;
   1-(2-(2-*tert-*butylphenoxy)pyridin-3-yl)-3-(3-((tetrahydrofuran-2-yl)methoxy)phenyl)urea;
   1-(2-(2-*tert*-butylphenoxy)pyridin-3-yl)-3-(3-((tetrahydrofuran-3-yl)methoxy)phenyl)urea;
   1-(2-(2-*tert*-butylphenoxy)pyridin-3-yl)-3-(3-(3,3-dimethylbutoxy)phenyl)urea;
   1-(2-(2-*tert*-butylphenoxy)pyridin-3-yl)-3-(3-(2-isopropoxyethoxy)phenyl)urea;
   1-(2-(2-*tert-*butylphenoxy)pyridin-3-yl)-3-(3-(1-methoxybutan-2-yloxy)phenyl)urea;
   1-(3-(benzyloxy)phenyl)-3-(2-(2-*tert-*butylphenoxy)pyridin-3-yl)urea;
   1-(2-(2-*tert*-butylphenoxy)pyridin-3-yl)-3-(3-(pyridin-2-ylmethoxy)phenyl)urea;
   1-(2-(2-*tert*-butylphenoxy)pyridin-3-yl)-3-(3-(pyridin-4-ylmethoxy)phenyl)urea;
   1-(2-(2-*tert*-butylphenoxy)pyridin-3-yl)-3-(3-(thiophen-3-ylmethoxy)phenyl)urea;
   1-(2-(2-*tert*-butylphenoxy)pyridin-3-yl)-3-(3-(2-(pyridin-2-yl)ethoxy)phenyl)urea;
   1-(2-(2-*tert*-butylphenoxy)pyridin-3-yl)-3-(3-(1-(pyridin-4-yl)ethoxy)phenyl)urea;
   1-(2-(2-*tert*-butylphenoxy)pyridin-3-yl)-3-(3-(2-(pyridin-4-yl)ethoxy)phenyl)urea;
   1-(2-(2-*tert*-butylphenoxy)pyridin-3-yl)-3-(3-(2-cyclohexylethoxy)phenyl)urea;
   1-(2-(2-*tert*-butylphenoxy)pyridin-3-yl)-3-(3-(3-(dimethylamino)-2,2-dimethylpropoxy)phenyl)urea;
   1-(3-(1-*tert*-butoxypropan-2-yloxy)phenyl)-3-(2-(2-*tert*-butylphenoxy)pyridin-3-yl)urea;
   1-(3-(4-cyanobenzyloxy)phenyl)-3-(2-(2-*tert*-butylphenoxy)pyridin-3-yl)urea;
   1-(3-(3-cyanobenzyloxy)phenyl)-3-(2-(2-*tert*-butylphenoxy)pyridin-3-yl)urea;
   1-(3-(2-methoxybenzyloxy)phenyl)-3-(2-(2-*tert*-butylphenoxy)pyridin-3-yl)urea;
   1-(3-(4-methoxybenzyloxy)phenyl)-3-(2-(2-*tert*-butylphenoxy)pyridin-3-yl)urea;
   1-(3-(4-chlorobenzyloxy)phenyl)-3-(2-(2-*tert*-butylphenoxy)pyridin-3-yl)urea;
   1-(3-(2-aminoethoxy)phenyl)-3-(2-(2-*tert-*butylphenoxy)pyridin-3-y)urea;
   1-(3-(4-acetamidobenzyloxy)phenyl)-3-(2-(2-*tert*-butylphenoxy)pyridin-3-yl)urea;
   1-(3-(2-(trifluoromethyl)benzyloxy)phenyl)-3-(2-(2-*tert*-butylphenoxy)pyridin-3-yl)urea;
   1-[3-(Biphenyl-4-ylmethoxy)-phenyl]-3-[2-(2-*tert*-butyl-phenoxy)-pyridin-3-yl]-urea;
   1-(3-(4-(trifluoromethoxy)benzyloxy)phenyl)-3-(2-(2-*tert*-butylphenoxy)pyridin-3-yl)urea;
   1-(3-(3-(trifluoromethoxy)benzyloxy)phenyl)-3-(2-(2-*tert*-butylphenoxy)pyridin-3-yl)urea;
   1-(2-(2-*tert*-butylphenoxy)pyridin-3-yl)-3-(3-(pyridin-3-ylmethoxy)phenyl)urea;
   1-(3-(3-methoxybenzyloxy)phenyl)-3-(2-(2-*tert*-butylphenoxy)pyridin-3-yl)urea;
   1-(3-(3-(dimethylamino)benzyloxy)phenyl)-3-(2-(2-*tert*-butylphenoxy)pyridin-3-yl)urea;
   1-(2-(2-*tert*-butylphenoxy)pyridin-3-yl)-3-(3-(naphthalen-2-ylmethoxy)phenyl)urea;
   1-(2-(2-*tert*-butylphenoxy)thiophen-3 -yl)-3 -p-tolylurea;
   1-(3-(2-*tert*-butylphenoxy)pyridin-4-yl)-3-p-tolylurea;
   1-(2-(2-*tert*-butylphenoxy)thiophen-3-yl)-3-(4-(trifluoromethoxy)phenyl)urea;
   1-(3-(2-*tert*-butylphenoxy)pyridin-4-yl)-3-(4-(trifluoromethoxy)phenyl)urea;
   1-p-tolyl-3-(2-(2-(trifluoromethyl)phenoxy)pyridin-3-yl)urea;
   1-(4-(trifluoromethoxy)phenyl)-3-(2-(2-(trifluoromethyl)phenoxy)pyridin-3-yl)urea;
   1-(2-(2-*tert*-butylphenoxy)pyridin-3-yl)-3-(4-(2-hydroxypropan-2-yl)phenyl)urea;
   1-(2-(2-cyclopentylpyridin-3-yloxy)pyridin-3-yl)-3-(4-(trifluoromethoxy)phenyl)urea;
   1-(2-(2-cyclopentenylpyridin-3-yloxy)pyridin-3 -yl)-3-(4-(trifluoromethoxy)phenyl)urea;
   1-(2-(2-*tert*-butylphenoxy)pyridin-3-yl)-3-(4-chlorophenyl)urea;
   1-(2-(2-*tert*-butylphenoxy)pyridin-3-yl)-3-(3-phenoxyphenyl)urea;
   1-(benzo[d]thiazol-2-yl)-3-(2-(2-*tert*-butylphenoxy)pyridin-3-yl)urea;
   1-(2-(2-*tert*-butylphenoxy)pyridin-3-yl)-3-(3-chloro-4-methoxyphenyl)urea;
   1-(4-cyclohexylphenyl)-3-(2-(3-(trifluoromethyl)phenoxy)pyridin-3-yl)urea;
   1-(4-(benzyloxy)phenyl)-3-(2-(2-*tert*-butylphenoxy)pyridin-3-yl)urea;
   1-(2-(2-*tert*-butylphenoxy)pyridin-3-yl)-3-m-tolylurea;
   1-(2-(2-*tert*-butylphenoxy)pyridin-3-yl)-3-(3-(trifluoromethyl)phenyl)urea;
   1-biphenyl-3-yl-3-[2-(2-*tert-*butyl-phenoxy)-pyridin-3-yl]-urea;
   1-(4-(trifluoromethoxy)phenyl)-3-(2-(3-(trifluoromethyl)phenoxy)pyridin-3-yl)urea;
   1-(2-(2-*tert*-butylphenoxy)pyridin-3-yl)-3-(4-chloro-3-(trifluoromethyl)phenyl)urea;
   1-(2-(2-*tert*-butylphenoxy)pyridin-3-yl)-3-(4-methylthiazol-2-yl)urea;
   1-(2-(2-*tert*-butylphenoxy)pyridin-3-yl)-3-(3-fluorophenyl)urea;
   1-(3,4-dimethylphenyl)-3 -(2-(3 -(trifluoromethyl)phenoxy)pyridin-3 -yl)urea;
   1-(2-(2-*tert*-butylphenoxy)pyridin-3-yl)-3-phenylurea;
   1-(2,3-dihydro-1H-inden-5-yl)-3-(2-(3-(trifluoromethyl)phenoxy)pyridin-3-yl)urea;
   1-(2-fluorophenyl)-3-(2-(3-(trifluoromethyl)phenoxy)pyridin-3-yl)urea;
   1-(2-(2-*tert*-butylphenoxy)pyridin-3-yl)-3-(3-chlorophenyl)urea;
   1-p-tolyl-3-(2-(3-(trifluoromethyl)phenoxy)pyridin-3-yl)urea;
   1-(5-*tert-*butyl-1,3,4-thiadiazol-2-yl)-3-(2-(2-*tert*-butylphenoxy)pyridin-3-yl)urea;
   1-(2-fluoro-5-methylphenyl)-3-(2-(3-(trifluoromethyl)phenoxy)pyridin-3-yl)urea;
   1-(4-bromophenyl)-3-(2-(3-(trifluoromethyl)phenoxy)pyridin-3-yl)urea;
   1-(4-(benzyloxy)phenyl)-3-(2-(3-(trifluoromethyl)phenoxy)pyridin-3-yl)urea;
   1-(2-(3-(trifluoromethyl)phenoxy)pyridin-3-yl)-3-(4-(trifluoromethyl)phenyl)urea;
   1-(4-chlorophenyl)-3-(2-(3-(trifluoromethyl)phenoxy)pyridin-3-yl)urea;
   1-(3-fluoro-4-methylphenyl)-3-(2-(3-(trifluoromethyl)phenoxy)pyridin-3-yl)urea;
   1-(2-(2-*tert*-butylphenoxy)pyridin-3-yl)-3-((1R,2R,3R,5S)-2,6,6-trimethylbicyclo[3.1.1]heptan-3-yl)urea;
   1-(2-(2-*tert*-butylphenoxy)pyridin-3-yl)-3-(pyridin-2-yl)urea;
   1-(2-(2-*tert*-butylphenoxy)pyridin-3-yl)-3-(3-methoxyphenyl)urea;
   1-(3,5-dichlorobenzyl)-3-(2-(2-*tert*-butylphenoxy)pyridin-3-yl)urea;
   1-(2,5-difluorophenyl)-3-(2-(3-(trifluoromethyl)phenoxy)pyridin-3-yl)urea;
   1-(3,4-dichlorophenyl)-3-(2-(3-(trifluoromethyl)phenoxy)pyridin-3-yl)urea;
   1-(3,5-dimethylphenyl)-3-(2-(3-(trifluoromethyl)phenoxy)pyridin-3-yl)urea;
   1-(2-(2-*tert*-butylphenoxy)pyridin-3-yl)-3-(2-fluorophenyl)urea;
   1-biphenyl-4-ylmethyl-3-[2-(2-*tert*-butyl-phenoxy)-pyridin-3-yl]-urea;
   1-m-tolyl-3-(2-(3-(trifluoromethyl)phenoxy)pyridin-3-yl)urea;
   1-(2-(2-*tert*-butylphenoxy)pyridin-3-yl)-3-(thiazol-2-yl)urea;
   1-(2-(2-*tert*-butylphenoxy)pyridin-3-yl)-3-(2-hydroxyphenyl)urea;
   1-(2-(2-*tert*-butylphenoxy)pyridin-3-yl)-3-(4-cyanophenyl)urea;
   1-(2-(2-*tert*-butylphenoxy)pyridin-3-yl)-3-(2-chlorophenyl)urea;
   1-biphenyl-2-ylmethyl-3-[2-(2-*tert*-butyl-phenoxy)-pyridin-3-yl]-urea;
   1-(4-chloro-3-(trifluoromethyl)benzyl)-3-(2-(2-*tert*-butylphenoxy)pyridin-3-yl)urea;
   1-(3-chlorophenyl)-3-(2-(3-(trifluoromethyl)phenoxy)pyridin-3-yl)urea;
   1-(3,5-difluorophenyl)-3-(2-(3-(trifluoromethyl)phenoxy)pyridin-3-yl)urea;
   1-(4-(difluoromethoxy)phenyl)-3-(2-(3-(trifluoromethyl)phenoxy)pyridin-3-yl)urea;
   1-(3-(trifluoromethoxy)phenyl)-3-(2-(3-(trifluoromethyl)phenoxy)pyridin-3-yl)urea;
   1-(2,4-dichlorobenzyl)-3-(2-(2-*tert*-butylphenoxy)pyridin-3-yl)urea;
   1-(2-(2-*tert*-butylphenoxy)pyridin-3-yl)-3-(5-(trifluoromethyl)-1,3,4-thiadiazol-2-yl)urea;
   1-(2-(trifluoromethyl)benzyl)-3-(2-(2-*tert*-butylphenoxy)pyridin-3-yl)urea;
   1-(2-(2-*tert*-butylphenoxy)pyridin-3-yl)-3-(6-chloropyridin-3-yl)urea;
   (S)-1-(1-(4-bromophenyl)ethyl)-3-(2-(2-*tert*-butylphenoxy)pyridin-3-yl)urea.;
   1-(2-(2-*tert*-butylphenoxy)pyridin-3-yl)-3-(3-methylisoxazol-5-yl)urea;
   1 -(4-fluorophenyl)-3 -(2-(3 -(trifluoromethyl)phenoxy)pyridin-3 -yl)urea;
   1-(3,5-dichlorophenyl)-3-(2-(3-(trifluoromethyl)phenoxy)pyridin-3-yl)urea;
   1-(2-(2-*tert*-butylphenoxy)pyridin-3-yl)-3-(3,3-dimethylbutyl)urea;
   1-(3-chloro-4-methoxyphenyl)-3-(2-(3-(trifluoromethyl)phenoxy)pyridin-3-yl)urea;
   1-(2-(2-*tert*-butylphenoxy)pyridin-3-yl)-3-(quinolin-3-yl)urea;
   1-biphenyl-3-yl-3-[2-(3-trifluoromethyl-phenoxy)-pyridin-3-yl]-urea;
   1-(4-phenoxy-phenyl)-3-[2-(3-trifluoromethyl-phenoxy)-pyridin-3-yl]-urea;
   1-(5-*tert*-butylisoxazol-3-yl)-3-(2-(3-(trifluoromethyl)phenoxy)pyridin-3-yl)urea;
   1-(2-(2-*tert*-butylphenoxy)pyridin-3-yl)-3-(4-*tert*-butylthiazol-2-yl)urea;
   (S)-1-(2-(2-*tert*-butylphenoxy)pyridin-3-yl)-3-(1-phenylethyl)urea;
   1-(2-(2-*tert*-butylphenoxy)pyridin-3-yl)-3-(5-chloro-2-methoxyphenyl)-urea;
   1-(3-phenoxyphenyl)-3-(2-(3-(trifluoromethyl)phenoxy)pyridin-3-yl)urea;
   1-(2-(2-*tert*-butylphenoxy)pyridin-3-yl)-3-(isoxazol-3-yl)urea;
   1-(2-(2-*tert*-butylphenoxy)pyridin-3-yl)-3-(3-cyanophenyl)urea;
   1-(2,3-dichlorobenzyl)-3-(2-(2-*tert*-butylphenoxy)pyridin-3-yl)urea;
   1-(4-bromobenzyl)-3-(2-(2-*tert*-butylphenoxy)pyridin-3-yl)urea;
   1-(2-chlorobenzyl)-3-(2-(2-*tert*-butylphenoxy)pyridin-3-yl)urea;
   (S)-1-(2-(2-*tert*-butylphenoxy)pyridin-3-yl)-3-(1-(4-methoxyphenyl)ethyl)urea;
   1-(4-methoxy-phenyl)-3-[2-(3-trifluoromethyl-phenoxy)-pyridin-3-yl]-urea;
   1-(2-(2-*tert*-butylphenoxy)pyridin-3-yl)-3-(5-methylpyridin-2-yl)urea;
   1-(4-acetylphenyl)-3-(2-(2-*tert*-butylphenoxy)pyridin-3-yl)urea;
   1-(2-(2-*tert*-butylphenoxy)pyridin-3-yl)-3-(pyrazin-2-yl)urea;
   1-(4-fluorobenzyl)-3-(2-(2-*tert*-butylphenoxy)pyridin-3-yl)urea;
   1-(2-(2-*tert*-butylphenoxy)pyridin-3-yl)-3-(2-chloropyridin-4-yl)urea;
   1 -(2-(2-*tert*-butylphenoxy)pyridin-3-yl)-3-(3-phenylpropyl)urea;
   1-(2-(2-*tert*-butylphenoxy)pyridin-3-yl)-3-(2-chloro-5-methoxyphenyl)urea;
   (S)-1-(2-(2-*tert*-butylphenoxy)pyridin-3-yl)-3-(1-(3-methoxyphenyl)ethyl)urea;
   1-[2-(2-*tert*-butyl-phenoxy)-pyridin-3-yl]-3-(4-methyl-benzyl)-urea;
   1-(thiazol-2-yl)-3-(2-(3-(trifluoromethyl)phenoxy)pyridin-3-yl)urea;
   1-(2-(2-*tert-*butylphenoxy)pyridin-3-yl)-3-cyclohexylurea;
   1-(6-bromo-2-(2-(2-ethoxypropan-2-yl)phenoxy)pyridin-3-yl)-3-(4-(trifluoromethoxy)phenyl)urea;
   1-(6-cyano-2-(2-(2-ethoxypropan-2-yl)phenoxy)pyridin-3-yl)-3-(4-(trifluoromethoxy)phenyl)urea;
   1-(4-(trifluoromethoxy)phenyl)-3-(2-(2-(2,4,4-trimethylchroman-2-yl)phenoxy)pyridin-3-yl)urea;
   1-(2-(2-(2-methyl-1,3-dioxolan-2-yl)phenoxy)pyridin-3-yl)-3-(4-(trifluoromethoxy)phenyl)urea;
   1-(2-(3-(2-(1H-tetrazol-1-yl)propan-2-yl)phenoxy)pyridin-3-yl)-3-(4-(trifluoromethoxy)phenyl)urea;
   1-(2-(3-(prop-l-en-2-yl)phenoxy)pyridin-3-yl)-3-(4-(trifluoromethoxy)phenyl)urea;
   1-(2-(2-(4-ethoxy-tetrahydro-2H-pyran-4-yl)phenoxy)pyridin-3-yl)-3-(4-(trifluoromethoxy)phenyl)urea;
   1-(2-(2-(3-hydroxypentan-3-yl)phenoxy)pyridin-3-yl)-3-(4-(trifluoromethoxy)phenyl)urea;
   1-(2-(2-isobutylisoindolin-4-yloxy)pyridin-3-yl)-3-(4-(trifluoromethoxy)phenyl)urea;
   1-(2-(3-methyl-1H-inden-4-yloxy)pyridin-3-yl)-3-(4-(trifluoromethoxy)phenyl)urea;
   1-(2-(2-(isobutylamino)phenoxy)pyridin-3-yl)-3-(4-(trifluoromethoxy)phenyl)urea;
   1-(2-(2-*tert*-butylphenoxy)pyridin-3-yl)-3-(4-(1-((R)-1-phenylethylamino)ethyl)phenyl)urea;
   ethyl 3-(3-(3-(4-(trifluoromethoxy)phenyl)ureido)pyridin-2-yloxy)benzoate;
   1-(2-(2-(1,2-dimethoxypropan-2-y1)phenoxy)pyridin-3-yl)-3-(4-(trifluoromethoxy)phenyl)urea;
   1-(2-(3,3-dimethyl-1-oxo-1,3-dihydroisobenzofuran-4-yloxy)pyridin-3-yl)-3-(4-(trifluoromethoxy)phenyl)urea;
   1-(2-(3-(4-hydroxyhepta-1,6-dien-4-yl)phenoxy)pyridin-3-yl)-3-(4-(trifluoromethoxy)phenyl)urea;
   1-(2-(3-(4-methoxyhepta-1,6-dien-4-yl)phenoxy)pyridin-3-yl)-3-(4-(trifluoromethoxy)phenyl)urea;
   1-(2-(3-cyclopentylphenoxy)pyridin-3-y1)-3-(4-(trifluoromethoxy)phenyl)urea;
   1-(2-(2-(1-(*tert-*butyldimethylsilyloxy)-2-methylpropan-2-yl)phenoxy)pyridin-3-yl)-3-(4-(trifluoromethoxy)phenyl)urea;
   1-[2(2-{1-[2-(*tert*-butyl-dimethyl-silanyloxy)-ethoxy]-1-methyl-ethy}-phenoxy)-pyridin-3-yl]-3-(4-trifluoromethoxy-phenyl)-urea;
   1-{2-[2-(1-methyl-1-butoxy-ethyl)-phenoxy]pyridin-3-yl)-3-(4-trifluoromethoxy-pheny)-urea;
   1-[2-(2-{1-[2-(furan-2-ylmethylsulfanyl)-ethoxy]-1-methyl-ethyl}-phenoxy)-pyridin-3-yl]-3-(4-trifluoromethoxy-phenyl)-urea;
   1-(2-{2-[1-(2-isobutysulfanyl-ethoxy)-1-methyl-ethyl]-phenoxy-pyridin-3-yl)-3-(4-trifluoromethoxy-phenyl)-urea;
   1-[2-(2-{1-methyl-1-[2-(pyridin-2-ylsulfanyl)ethoxy]-phenoxy)-pyridin-3-yl]-3-(4-trifluoromethoxy-phenyl)-urea;
   1-[2-(2-{1-methyl-1-[2-(2-methyl-propane-1-sulfinyl)ethoxy)--ethyl)-phenoxy)-pyridin-3-yl]-3-(4-trifluoromethoxy-phenyl)-urea;
   1-(2-{2-[1-(2-diisobutylamino-ethoxy)-1-methyl-ethyl]-phenoxy-pyridin-3-yl)-3-(4-trifluoromethoxy-phenyl)-urea;
   1-[2-(2-{1-methyl-1-[2-(pyridin-2-sulfonyl)ethoxy)-ethyl)-phenoxy)-pyridin-3-yl]-3-(4-trifluoromethoxy-phenyl)-urea;
   methyl 2-fluoro-6-(3-(3-(4-(trifluoromethoxy)phenyl)ureido)pyridin-2-yloxy)benzoate;
   1-(2-(3,3-dimethyl-1,3-dihydroisobenzofuran-4-yloxy)pyridin-3-yl)-3-(4-(trifluoromethoxy)phenyl)urea;
   1-(2-(2-acetyl-3-fluorophenoxy)pyridin-3-yl)-3-(4-(trifluoromethoxy)phenyl)urea;
   1-(2-(2-(4-methoxybenzyl)isoindolin-4-yloxy)pyridin-3-yl)-3-(4-(trifluoromethoxy)phenyl)urea;
   1-(2-(2-neopentylisoindolin-4-yloxy)pyridin-3-yl)-3-(4-(trifluoromethoxy)phenyl)urea;
   1-(2-(2-neopentyl-1,2,3,4-tetrahydroisoquinolin-8-yloxy)pyridin-3-yl)-3-(4-(trifluoromethoxy)phenyl)urea;
   1-(2-(3-tert-butyl-2-methoxyisoindolin-4-yloxy)pyridin-3-yl)-3-(4-(trifluoromethoxy)phenyl)urea;
   1-(2-(3-tert-butylisoindolin-4-yloxy)pyridin-3-yl)-3-(4-(trifluoromethoxy)phenyl)urea;
   1-(2-(2-(1-benzoylpiperidin-4-yl)phenoxy)pyridin-3-yl)-3-(4-(trifluoromethoxy)phenyl)urea;
   1-(2-(2-(1-isobutylpiperidin-4-yl)phenoxy)pyridin-3-yl)-3-(4-(trifluoromethoxy)phenyl)urea;
   1-(2-(2-(1-neopentylpiperidin-4-yl)phenoxy)pyridin-3-yl)-3-(4-(trifluoromethoxy)phenyl)urea;
   1-(2-(2-(cyclohexylmethyl)-1,2,3,4-tetrahydroisoquinolin-5-yloxy)pyridin-3-yl)-3-(4-(trifluoromethoxy)phenyl)urea;
   1-(2-(2-(furan-2-ylmethyl)-1,2,3,4-tetrahydroisoquinolin-5-yloxy)pyridin-3-yl)-3-(4-(trifluoromethoxy)phenyl)urea;
   1-(2-(2-(2-morpholinobenzyl)-1,2,3,4-tetrahydroisoquinolin-5-yloxy)pyridin-3-yl)-3-(4-(trifluoromethoxy)phenyl)urea;
   1-(2-(2-(2-methylbutyl)-1,2,3,4-tetrahydroisoquinolin-5-yloxy)pyridin-3-yl)-3-(4-(trifluoromethoxy)phenyl)urea;
   1-(2-(2-(3,3-dimethylbutyl)-1,2,3,4-tetrahydroisoquinolin-5-yloxy)pyridin-3-yl)-3-(4-(trifluoromethoxy)phenyl)urea;
   1-(2-(2-(bicyclo[2.2.1]hept-5-en-2-ylmethyl)-1,2,3,4-tetrahydroisoquinolin-5-yloxy)pyridin-3-yl)-3-(4-(trifluoromethoxy)phenyl)urea;
   1-(2-(2-butyl-1,2,3,4-tetrahydroisoquinolin-5-yloxy)pyridin-3-yl)-3-(4-(trifluoromethoxy)phenyl)urea;
   1-(2-(2-hexyl-1,2,3,4-tetrahydroisoquinolin-5-yloxy)pyridin-3-yl)-3-(4-(trifluoromethoxy)phenyl)urea;
   1-(2-(2-(2-phenylpropyl)-1,2,3,4-tetrahydroisoquinolin-5-yloxy)pyridin-3-yl)-3-(4-(trifluoromethoxy)phenyl)urea;
   1-(2-(2-(3-phenylpropyl)-1,2,3,4-tetrahydroisoquinolin-5-yloxy)pyridin-3-yl)-3-(4-(trifluoromethoxy)phenyl)urea;
   1-(2-(2-isobutyl-1,2,3,4-tetrahydroisoquinolin-5-yloxy)pyridin-3-yl)-3-(4-(trifluoromethoxy)phenyl)urea;
   1-(2-(2-isopentyl-1,2,3,4-tetrahydroisoquinolin-5-yloxy)pyridin-3-yl)-3-(4-(trifluoromethoxy)phenyl)urea;
   1-(2-(2-((1-methyl-1H-pyrrol-2-yl)methyl)-1,2,3,4-tetrahydroisoquinolin-5-yloxy)pyridin-3-yl)-3-(4-(trifluoromethoxy)phenyl)urea;
   1-(2-(2-(2-cyanobenzyl)-1,2,3,4-tetrahydroisoquinolin-5-yloxy)pyridin-3-yl)-3-(4-(trifluoromethoxy)phenyl)urea;
   1-(2-(2-(2-fluorobenzyl)-1,2,3,4-tetrahydroisoquinolin-5-yloxy)pyridin-3-yl)-3-(4-(trifluoromethoxy)phenyl)urea;
   1-(2-(2-phenethyl-1,2,3,4-tetrahydroisoquinolin-5-yloxy)pyridin-3-yl)-3-(4-(trifluoromethoxy)phenyl)urea;
   1-(2-(2-(benzo[d][1,3]dioxol-5-ylmethyl)-1,2,3,4-tetrahydroisoquinolin-5-yloxy)pyridin-3-yl)-3-(4-(trifluoromethoxy)phenyl)urea;
   1-(2-(2-((1H-pyrrol-2-yl)methyl)-1,2,3,4-tetrahydroisoquinolin-5-yloxy)pyridin-3-yl)-3 -(4-(trifluoromethoxy)phenyl)urea;
   1-(2-(2-(2-hydroxybenzyl)-1,2,3,4-tetrahydroisoquinolin-5-yloxy)pyridine-3-yl)-3-(4-(trifluoromethoxy)phenyl)urea;
   1-(2-(2-neopentyl-1,2,3,4-tetrahydroisoquinolin-5-yloxy)pyridin-3-yl)-3-(4-(trifluoromethoxy)phenyl)urea;
   1-(2-(2-(thiophen-2-ylmethyl)-1,2,3,4-tetrahydroisoquinolin-5-yloxy)pyridin-3-yl)-3-(4-(trifluoromethoxy)phenyl)urea;
   1-(2-(2-(3-cyanobenzyl)-1,2,3,4-tetrahydroisoquinolin-5-yloxy)pyridin-3-yl)-3-(4-(trifluoromethoxy)phenyl)urea;
   1-(2-(2-(furan-3-ylmethyl)-1,2,3,4-tetrahydroisoquinolin-5-yloxy)pyridin-3-yl)-3-(4-(trifluoromethoxy)phenyl)urea;
   1-(2-(2-(4-cyanobenzyl)-1,2,3,4-tetrahydroisoquinolin-5-yloxy)pyridin-3-yl)-3-(4-(trifluoromethoxy)phenyl)urea;
   methyl 4-((5-(3-(3-(4-(trifluoromethoxy)phenyl)ureido)pyridin-2-yloxy)-3,4-dihydroisoquinolin-2(1H)-yl)methyl)benzoate;
      or (b) is a tautomer, N-oxide, pharmaceutically acceptable salt, or solvate thereof;
   for use in a method for treating and/or preventing a CNS disorder.
20. The compound for use according to aspect 1, wherein:
   X₂ is -(CR¹⁶R¹⁷)_{S}-;
   R¹⁶ is, independently at each occurrence, H, or C₁₋₆ alkyl; and
   R¹⁷ is, independently at each occurrence, H or C₁₋₆ alkyl.
21. The compound for use according to aspect 20, wherein:
   R¹⁶ and R¹⁷ are H.
22. The compound for use according to aspect 21, wherein:
   X₂ is a bond.
23. The compound for use according to any one of aspects 1 or 20 to 22, wherein:
   W is O.
24. The compound for use according to any one of aspects 1 or 20 or 23, wherein:
   Y is O or S.
25. The compound for use according to any one of aspects 1 or 20 to 24, wherein:
   Y is O.
26. The compound for use according to any one of aspects 1 or 20 to 25, wherein:
   ring A is substituted with 0-5 R¹ and selected from 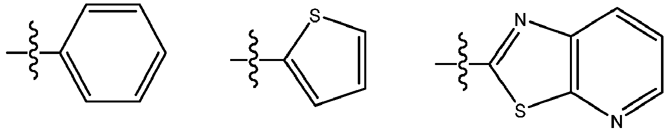 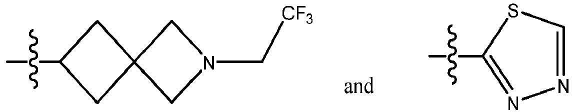
27.The compound for use according to aspect 26, wherein:
   ring A is substituted with 0-5 R¹ and selected from 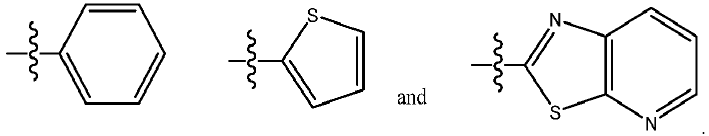
28. The compound for use according to any one of aspects 1 or 20 to 27, wherein ring A is substituted with 0 R¹.
29. The compound for use according to aspect 28, wherein ring A is: 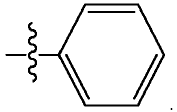
30. The compound for use according to any one of aspects 1 or 20 to 27, wherein:
   ring A is substituted with 1-2 R¹;
   R¹ is, independently at each occurrence, F, Cl, CF₃, -CF₂CF₃, OCF₃, -OCF₂CF₂H, - OCF₂CF₃, -SF₅, -(CR^{f}R^{f})ᵣ-OR^{c}" -(CR^{f}R^{f})ᵣ-CO₂R^{c}, C₁₋₈ alkyl, -(CR^{f}R^{f})ᵣ-C₃₋₁₃ carbocycle substituted with 0-5 R^{b};
   alternatively, two R¹s on two adjacent carbon atoms are combined with the carbon atoms to which they are attached to form a 5-7 membered carbocycle or heterocycle comprising: carbon ring atoms and 0-3 additional ring heteroatoms selected from N, NR¹¹, O, S(O)ₚ, and 0-2 carbonyl groups, wherein said carbocycle or heterocycle is substituted with 0-4 R^{b}.
31. The compound for use according to aspect 30, wherein:
   ring A is substituted with 1-2 R¹;
   R¹ is, independently at each occurrence, F, Cl, CF₃, OCF₃, -SF₅, -OCH₃, C₁₋₄ alkyl, phenyl substituted with 1 R^{b};
   alternatively, two R¹s on two adjacent carbon atoms are combined with the carbon atoms to which they are attached to form a 5 membered heterocycle comprising 2 additional ring heteroatoms selected from O wherein said carbocycle or heterocycle is substituted with 2 R^{b}
   R^{b} is, independently at each occurrence, H, F, or C₁₋₈ alkyl substituted with 2 R^{a};
   R^{a} is, independently at each occurrence, OH or NMe₂.
32. The compound for use according to aspect 31, wherein:
   ring A is selected from: 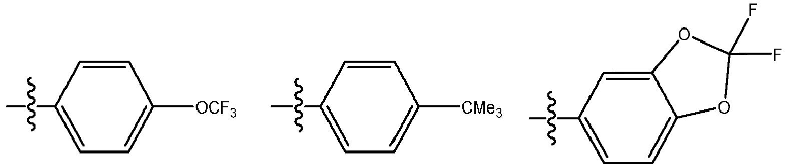 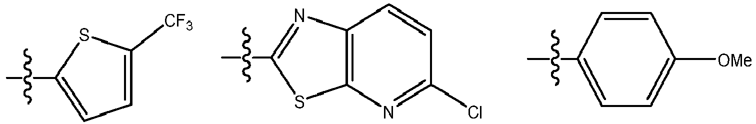 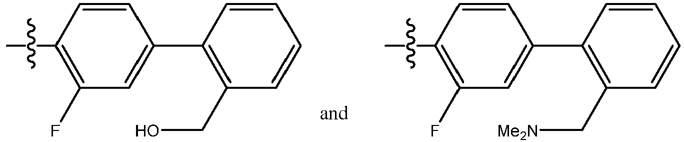
33. The compound for use according to aspect 32, wherein:
   ring A is selected from: 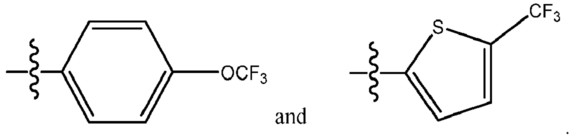
34. The compound for use according to any one of aspects 1 or 20 to 33, wherein:
   ring B is substituted with 0-3 R⁷ and selected from: 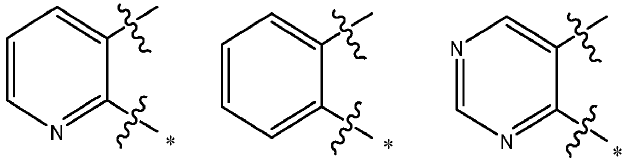 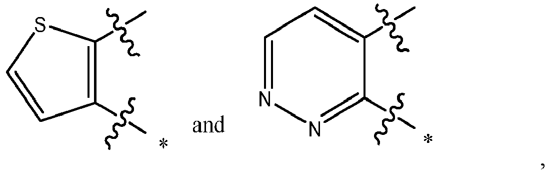 wherein * denotes connection to Y.
35. The compound for use according to any one of aspects 1 or 20 to 34, wherein:
   ring B is substituted with 0-2 R⁷;
   R⁷ is, independently at each occurrence, F, Cl, Br, OR^{c}, CF₃, CF₂H, -SF₅, CN,-NR¹²H, C₁₋₈ alkyl substituted with 0-2 R^{a}, C₂₋₈ alkenyl substituted with 0-2 R^{a}, C₂₋₈ alkynyl substituted with 0-2 R^{a}; phenyl substituted with 0-2 R^{j}, or 5- to 10-membered heteroaryl substituted with 0-2 C₁₋₄ alkyl;
   R^{j} is CN, or
   alternatively, two R^{j}s on two adjacent carbon atoms are combined with the carbon atoms to which they are attached to form a 5-7 membered carbocycle or heterocycle comprising: carbon ring atoms and 0-3 additional ring heteroatoms selected from N, NR¹¹, or O or S(O)_{p.}
36. The compound for use according to aspect 35, wherein:
   ring B is substituted with 0-1 R⁷;
   R⁷ is Br, OH, CF₃, CF₂H, -SF₅, CN, -NH-tetrahydropyran, C₁₋₈ alkyl, C₂₋₈ alkenyl, C₂₋₈ alkynyl, phenyl substituted with 0-1 R^{j}, or N-methylpyrazole;
   R^{j} is CN, or
   alternatively, two R^{j}s on two adjacent carbon atoms are combined with the carbon atoms to which they are attached to form a 6 membered heterocycle comprising: carbon ring atoms and 2 additional ring heteroatoms selected from NH or O.
37. The compound for use according to aspect 36, wherein:
   ring B is substituted with 1 R⁷;
   R⁷ is OH.
38. The compound for use according to aspect 37, wherein:
   ring B is selected from: 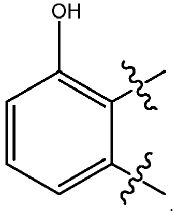
39. The compound for use according to any one of aspects 1 or 20 to 36, wherein:
   ring B is unsubstituted.
40. The compound for use according to aspect 39, wherein:
   ring B is: 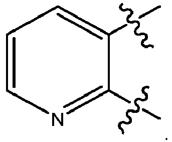
41. The compound for use according to any one of aspects 1 or 20 to 40, wherein:
   R⁶ is -(CH₂)₀₋₁-phenyl substituted with 0-3 R^{6a} or -(CH₂)₀₋₁-pyridinyl substituted with 0-3 R^{6a}_{.}
42. The compound for use according to aspect 41, wherein:
   R⁶ is -(CH₂)₀₋₁-phenyl substituted with 1-2 R^{6a} or -(CH₂)₀₋₁-pyridinyl substituted with 1-2 R^{6a}_{.}
43. The compound for use according to aspect 42, wherein:
   R⁶ is -(CH₂)₀₋₁-phenyl substituted with 1-2 R^{6a}_{.}
44. The compound for use according to aspect 43, wherein:
   R⁶ is phenyl substituted with 1-2 R^{6a}_{.}
45. The compound for use according to any one of aspects 1 or 20 to 44, wherein at least one R^{6a} is in the 2-position of the R⁶ group relative to the Y group.
46. The compound for use according to any one of aspects 1 or 20 to 45, wherein:
   R^{6a} is, independently at each occurrence, F, Cl, Br, I, -(CRⁱRⁱ)ᵣ-OR^{c}, CN, NO₂, CF₃, OCF₃,-CF₂CF₃, -OCF₂CF₂H, -OCF₂CF₃, -SF₅, -NR^{12a}R^{13a}, -C(O)R^{c}, -C(O)OR^{c},-C(O)NR¹²R¹³, -NR¹⁴C(O)R^{d}, -S(O)ₚNR¹²R¹³, -S(O)R^{d}, -S(O)₂R^{d}, C₁₋₄ haloalkyl, C₁₋₄ haloalkyloxy-, C₁₋₄ alkyloxy-, C₁₋₄ alkylthio-, C₁-C₄ alkyl-C(O)-, C₁₋₄ alkyl-O-C(O)-, C₁₋₄ alkyl-C(O)NH-, C₁₋₈ alkyl substituted with 0-2 R^{a}, C₂₋₈ alkenyl substituted with 0-2 R^{a}, C₂₋₈ alkynyl substituted with 0-2 R^{a}, -(CR^{f}R^{f})ᵣ-C₃₋₁₀ carbocycle substituted with 0-2 R^{e}, - (CR^{f}R^{f})ᵣ-5- to 10-membered heteroaryl comprising: carbon ring atoms and 1-2 ring heteroatoms selected from N, NR¹¹, O, and S(O)ₚ, or -(CR^{f}R^{f})ᵣ-5- to 6-membered non-aromatic heterocycle comprising 1-2 heteroatoms selected from 0-1 N and 0-2 O, wherein said heteroaryl and said non-aromatic heterocycle are each substituted with 0-2 R^{e};
   alternatively, when two R^{6a} groups are attached to adjacent atoms, together with the atoms to which they are attached they form a 5- to 7-membered carbocyclic or heterocyclic ring comprising: carbon ring atoms and 0-2 ring heteroatoms selected from N, NR¹¹, O, and S(O)ₚ, 0-1 carbonyl and 0-3 ring double bonds, wherein said carbocyclic or heterocyclic ring is substituted with 0-2 R^{b};
   R^{a} is, independently at each occurrence, F, OCF₃, CF₃, OR^{c}, SR^{c}, CN, -NR^{12a}R^{13a}, -C(O)R^{c}, -C(O)OR^{c}, -C(O)NR¹²R¹³, -NR¹⁴C(O)R^{d}, -S(O)ₚNR¹²R¹³,
   -S(O)R^{d}, -S(O)₂R^{d}, or-(CH₂)ᵣ-C₃₋₁₀ carbocycle substituted with 0-3 R^{e};
   R^{12a} and R^{13a} are each independently selected from C₁₋₆ alkyl, or -(CH₂)ₙ-phenyl.
47. The compound for use according to aspect 46, wherein:
   R^{6a} is, independently at each occurrence, F, Cl, Br, I,-(CRⁱRⁱ)ᵣ-OR^{c}, CN, CF₃, OCF₃,-CF₂CF₃, -OCF₂CF₂H, -OCF₂CF₃, -SF₅, -NR^{12a}R^{13b}, -S(O)R^{d}, -S(O)₂R^{d}, C₁₋₄ haloalkyl, C₁₋₈ alkyl substituted with 0-2 R^{a}, -(CR^{f}R^{f})ᵣ-C₃₋₁₀ carbocycle substituted with 0-2 R^{e}, -(CR^{f}R^{f})ᵣ-5- to 10-membered heteroaryl comprising: carbon ring atoms and 1-2 ring heteroatoms selected from N, NR¹¹, O, and S(O)ₚ, or -(CR^{f}R^{f})ᵣ-5- to 6-membered non-aromatic heterocycle comprising ring 1-2 heteroatoms selected from 0-1 N and 0-2 O, wherein said heteroaryl and said non-aromatic heterocycle are each substituted with 0-2 R^{e};
   alternatively, when two R^{6a} groups are attached to adjacent atoms, together with the atoms to which they are attached they form a 5- to 7-membered carbocyclic or heterocyclic ring comprising: carbon ring atoms and 0-2 ring heteroatoms selected from N, NR¹¹, O, and S(O)ₚ, 0-1 carbonyl and 0-3 ring double bonds, wherein said carbocyclic or heterocyclic ring is substituted with 0-2 R^{b}.
48. The compound for use according to aspect 47, wherein:
   R^{6a} is, independently at each occurrence, F, Cl, Br, I, OH, CN, CF₃, OCF₃,-CF₂CF₃, -OCF₂CF₂H, -OCF₂CF₃, -SF₅, -NR^{12a}R^{13a}, -S(O)₂R^{d}, C₁₋₄ haloalkyl, C₁₋₈ alkyl substituted with 0-2 R^{a}, C₃₋₁₀ carbocycle substituted with 0-2 R^{e}, 5- to 10-membered heteroaryl comprising: carbon ring atoms and 1-2 ring heteroatoms selected from N, NR¹¹, O, and S(O)ₚ, or 5- to 6-membered non-aromatic heterocycle comprising ring 1-2 heteroatoms selected from 0-1 N and 0-2 O, wherein said heteroaryl and said non-aromatic heterocycle are each substituted with 0-2 R^{e};
   alternatively, when two R^{6a} groups are attached to adjacent atoms, together with the atoms to which they are attached they form a 5- membered heterocyclic ring comprising: carbon ring atoms and 1 ring heteroatom selected from NR¹¹, and 2 ring double bonds.
49. The compound for use according to aspect 48, wherein:
   R^{6a} is, independently at each occurrence, F, Cl, Br, I, OH, CN, CF₃, OCF₃,-CF₂CF₃, -OCF₂CF₂H, -OCF₂CF₃, -SF₅, -NR¹²R¹³, -S(O)₂R^{d}, C₁₋₄ haloalkyl, C₁₋₈ alkyl substituted with 0-2 R^{a}, C₃₋₁₀ carbocycle substituted with 0-2 R^{e}, 5- to 10-membered heteroaryl comprising: carbon ring atoms and 1-2 ring heteroatoms selected from N, NR¹¹, and O, or 5- to 6-membered non-aromatic heterocycle comprising 1-2 heteroatoms selected from 0-1 N and 0-2 O, wherein said heteroaryl and said non-aromatic heterocycle are each substituted with 0-2 R^{e};
   alternatively, when two R^{6a} groups are attached to adjacent atoms, together with the atoms to which they are attached they form a 5- membered heterocyclic ring comprising: carbon ring atoms and 1 ring heteroatom selected from NR¹¹, and 2 ring double bonds.
50. The compound for use according to aspect 49, wherein:
   R^{6a} is, independently at each occurrence, C₁₋₈ alkyl substituted with 0-2 R^{a}, C₃₋₁₀ carbocycle substituted with 0-2 R^{e}, or 5- to 6-membered non-aromatic heterocycle comprising: carbon ring atoms and 1-2 ring heteroatoms selected from 0-1 N and 0-2 O, wherein said heterocycle is substituted with 0-2 R^{e}.
51. The compound for use according to aspect 50, wherein:
   R^{6a} is, independently at each occurrence, C₁₋₄ alkyl, cyclopropyl substituted with CF₃, or piperidine.
52. The compound for use according to aspect51, wherein:
   R^{6a} is, independently at each occurrence, C₁₋₄ alkyl or cyclopropyl substituted with CF₃.
53. The compound for use according to aspect 52, wherein:
   R^{6a} is, independently at each occurrence, *tert*-butyl or (1-CF₃)-cyclopropyl.
54. The compound for use according to any one of aspects 1 or 20 to 53, wherein:
   R^{a} is, independently at each occurrence, F, N(CH₃)₂, or OH.
55. The compound for use according to any one of aspects 1 or 20 to 54, wherein:
   R^{d} is, independently at each occurrence, C₁₋₆ alkyl.
56. The compound for use according to any one of aspects 1 or 20 to 55, wherein:
   R^{e} is, independently at each occurrence, H, CN, -(CF₂)ᵣ-CF₃, and C₁₋₈ alkyl;
   alternatively, two R^{e} groups on the same carbon atom combine to form an oxetane;
   alternatively, two R^{e} groups within the same heterocycle combine to form a methylene bridge.
57. The compound for use according to aspect 56, wherein r is 0.
58. The compound for use according to any one of aspects 1 or 20 to 57, wherein:
   R¹¹ is C₁₋₈ alkyl.
59. The compound for use according to any one of aspects 1 or 20 to 58, wherein:
   R¹² and R^{12a} are C₁₋₆ alkyl, and
   R¹³ and R^{13a} are C₁₋₆ alkyl.
60. The compound for use according to any one of aspects 1 or 20 to 40, wherein:
   R⁶ is 2-*t*-Bu-Ph, 2-(1-CF₃)-cyclopropyl-Ph, or 2-Me-3 -(piperidin-1-yl)-Ph.
61. The compound for use according to aspect 55, wherein:
   R⁶ is 2-*t*-Bu-Ph or 2-(1-CF₃)-cyclopropyl-Ph,.
62. The compound for use according to aspect 1, wherein the formula (I) is selected from the group: 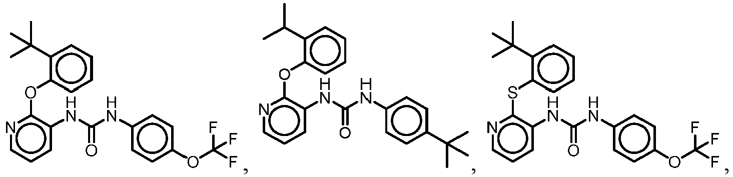 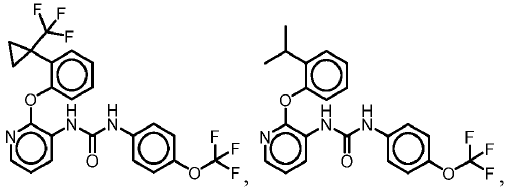 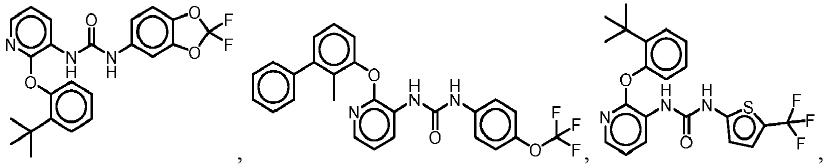 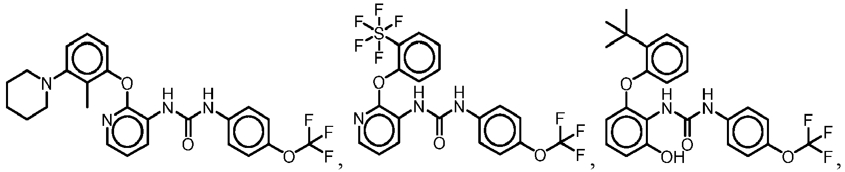 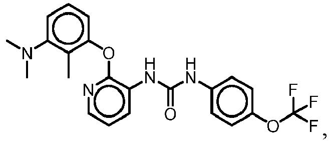 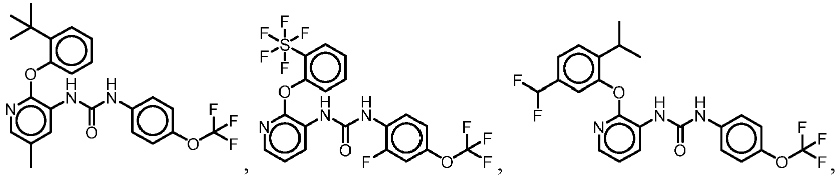 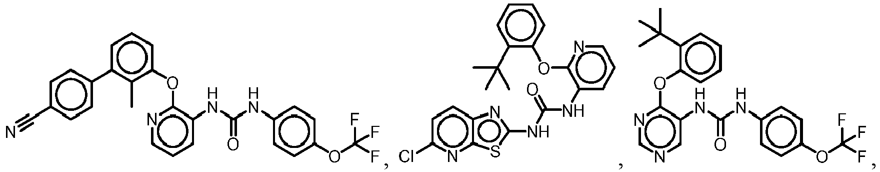 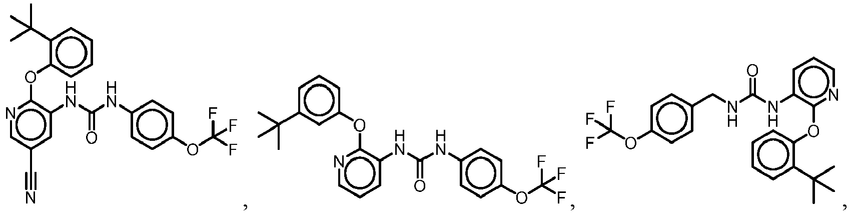 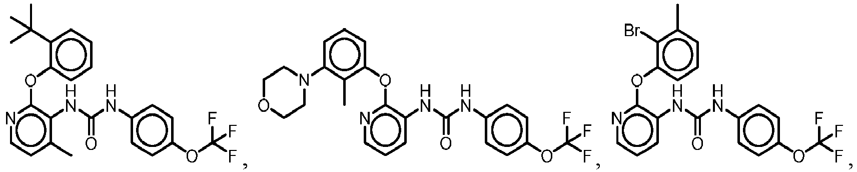 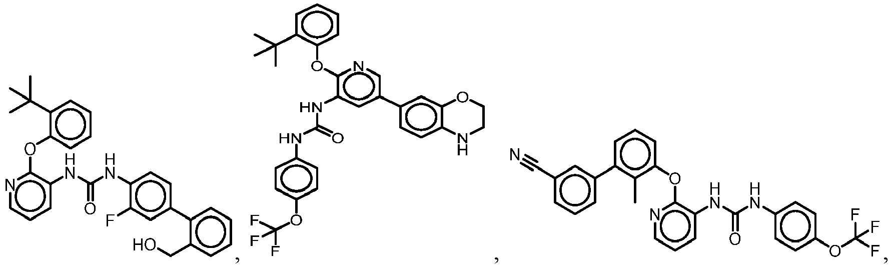 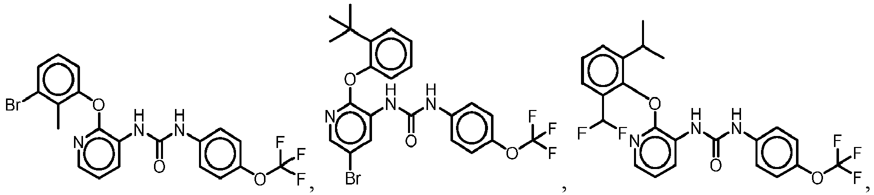 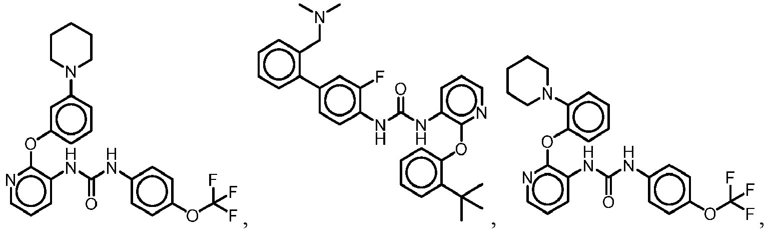 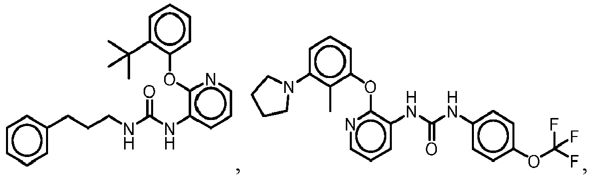 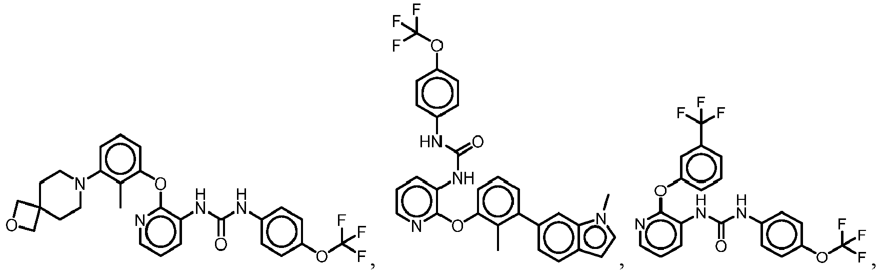 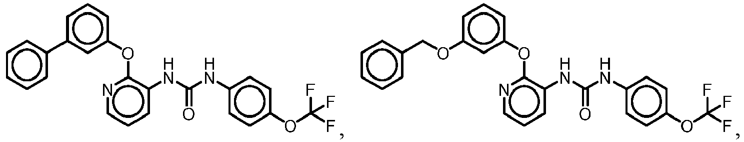 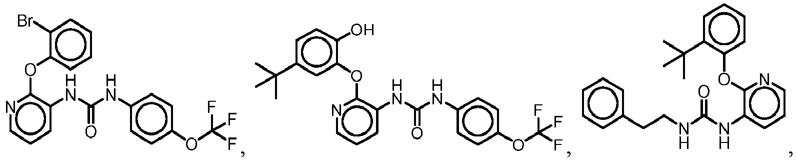 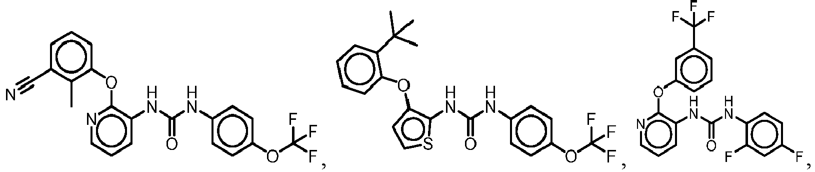 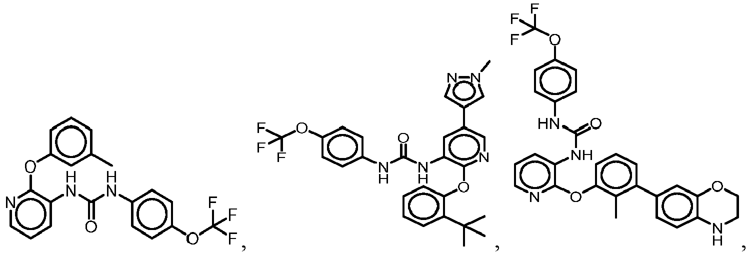 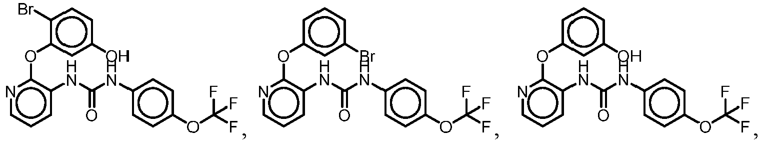 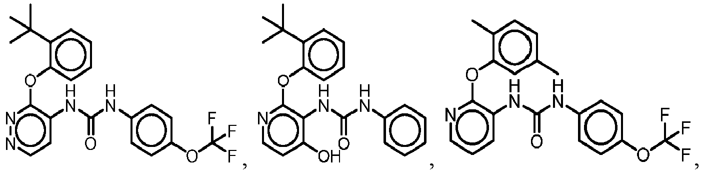 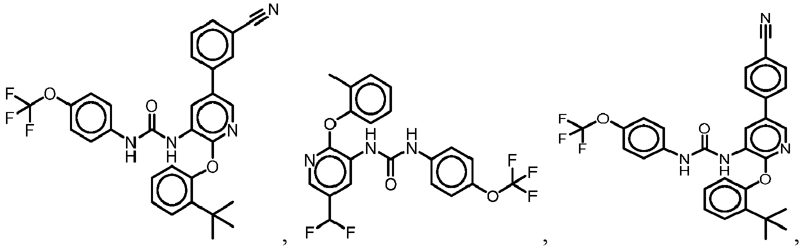 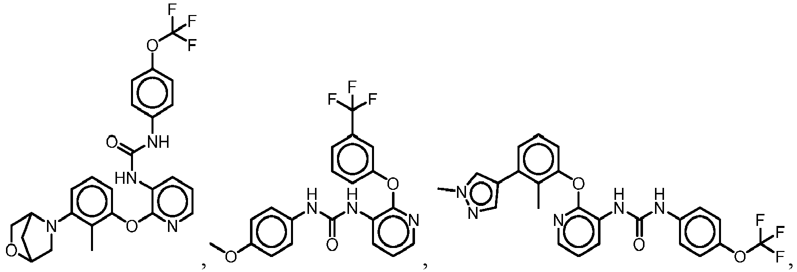 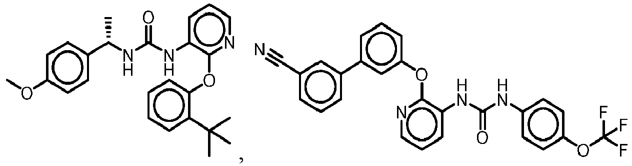 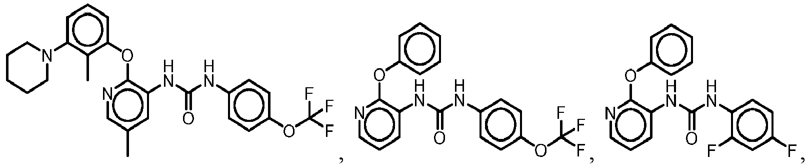 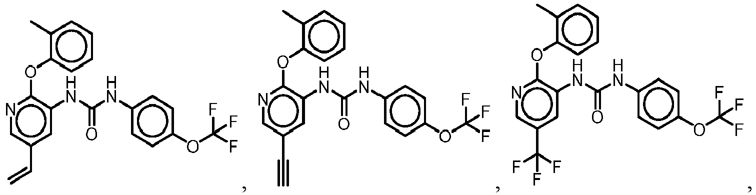 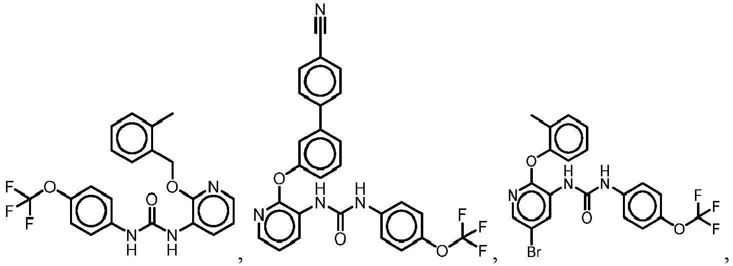 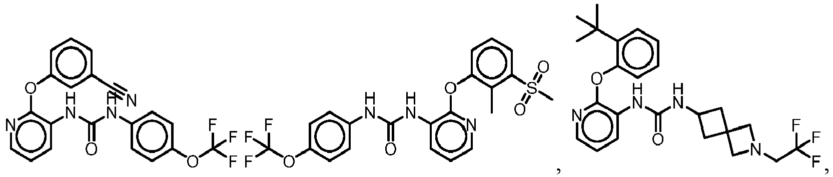 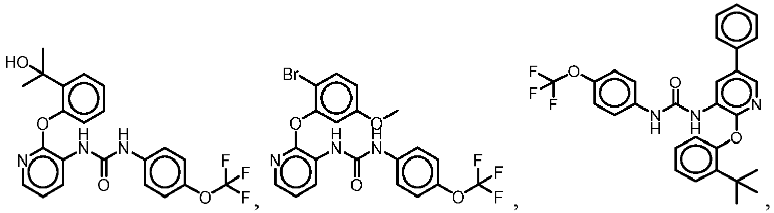 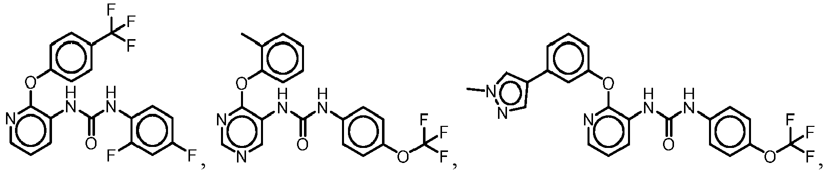 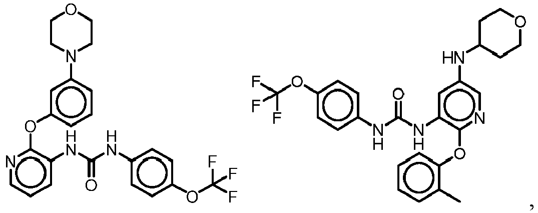 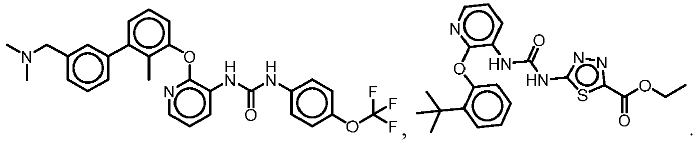
63. The compound for use according to aspect 1, wherein the formula (I) is selected from the group: 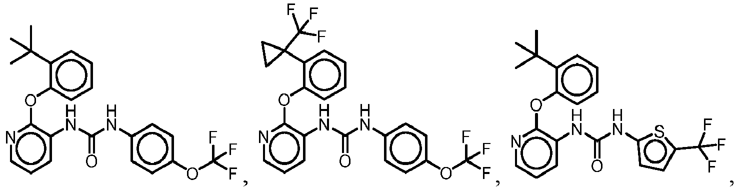 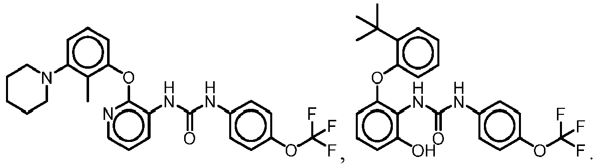
64. The compound for use according to aspect 1, wherein the formula (I) is selected from the group: 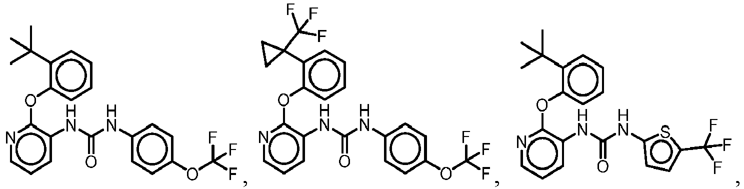 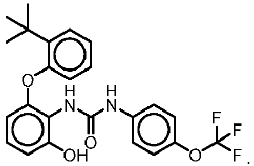
65. The compound for use according to aspect 1, wherein the formula (I) is 1-(2-(2-(*tert-*butyl)phenoxy)pyridin-3-yl)-3-(4-(trifluoromethoxy)phenyl)urea 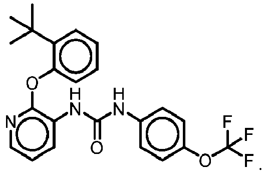
66. A compound that (a) is of Formula (III): 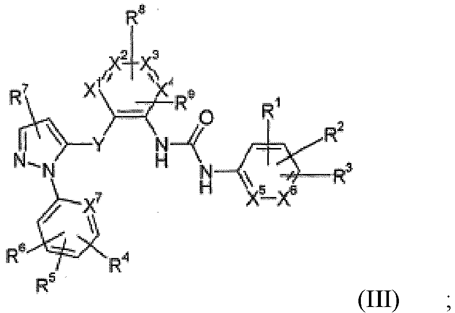 or (b) is a tautomer, N-oxide, pharmaceutically acceptable salt, or solvate thereof;
   wherein:
   X¹, X², X³, X⁴, X⁵, X⁶, and X⁷ are each independently CH or N, with the proviso that no more than two of X¹, X², X³, X⁴ can be N at the same time;
   Y is oxy or thio;
      R¹, R², R³, R⁴, R⁵, and R⁶ are each independently -H, C₁-C₆ alkyl, C₅-C₈ cycloalkyl, cycloheteroalkyl, hydroxy, C₁-C₆ alkoxy, halo, -CF₃, -CF₂CF₃, -OCF₃, -OCF₂CF₃,-OCF₂CF₂H, optionally substituted phenyl, -SiMe₃, -(CR¹⁰R¹¹)ₙ-OR¹², -SR¹³, -CN,-NO₂, -(CR¹⁰R¹¹)ₙNR¹⁴R¹⁵, -(CR¹⁰R¹¹)ₙ-C(O)R¹², -(CR¹⁰R¹¹)ₙ-CO₂R¹², -(CR¹⁰R¹¹)ₙ-C(O)-NR¹⁴R^{15,} or -S(O)ₚR¹⁶;
   R⁷ is -H, C₁-C₄ alkyl, halo, -CF₃, or -(CR¹⁰R¹¹)ₙ-CO₂R¹²;
   R⁸ and R⁹ are each independently -H, C₁-C₆ alkyl, hydroxy, C₁-C₆ alkoxy, halo, -CF₃, or -SR¹³ and are each only bound to a carbon atom;
   R¹⁰ and R¹¹ are each independently at each occurrence -H, C₁-C₄ alkyl, or halo;
   R¹² and R¹³ are each independently at each occurrence -H or C₁-C₆ alkyl;
   R¹⁴ and R¹⁵ are each independently at each occurrence -H, C₁-C₆ alkyl, - C(O)(C₁-C₆ alkyl), -S(O)ₚ(C₁-C₆ alkyl), or R¹⁴ and R¹⁵, taken together in combination with the nitrogen to which they are attached combine to form a piperidinyl or pyrrolidinyl ring;
   R¹⁶ is -H, C₁-C₄ alkyl;
   n, at each occurrence, is selected from 0, 1, 2, 3, and 4; and
   p, at each occurrence, is selected from 0, 1, and 2;
   or a stereoisomer or a pharmaceutically acceptable salt, or solvate thereof;
   for use in a method for treating and/or preventing a CNS disorder.
67. The compound for use according to aspect 66 wherein
   R¹ is C₁-C₆ alkyl, -F, -Cl, -Br, -I, -OCF₃, -OCF₂CF₃, -OCF₂CF₂H, -SR¹³, or - (CR¹⁰R¹¹)ₙ-CO₂R¹².
68. The compound for use according to either of aspects 66 or 67 wherein
   R² and R³ are each independently -H, -F, -Cl, methyl, or methoxy.
69. The compound for use according to either of aspects 66 or 67 wherein
   R² and R³ are each -H.
70. The compound for use according to any one of aspects 66 to 69 wherein
   R⁴ is C₁-C₆ alkyl, C₁-C₆ alkoxyl, -F, -Cl, -Br, -I, -CF₃, -CF₂CF₃, or -(CR¹⁰R¹¹)ₙ-CO₂R¹².
71. The compound for use according to any one of aspects 66 to 70 wherein
   R⁵ and R⁶ are each -H;
   R⁷ is -H or C₁-C₄ alkyl; and
   R⁸ and R⁹ are both -H.
72. The compound for use according to any one of aspects 66 to 71 wherein
   X¹ and X³ are each independently CH or N and X², X⁴, X⁵, X⁶, and X⁷ are all CH.
73. The compound for use according to any one of aspects 66 to 71 wherein
   R¹ is -OCF₃ or t-butyl;
   R², R³, R⁵, R⁶, R⁸, and R⁹ are all -H;
   R⁴ is methyl, ethyl, methoxy, ethoxy, -F, -Cl, or -CF₃; and
   R⁷ is -H or C₁-C₄ alkyl.
74. A compound that (a) is of Formula (IIIA) 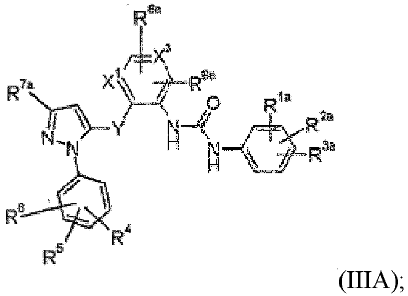
   or (b) is a tautomer, N-oxide, pharmaceutically acceptable salt, or solvate thereof;
   wherein X¹ and X³ are each independently CH or N;
   Y is oxy or thio;
   R^{1a}, R^{2a}, and R^{3a} are each independently -H, C₁-C₆ alkyl, C₁-C₆ alkoxy, halo, -CF₃, -OCF₃, -SR¹³, and -(CR¹⁰R¹¹)ₙ-CO₂R¹²;
   R⁴, R⁵, and R⁶ are each independently -H, C₁-C₆, alkyl, C₅-C₈ cycloalkyl, cycloheteroalkyl, hydroxy, C₁-C₆ alkoxy, halo, -CF₃, -CF₂CF₃, -OCF₃, -OCF₂CF₃, - OCF₂CF₂H, optionally substituted phenyl, -SiMe₃, -(CR¹⁰R¹¹)ₙ-OR¹², -SR¹³, -CN, - NO₂, -(CR¹⁰R¹¹)ₙNR¹⁴R¹⁵, -(CR¹⁰R¹¹)ₙ-C(O)R¹², -(CR¹⁰R¹¹)ₙ-CO₂R¹², -(CR¹⁰R¹¹)ₙ-C(O)-NR¹⁴R¹⁵, or -S(O)ₚR¹⁶;
   R^{7a} is -H, C₁-C₄ alkyl, halo, or -CF₃;
   R^{8a} and R^{9a} are each independently -H, C₁-C₆ alkyl, hydroxy, C₁-C₆ alkoxy, halo, or -CF₃;
   R¹⁰ and R¹¹ are each independently at each occurrence -H, C₁-C₄ alkyl, or halo;
   R¹² and R¹³ are each independently at each occurrence -H or C₁-C₆ alkyl;
   R¹⁴ and R¹⁵ are each independently at each occurrence -H, C₁-C₆ alkyl, -C(O)(C₁-C₆ alkyl), -S(O)ₚ(C₁-C₆ alkyl), or R¹⁴ and R¹⁵, taken together in combination with the nitrogen to which they are attached combine to form a piperidinyl or pyrrolidinyl ring;
   R¹⁶ is -H, C₁-C₄ alkyl;
   n, at each occurrence, is selected from 0, 1, 2, 3, and 4; and
   p, at each occurrence, is selected from 0, 1, and 2;
   or a stereoisomer or pharmaceutically acceptable salt, or solvate hereof;
   for use in a method for treating and/or preventing a CNS disorder.
75. The compound for use according to aspect 74, wherein
   R^{1a} is -C₁-C₆ alkyl, -F, -Cl, -OCF₃, -SR¹³, or -(CR¹⁰R¹¹)ₙ-CO₂R¹²;
   both R^{2a} and R^{3a} are -H;
   R^{4a} is -H, C₁-C₆ alkyl, C₁-C₆ alkoxyl -F, -Cl, -Br, -I, -CF₃, -CF₂CF₃, or -(CR¹⁰R¹¹)ₙ-CO₂R¹²; and
   R^{5a}, R^{6a}, R^{8a}, and R^{9a} are all -H.
76. A compound that (a) is of Formula (IIIB) 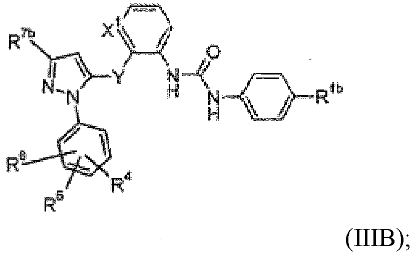 or (b) is a tautomer, N-oxide, pharmaceutically acceptable salt, or solvate thereof; wherein X¹ is CH or N;
   Y is oxy or thio;
   R^{1b} is -H, C₁-C₆ alkyl, C₁-C₆ alkoxy, halo, -CF₃, -OCF₃, -SR¹³, and -(CR¹⁰R¹¹)ₙ-CO₂R¹²;
   R⁴, R⁵, and R⁶ are each independently -H, C₁-C₆ alkyl, C₅-C₈ cycloalkyl, cycloheteroalkyl, hydroxy, C₁-C₆ alkoxy, halo, -CF₃, -CF₂CF₃, -OCF₃, -OCF₂CF₃, - OCF₂CF₂H, optionally substituted phenyl, -SiMe₃, -(CR¹⁰R¹¹)ₙ-OR¹², -SR¹³, -CN, -NO₂, - (CR¹⁰R¹¹)ₙNR¹⁴R¹⁵, -(CR¹⁰R¹¹)ₙ-C(O)R¹², -(CR¹⁰R¹¹)ₙ-CO₂R¹², -(CR¹⁰R¹¹)ₙ-C(O)-NR¹⁴R¹⁵, or -S(O)ₚR¹⁶;
   R^{7b} is -H, C₁-C₄ alkyl, halo, or -CF₃;
   R¹⁰ and R¹¹ are each independently at each occurrence -H, C₁-C₄ alkyl, or halo;
   R¹² and R¹³ are each independently at each occurrence -H or C₁-C₆ alkyl;
   R¹⁴ and R¹⁵ are each independently at each occurrence -H, C₁-C₆ alkyl, -C(O)(C₁-C₆ alkyl), -S(O)ₚ(C₁-C₆ alkyl), or R¹⁴ and R¹⁵, taken together in combination with the nitrogen to which they are attached combine to form a piperidinyl or pyrrolidinyl ring;
   R¹⁶ is -H, C₁-C₄ alkyl;
   n, at each occurrence, is selected from 0, 1, 2, 3, and 4; and
   p, at each occurrence, is selected from 0, 1, and 2;
   or a stereoisomer or a pharmaceutically acceptable salt, or solvate thereof;
   for use in a method for treating and/or preventing a CNS disorder.
77. The compound for use according to aspect 76 wherein
   Y is oxy;
   R^{1b} is optionally substituted phenyl, C₁-C₆ alkyl or -OCF₃;
   R⁴ is C₁-C₆ alkyl, C₁-C₆ alkoxy, -F, -Cl, -Br, -I, -CF₃, -CF₂CF₃, or -(CR¹⁰R¹¹)ₙ-CO₂R¹²;
   R⁵ and R⁶ are each -H; and
   R^{7b} is C₁-C₄ alkyl or halo.
78. The compound for use according to either of aspects 76 or 77 wherein
   X¹ is CH.
79. The compound for use according to any one of aspects 76 to 78 wherein
   R¹⁰, R¹¹, R¹², and R¹³ are all -H in all occurrences.
80. The compound for use according to aspect 66 wherein formula (III) is selected from the group 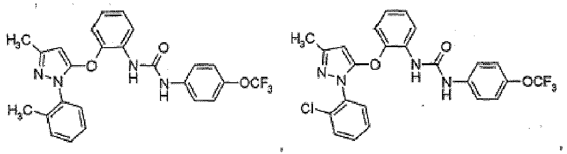 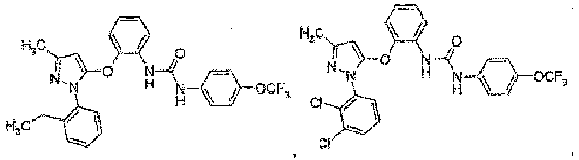 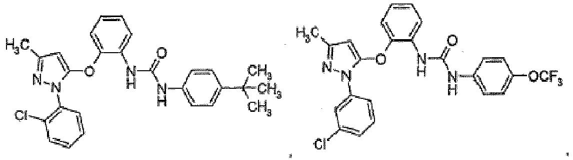 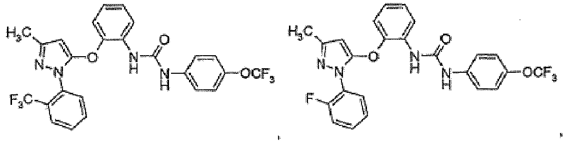 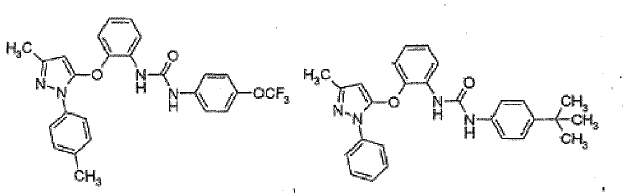 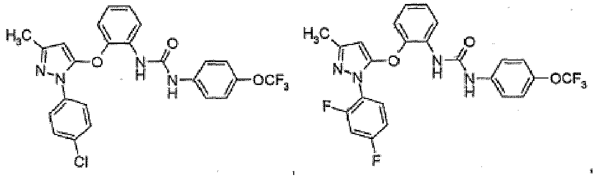 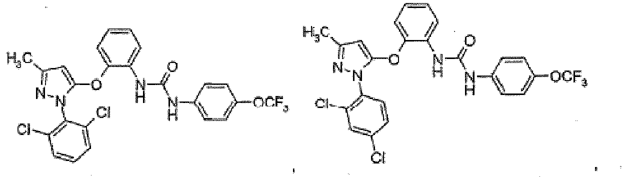 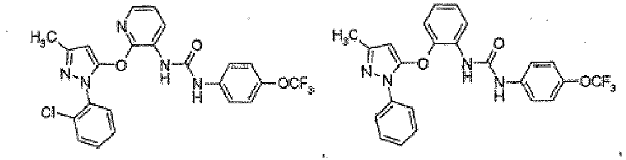 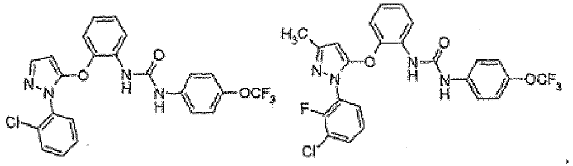 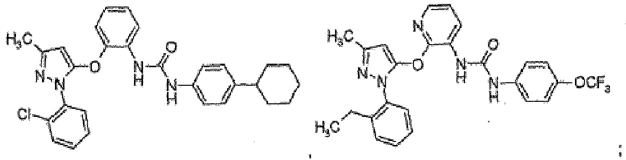 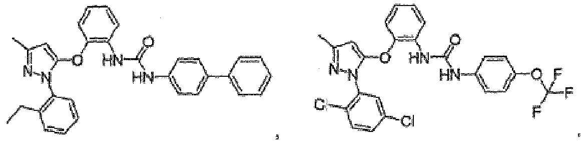 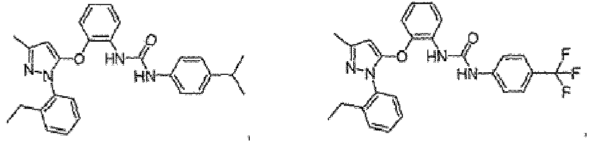 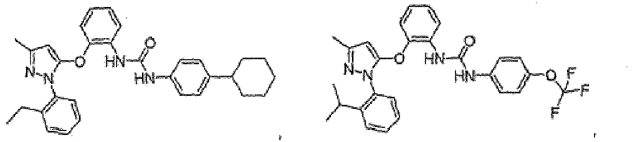 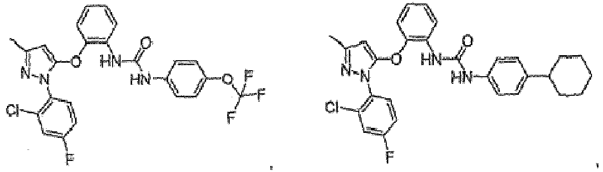 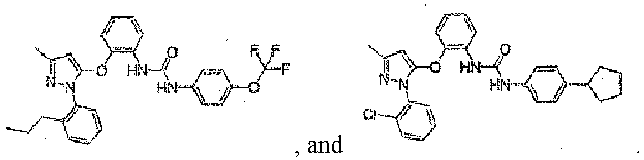
81. The compound for use according to aspect 66 wherein formula (III) is selected from the group 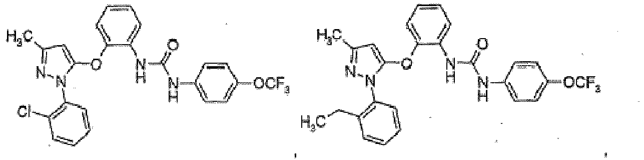 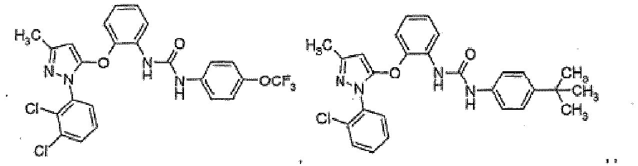 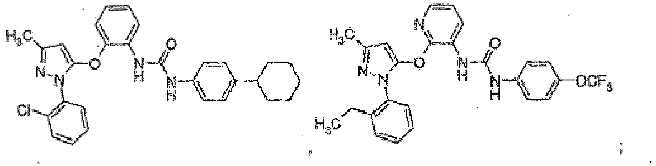
82. The compound for use according to aspect 66 wherein formula (III) is 1-{2-[2-(2-Chloro-phenyl)-5-methyl-2H-pyrazol-3-yloxy]-phenyl}-3-(4-trifluoromethoxy-phenyl)-urea.
83. The compound for use according to aspect 66 wherein formula (III) is 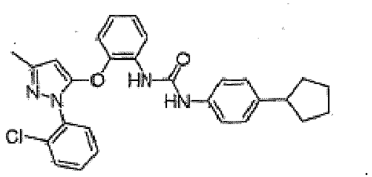
84. The compound for use according to any one of aspects 1 to 83, wherein the compound is a CNS penetrant.
85. The compound for use according to aspect 84, wherein the compound that is a CNS penetrant achieves a brain to plasma ratio that is 0.3 or greater when measured in accordance with the method defined in Reference Example 1.
86. The compound for use according to aspect 85, wherein the compound that is a CNS penetrant achieves a brain to plasma ratio that is 0.5 or greater when measured in accordance with the method defined in Reference Example 1.
87. The compound for use according to aspect 86, wherein the compound that is a CNS penetrant achieves a brain to plasma ratio that is 1.0 or greater when measured in accordance with the method defined in Reference Example 1.
88. The compound for use according to any one of aspects 1 to 87, wherein all of the atoms of the compound are in their natural isotopic form.
89. The compound for use according to any one of aspects 1 to 11 and 20-65, which is a tautomer of formula (I).
90. The compound for use according to any one of aspects 1 to 11, 20-65 and 89, which is an N-oxide of: (a) formula (I); or (b) the tautomer according to aspect 89.
91. The compound for use according to any one of aspects 1 to 11, 20-65, 89 and 90, wherein the compound is a pharmaceutically acceptable salt of: (a) formula (I); (b) the tautomer according to aspect 89; or (c) the N-oxide according to aspect 90.
92. The compound for use according to any one of aspects 1 to 11, 20-65 and 89-91, wherein the compound is a solvate of: (a) formula (I); (b) the tautomer according to aspect 89; (c) the N-oxide according to aspect 90; or (d) the pharmaceutically acceptable salt according to aspect 91.
93. The compound for use according to any one of aspects 1 to 11 and 20-65, which is a compound of formula (I).
94. A pharmaceutical composition for use in a method for treating and/or preventing a CNS disorder, the pharmaceutical composition comprising a compound according to any one of aspects 1 to 93 in association with one or more pharmaceutically acceptable carriers.
95. The use of a compound according to any one of aspects 1 to 93, or the pharmaceutical composition according to aspect 94, for the manufacture of a medicament for use in a method for treating and/or preventing a CNS disorder.
96. A method for treating and/or preventing a CNS disorder in a patient in need thereof, the method comprising administering a compound according to any one of aspects 1 to 93, or the pharmaceutical composition according to aspect 94, to the patient.
97. The compound for use according to any one of aspects 1 to 93, the pharmaceutical composition for use according to aspect 94, the use according to aspect 95, or the method according to aspect 96, wherein the method comprises orally administering the compound.
98. The compound for use, pharmaceutical composition for use, use, or method according to aspect 97, wherein the method comprises orally administering a therapeutically effective amount of the compound.
99. The compound for use according to any one of aspects 1 to 93, 97 and 98, the pharmaceutical composition for use according to any one of aspects 94, 97 and 98, the use according to any one of aspects 95, 97 and 98, or the method according to any one of aspects 96 to 98, wherein the patient is a human.
100. The compound for use according to any one of aspects 1 to 93 and 97 to 99, the pharmaceutical composition for use according to any one of aspects 94 and 97 to 99, the use according to any one of aspects 95 and 97 to 99, or the method according to any one of aspects 96 to 99, wherein the method for treating and/or preventing a CNS disorder is a method for treating a CNS disorder.
101. The compound for use according to any one of aspects 1 to 93 and 97 to 99, the pharmaceutical composition for use according to any one of aspects 94 and 97 to 99, the use according to any one of aspects 95 and 97 to 99, or the method according to any one of aspects 96 to 99, wherein the method for treating and/or preventing a CNS disorder is a method for preventing a CNS disorder.
102. The compound for use according to any one of aspects 1 to 93 and 97 to 101, the pharmaceutical composition for use according to any one of aspects 94 and 97 to 101, the use according to any one of aspects 95 and 97 to 101, or the method according to any one of aspects 96 to 101, wherein the CNS disorder is a CNS disorder susceptible to treatment by P2Y1 inhibition.
103. The compound for use, pharmaceutical composition for use, use, or method according to aspect 102, wherein the CNS disorder is Alzheimer's disease.
104. The compound for use, pharmaceutical composition for use, use, or method according to aspect 103, wherein the Alzheimer's disease is early-onset Alzheimer's disease.
105. The compound for use, pharmaceutical composition for use, use, or method according to aspect 103, wherein the Alzheimer's disease is late-onset Alzheimer's disease.
106. The compound for use, pharmaceutical composition for use, use, or method according to aspect 102, wherein the CNS disorder is pain.
107. The compound for use, pharmaceutical composition for use, use, or method according to aspect 106, wherein the pain comprises neuropathic pain.
108. The compound for use, pharmaceutical composition for use, use, or method according to aspect 106, wherein the pain comprises nociceptive pain.
109. The compound for use, pharmaceutical composition for use, use, or method according to aspect 102, wherein the CNS disorder is epilepsy.
110. The compound for use, pharmaceutical composition for use, use, or method according to aspect 102, wherein the CNS disorder is stroke.
111. The compound for use, pharmaceutical composition for use, use, or method according to aspect 110, wherein the treating and/or preventing said stroke comprises treating and/or preventing said stroke by inhibiting P2Y1 receptors expressed on astrocytes.
112. The compound for use, pharmaceutical composition for use, use, or method according to aspect 102, wherein the CNS disorder is traumatic brain injury.
113. The compound for use, pharmaceutical composition for use, use, or method according to aspect 102, wherein the CNS disorder is amyotrophic lateral sclerosis.
114. The compound for use, pharmaceutical composition for use, use, or method according to aspect 102, wherein the CNS disorder is Huntington's disease.
115. The compound for use, pharmaceutical composition for use, use, or method according to aspect 102, wherein the CNS disorder is Parkinson's disease.
116. The compound for use, pharmaceutical composition for use, use, or method according to aspect 102, wherein the CNS disorder is frontotemporal dementia.
117. A solid dosage form for oral administration comprising a compound according to any one of aspects 1 to 93, in association with one or more pharmaceutically acceptable carriers.
118. A liquid dosage form for oral administration comprising a compound according to any one of aspects 1 to 93, in association with one or more pharmaceutically acceptable carriers.

## Claims

1. A compound that (a) is 1-(2-(2-(*tert*-butyl)phenoxy)pyridin-3-yl)-3-(4-(trifluoromethoxy)phenyl)urea, or (b) is a tautomer, pharmaceutically acceptable salt, or solvate thereof, for use in a method for treating and/or preventing Alzheimer's disease, the method comprising orally administering the compound.

2. A pharmaceutical composition for use in a method for treating and/or preventing Alzheimer's disease, the method comprising orally administering the pharmaceutical composition, the pharmaceutical composition comprising: a compound that (a) is 1-(2-(2-(*tert*-butyl)phenoxy)pyridin-3-yl)-3-(4-(trifluoromethoxy)phenyl)urea, or (b) is a tautomer, pharmaceutically acceptable salt or solvate thereof; in association with one or more pharmaceutically acceptable carriers.

3. A solid dosage form for oral administration comprising: a compound that (a) is 1-(2-(2-(*tert*-butyl)phenoxy)pyridin-3-yl)-3-(4-(trifluoromethoxy)phenyl)urea, or (b) is a tautomer, pharmaceutically acceptable salt or solvate thereof; in association with one or more pharmaceutically acceptable carriers.

4. A liquid dosage form for oral administration comprising: a compound that (a) is 1-(2-(2-(*tert*-butyl)phenoxy)pyridin-3-yl)-3-(4-(trifluoromethoxy)phenyl)urea, or (b) is a tautomer, pharmaceutically acceptable salt or solvate thereof; in association with one or more pharmaceutically acceptable carriers.
